# EUROPEAN PATENT APPLICATION

(11) **EP 1 832 660 A1**
(43) Date of publication of application: **12.09.2007**
(21) Application number: 05811239.2
(22) Date of filing: 01.12.2005
(51) Int. Cl.: C12Q 1/68, C12N 15/09, C12Q 1/02, G01N 33/15, G01N 33/50

(54) **METHOD OF SCREENING ANTIOBESITY DRUG**

(30) Priority: 03.12.2004 JP 2004351814
(71) Applicant: Astellas Pharma Inc., Tokyo 103-8411 (JP); Keio University, Tokyo 108-8345 (JP)
(72) Inventor: YASUNAGA, Kunio, c/o Astellas Pharma Inc., Tokyo 1038411 (JP); YAMAJI, Noboru, c/o Astellas Pharma Inc., Tokyo 1038411 (JP); ABE, Kunitake, c/o Astellas Pharma Inc., Tokyo 1038411 (JP); OIKE, Yuichi, Keio University School of Medicine, Tokyo 1608582 (JP)
(74) Representative: Bates, Philip Ian
(86) International application number: PCT/JP2005/022107
(87) International publication number: WO 2006/059691

(57) **Abstract**

The present invention relates to a method of screening for an antiobesity agent, an antidiabetic agent, and/or a hypolipidemic agent, comprising the step of analyzing whether or not a substance to be tested promotes an expression and/or a function of an angiopoietin-related growth factor. The present invention relates to a method of screening for an agent for promoting an expression and/or a function of an angiopoietin-related growth factor.

## Description

### TECHNICAL FIELD

The present invention relates to a method of screening for antiobesity agents.

### BACKGROUND ART

Although the deleterious effect of obesity is widely known, there has been a remarkable increase in obesity in recent years. It is well-known that obesity (i.e., overaccumulation of fat in fatty tissues) causes various diseases, and thus it is proposed that obesity should be addressed as a disease to be treated. Diseases caused by obesity as a factor include, for example, lumbago, knee joint pain, and osteoarthrosis. Such orthopedic diseases are directly caused by a gain in body weight due to obesity. The overaccumulation of fat associated with obesity causes diabetes, hyperlipemia, hypertension, or arteriosclerotic disease. In particular, it is known that an overaccumulation of visceral fat is involved in the development of such diseases (non-patent reference 1).

Basic methods for alleviating obesity include kinesitherapy and diet therapy, but to continue with such therapy is difficult. As methods other than the kinesitherapy and diet therapy, medicaments are used. At present, Sibutramine and orlistat are mainly used on a global scale. However, these medicaments have not only a weak, but also an adverse effect. In Japan, only mazindol is authorized, but the application thereof is limited to severe obesity, and the period of administration is also limited (non-patent reference 2).

Due to the modernization of society, the number of patients suffering from diabetes is rapidly increasing, not only in Japan but also globally. In particular, it is known that the development of type II diabetes having a number of patients is involved in obesity or an overaccumulation of fat. As with obesity, treatments for type II diabetes include kinesitherapy and diet therapy, but medicaments are used because it is difficult to continue this therapy. Patients suffering from severe diabetes are treated with insulin, but the treatment with insulin has a risk of an adverse effect such as hypoglycemia. As oral hypoglycemic drugs, thiazolidinediones or sulfonylurea (SU) agents are mainly used. However, the thiazolidinediones have an adverse effect such as hepatopathy, edema, or heart failure, and the SU agents have an adverse effect such as the promotion of obesity, and thus, an agent for alleviating insulin resistance without an increase in body weight or such adverse effects is greatly desired (non-patent reference 3).

Hypertrophied adipocytes are observed in the visceral fat of an obese patient suffering from diabetes. Adipocytokines capable of promoting insulin resistance are produced and secreted from hypertrophied adipocytes, and act on adipocytes and/or myocytes close to the hypertrophied adipocytes to promote insulin resistance. In patients suffering from diabetes, adipose tissues change to tissues which are involved in the promotion of insulin resistance (non-patent references 4 and 5).

Leptin is well-known as a factor involved in the accumulation of adipose tissues which cause obesity or diabetes. Leptin is an inhibitory hormone for bodyweight gain, and it is known that a deficiency of leptin causes obesity by promoting the appetite and reducing energy consumption. The findings of such factors involved in the accumulation of adipose tissues and hypertrophy of adipocytes are very useful in developing therapeutic agents for diseases such as obesity, diabetes, or hyperlipemia (non-patent reference 6).

An angiopoietin-related growth factor (AGF) is a secretory protein having a coiled-coil domain at the N-terminal side and a fibrinogen-like domain at the C-terminal side. The AGF is identical with NL8 reported in patent reference 1. It is reported that when CHO cells stably expressing NL8 are subcutaneously implanted into a nude mouse, the CHO cells exhibit tumorigenicity. Transgenic (Tg) mice in which AGF was overexpressed in epidermal cells utilizing a K14 promoter were used to find that AGF exhibits an angiogenetic activity, an epidermal cell proliferating activity, a chondrocyte proliferating activity, an activity of promoting wound healing, and a tissue generative activity (patent reference 1 and non-patent reference 7). Patent references 3 to 13 disclose polypeptides having an amino acid sequence identical to that of a human AGF as described herein. These references disclose an analysis of the expression distribution thereof (patent references 3 and 4), an activity of inhibiting proliferation by stimulation of the vascular endothelial growth factor (VEGF) (patent reference 4), and an overexpression thereof in human umbilical vein endothelial cell (HUVEC) treated with a growth factor (patent references 5 and 6), and further disclose that the polypeptide identical with the AGF is involved in the promotion or inhibition of angiogenesis, and many diseases are listed as cardiovascular, endothelial, or angiogenetic diseases which may be treated with the polypeptide (patent references 3 to 9). Further, patent references 10 to 13 suggest that AGF may be used for a proliferation of epithelial cells or healing of wounds.

[non-patent reference 1] Metabolism, (U.S.A.), 1987, vol. 36, p.54-59
[non-patent reference 2] Nippon Rinsho, 2003, vol. 61, supplement 6, "Obesity", p.649-654
[non-patent reference 3] Nippon Rinsho, 2002, vol. 60, supplement 9, Shin-jidai no Tounyoubyougaku 3, p.310-331
[non-patent reference 4] Igaku no ayumi, 2000, vol. 192, p.513-518
[non-patent reference 5] Igaku no ayumi, 2000, vol. 192, p.541-545
[non-patent reference 6] Trends in Molecular Medicine, (Netherlands), 2002, vol. 8, no. 9, p.442-447
[non-patent reference 7] Proceedings of the National Academy of Sciences of the United States of America, (U.S.A.), 2003, Vol. 100, p. 9494-9499
[patent reference 1] International Publication No. WO99/15653
[patent reference 2] International Publication No. WO03/083114
[patent reference 3] International Publication No. WO00/32221
[patent reference 4] International Publication No. WO00/53753
[patent reference 5] International Publication No. WO02/00690
[patent reference 6] International Publication No. WO02/08284
[patent reference 7] U.S. Patent Application Publication No. 2004/0043927
[patent reference 8] U.S. Patent Application Publication No. 2003/0105012
[patent reference 9] U.S. Patent Application Publication No. 2003/0105011
[patent reference 10] U.S. Patent No. 6350450
[patent reference 11] U.S. Patent No. 6413770
[patent reference 12] Japanese Unexamined Patent Publication (Kokai) No. 2000-300263
[patent reference 13] U.S. Patent No. 6420542

### DISCLOSURE OF INVENTION

### PROBLEMS TO BE SOLVED BY THE INVENTION

An object of the present invention is to provide a novel method of screening for an antiobesity agent, an antidiabetic agent, and/or a hypolipidemic agent.

### MEANS FOR SOLVING THE PROBLEMS

The present inventors have conducted intensive studies and, as a result, can now reveal that AGF has an antiobesity activity, an antidiabetic activity, and a hypolipidemic activity, by preparing and analyzing AGF knockout (KO) mice and AGF transgenic (Tg) mice. That is, the present inventors found that AGF KO mice became remarkably obese, there was an increase in weight of adipose tissues accompanied by obesity, adipocytes were enlarged, there was an increase in each amount of triglyceride contained in skeletal muscles or the liver, and the AGF KO mice suffered from hyperinsulinemia and exhibited an abnormality in glucose tolerance (Referential Examples 4 to 8). In this connection, it is known that the triglyceride contents in skeletal muscles or the liver are increased by obesity. In contrast, the present inventors found that, in the AGF Tg mice (CAG-AGF Tg mice, in which AGF was systemically overexpressed), an increase in body weight was suppressed, an increase in weight of adipose tissues was suppressed, an enlargement of adipocytes was suppressed, each amount of triglyceride contained in skeletal muscles or the liver was decreased, and the insulin sensitivity, lipid metabolism, and the glucose tolerance were improved (Referential Examples 4 to 7 and 15 to 16). Furthermore, it was found that, in mice to which an adenovirus expressing AGF was administered, an increase in body weight was suppressed and glucose tolerance was improved (Referential Examples 14 and 19), and that, in other AGF transgenic mice (K14-AGF Tg mice, in which AGF was overexpressed in epidermal cells), AGF exhibited an antiobesity activity, an activity of decreasing fat tissue, and an activity of increasing insulin sensitivity (Referential Examples 17 and 18).

From these findings, the present inventors clarified that AGF can be used as an active ingredient of an antiobesity agent, an antidiabetic agent, and/or a hypolipidemic agent. Further, these findings show that an agent for promoting the expression of AGF and an agent for promoting the functions of AGF (i.e., a substance having an agonist activity of AGF, and an agent for promoting a binding of AGF to a cell) are useful as an active ingredient of an antiobesity agent, an antidiabetic agent, and/or a hypolipidemic agent.

The present inventors constructed ELISA (enzyme-linked immunosorbent assay) systems for measuring an amount of AGF protein (Examples 3, 4, 7, and 10), and established a quantitative PCR method for measuring an amount of an AGF gene (Example 13). Further, the present inventors obtained DNAs containing a mouse AGF promoter region, and used the DNAs to construct a method of screening for an agent for promoting an AGF expression (Example 15). Furthermore, the present inventors found that AGF promoted phosphorylation of mitogen-activated protein kinases (MAPKs), and constructed a method of screening for an agent for promoting an AGF function by analyzing the phosphorylation of an MAPK (Example 16). Furthermore, the present inventor found that AGF bound to the surface of a cell, and constructed a method of screening for an agent for promoting an AGF function by analyzing the binding of AGF to a cell (Example 17). That is, the present inventors constructed a novel method of screening for an antiobesity agent, an antidiabetic agent, and/or a hypolipidemic agent (Examples 3, 4, 7, 10, 13, and 15 to 17), and completed the present invention.

The present invention relates to:
[1] a method of screening for an antiobesity agent, an antidiabetic agent, and/or a hypolipidemic agent, comprising the step of analyzing whether or not a substance to be tested promotes an expression and/or a function of an angiopoietin-related growth factor;
[2] the method of [1], wherein the analyzing step comprises the steps of:
   i) bringing a substance to be tested into contact with a cell, and
   ii) measuring an amount of an endogenous angiopoietin-related growth factor protein or gene contained in the cell, and analyzing a test substance dependent change in the amount thereof;
[3] the method of [2], wherein an amount of an angiopoietin-related growth factor protein is measured in the measuring step;
[4] the method of [2], wherein an amount of an angiopoietin-related growth factor gene is measured in the measuring step;
[5] the method of [1], wherein the analyzing step comprises the steps of:
   i) bringing a substance to be tested into contact with a cell transformed with a vector in which a reporter gene is fused downstream of a DNA selected from
      a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor, or
      a DNA comprising a nucleotide sequence having a 90% or more identity with that consisting of nucleotides 672-5572 of SEQ ID NO: 1, and having a promoter activity for an angiopoietin-related growth factor, and
   ii) measuring an expression of the reporter gene, and analyzing a test substance dependent change in the expression;
[6] the method of [1], wherein the analyzing step comprises the steps of:
   i) bringing a substance to be tested into contact with a cell; and
   ii) measuring a phosphorylation of a mitogen-activated protein kinase, and analyzing a test substance dependent change in the phosphorylation;
[7] the method of [1], wherein the analyzing step comprises the steps of:
   i) bringing a substance to be tested and an angiopoietin-related growth factor into contact with a cell; and
   ii) measuring an amount of the angiopoietin-related growth factor which binds to the cell, and analyzing a test substance dependent change in the amount thereof;
[8] a method of screening for an agent for promoting an expression and/or a function of an angiopoietin-related growth factor, comprising the steps of:
   analyzing whether or not a substance to be tested promotes the expression and/or the function of an angiopoietin-related growth factor, and
   analyzing effects of a selected candidate on a change in body weight, a blood glucose level, and/or a lipid content in blood;
[9] the method of [8], wherein the step of analyzing whether or not the expression and/or the function of an angiopoietin-related growth factor is promoted comprises the steps of:
   i) bringing a substance to be tested into contact with a cell, and
   ii) measuring an amount of an endogenous angiopoietin-related growth factor protein or gene contained in the cell, and analyzing a test substance dependent change in the amount thereof;
[10] the method of [9], wherein an amount of an angiopoietin-related growth factor protein is measured in the measuring step;
[11] the method of [9], wherein an amount of an angiopoietin-related growth factor gene is measured in the measuring step;
[12] the method of [8], wherein the step of analyzing whether or not the expression and/or the function of an angiopoietin-related growth factor is promoted comprises the steps of:
   i) bringing a substance to be tested into contact with a cell transformed with a vector in which a reporter gene is fused downstream of a DNA selected from
      a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor, or
      a DNA comprising a nucleotide sequence having a 90% or more identity with that consisting of nucleotides 672-5572 of SEQ ID NO: 1, and having a promoter activity for an angiopoietin-related growth factor, and
   ii) measuring an expression of the reporter gene, and analyzing a test substance dependent change in the expression;
[13] the method of [8], wherein the step of analyzing whether or not the expression and/or the function of an angiopoietin-related growth factor is promoted comprises the steps of:
   i) bringing a substance to be tested into contact with a cell; and
   ii) measuring a phosphorylation of a mitogen-activated protein kinase, and analyzing a test substance dependent change in the phosphorylation; and
[14] the method of [8], wherein the step of analyzing whether or not the expression and/or the function of an angiopoietin-related growth factor is promoted comprises the steps of:
   i) bringing a substance to be tested and an angiopoietin-related growth factor into contact with a cell; and
   ii) measuring an amount of the angiopoietin-related growth factor which binds to the cell, and analyzing a test substance dependent change in the amount thereof.

WO04/108920, published after the priority date of the present application, discloses that AGF has an activity of alleviating obesity, diabetes, and/or hyperlipemia.
Further, Nature Medicine, 11(4), 400-408, 2005, published after the priority date of the present application, discloses the relationship of AGF with obesity and insulin resistance. Patent references 3 to 9 disclose many diseases including diabetes, as a disease involved in AGF, but no support for the relationship between AGF and diabetes is disclosed therein. Further, on the priority date of the present application, the relationship of AGF with antiobesity and/or hyperlipemia was not known.

The term "antiobesity agent" as used herein includes both an agent used for treating a patient suffering from obesity and an agent used preventively for a subject exhibiting signs of obesity.
The term "antidiabetic agent" as used herein includes both an agent used for treating a patient suffering from diabetes and an agent used preventively for a subject exhibiting signs of diabetes.
The term "hypolipidemic agent" as used herein includes both an agent used for treating a patient suffering from hyperlipemia and an agent used preventively for a subject exhibiting signs of hyperlipemia.

The term "screening" as used herein includes both an identification of one or more substances having an activity of interest from many test substances, and a judgment of whether or not a substance to be tested has an activity of interest.

The term "agent for promoting an expression of an AGF" as used herein means a substance which promotes an amount of the AGF protein or an amount of the AGF gene. The term "agent for promoting a function of an AGF" as used herein means a substance which promotes a function of the AGF.

### EFFECTS OF THE INVENTION

According to the screening method of the present invention, substances which are useful as an antiobesity agent, an antidiabetic agent, and/or a hypolipidemic agent can be obtained.

### BRIEF DESCRIPTION OF THE DRAWINGS

Figure 1 is a schematic drawing showing the structure of plasmid pBS-loxP-lox71-mAGF-βgeo. The abbreviations "pro." and "pri." mean a promoter and a primer, respectively.
Figure 2 is a graph showing changes in body weight of AGF KO mice. The horizontal axis indicates an age in weeks (weeks), and the vertical axis indicates body weight (g). The abbreviations "WT", "HTR", and "HM" mean WT mice, heterozygous KO mice, and homozygous KO mice, respectively.
Figure 3 is a graph showing changes in the weight of genital fat pads (white adipose tissue) of CAG-AGF Tg mice (normal diet). The vertical axis indicates [weight of white adipose tissue (g)]/[body weight (g)]. The abbreviations "WT" and "Tg" mean WT mice and Tg mice, respectively.
Figure 4 is a graph showing changes in the weight of genital fat pads (white adipose tissue) of CAG-AGF Tg mice (high fat diet). The vertical axis indicates [weight of white adipose tissue (g)]/[body weight (g)]. The abbreviations "WT" and "Tg" mean WT mice and Tg mice, respectively.
Figure 5 is a graph showing changes in the weight of genital fat pads (white adipose tissue) of AGF KO mice (weight of white adipose tissue). The vertical axis indicates the weight of white adipose tissue (g). The abbreviations "WT", "HTR", and "HM" mean WT mice, heterozygous KO mice, and homozygous KO mice, respectively.
Figure 6 is a graph showing changes in the weight of genital fat pads (white adipose tissue) of AGF KO mice (weight of white adipose tissue/body weight). The vertical axis indicates [weight of white adipose tissue (g)]/[body weight (g)]. The abbreviations "WT", "HTR", and "HM" mean WT mice, heterozygous KO mice, and homozygous KO mice, respectively.
Figure 7 is a microphotograph showing the form of adipocytes in the CAG-AGF Tg mouse. The abbreviations "Tg" and "NTG" mean Tg mice and WT mice, respectively.
Figure 8 is a microphotograph showing the form of adipocytes in the AGF homozygous KO mouse.
Figure 9 is a microphotograph showing the form of adipocytes in the littermate WT mouse.
Figure 10 is a graph showing TG content in tissue (liver) of the CAG-AGF Tg mouse. The horizontal axis indicates the period of breeding with a high fat diet (month). The vertical axis indicates a TG content (mg/g tissue). The abbreviations "WT" and "Tg" mean WT mice and Tg mice, respectively.
Figure 11 is a graph showing TG content in tissue (skeletal muscles) of the CAG-AGF Tg mouse. The horizontal axis indicates the period of breeding with a high fat diet (months). The vertical axis indicates a TG content (mg/g tissue). The abbreviations "WT" and "Tg" mean WT mice and Tg mice, respectively.
Figure 12 is a graph showing TG content in tissue of the AGF KO mice. The vertical axis indicates a TG content (mg/g tissue). The abbreviations "L" and "SM" in the horizontal axis mean the liver and skeletal muscles, respectively. The abbreviations "WT", "HTR", and "HM" mean WT mice, heterozygous KO mice, and homozygous KO mice, respectively.
Figure 13 is a graph showing the result (blood glucose level) of a glucose tolerance test in the AGF KO mice. The horizontal axis indicates a time (minutes), and the vertical axis indicates a blood glucose level (mg/dL). The abbreviations "WT" and "HM" mean WT mice and homozygous KO mice, respectively.
Figure 14 is a graph showing the result (plasma insulin) of a glucose tolerance test in the AGF KO mice. The horizontal axis indicates a time (minutes), and the vertical axis indicates plasma insulin (ng/mL). The abbreviations "WT" and "HM" mean WT mice and homozygous KO mice, respectively.
Figure 15 is a graph showing the result of a glucose metabolism test in mice to which an adenovirus expressing the mouse AGF was administered. The solid circles indicate the result from mAGF-Adeno administered mice, and the open circles indicate the result from Cont-Adeno administered mice. The horizontal axis indicates a time (minutes), and the vertical axis indicates blood glucose level (mg/dL).
Figure 16 is a graph showing a concentration of insulin in plasma (vertical axis; ng/mL) of CAG-AGF Tg mice and littermate WT mice bred with a high fat diet. The abbreviations "TG" and "NTG" mean the CAG-AGF Tg mice and the littermate WT mice, respectively.
Figure 17 is a graph showing a concentration of cholesterol in serum (vertical axis; mg/dL) of CAG-AGF Tg mice and littermate WT mice bred with a high fat diet. The abbreviations "TG" and "NTG" mean the CAG-AGF Tg mice and the littermate WT mice, respectively.
Figure 18 is a graph showing a concentration of free fatty acids in serum (vertical axis; µEq/L) of CAG-AGF Tg mice and littermate WT mice bred with a high fat diet. The abbreviations "TG" and "NTG" mean the CAG-AGF Tg mice and the littermate WT mice, respectively.
Figure 19 is a graph showing the result of a glucose metabolism test in CAG-AGF Tg mice and littermate WT mice. The abbreviations "TG" and "NTG" mean the CAG-AGF Tg mice and the littermate WT mice, respectively. The horizontal axis indicates a time (minutes), and the vertical axis indicates blood glucose level (mg/dL).
Figure 20 is a graph showing the result of an insulin sensitivity test in CAG-AGF Tg mice and littermate WT mice. The abbreviations "TG" and "NTG" mean the CAG-AGF Tg mice and the littermate WT mice, respectively. The horizontal axis indicates a time (minutes), and the vertical axis indicates the ratio (%) of the blood glucose level after the insulin administration to that before the insulin administration.
Figure 21 is a graph showing the weight of visceral fat tissue in K14-AGF Tg mice and littermate WT mice. The abbreviations "K14-AGF" and "NTG" mean the K14-AGF Tg mice and the littermate WT mice, respectively. The vertical axis indicates the percentage (%) of the weight of visceral fat tissue per body weight.
Figure 22 is a graph showing the weight of subcutaneous fat tissue in K14-AGF Tg mice and littermate WT mice. The abbreviations "K14-AGF" and "NTG" mean the K14-AGF Tg mice and the littermate WT mice, respectively. The vertical axis indicates the percentage (%) of the weight of subcutaneous fat tissue per body weight.
Figure 23 is a graph showing the result of an insulin sensitivity test in K14-AGF Tg mice and littermate WT mice. The abbreviations "K14-AGF" and "NTG" mean the K14-AGF Tg mice and the littermate WT mice, respectively. The horizontal axis indicates a time (minutes), and the vertical axis indicates the ratio (%) of the blood glucose level after the insulin administration to that before the insulin administration.
Figure 24 is a graph showing the result of a glucose metabolism test in mice administrated adenovirus expressing human AGF. The solid circles indicate the result from hAGF-Adeno administered mice, and the open circles indicate the result from Cont-Adeno administered mice. The horizontal axis indicates a time (minutes), and the vertical axis indicates blood glucose level (mg/dL).
Figure 25 is a graph showing a mouse AGF promoter activity in FaO cells. The vertical axis indicates the activity (compensated).

### BEST MODE FOR CARRYING OUT THE INVENTION

The screening method of the present invention is based on the findings that AGF exhibits an antiobesity activity, an antidiabetic activity, and a hypolipidemic activity, and thus an agent for promoting an expression of AGF and/or an agent for promoting a function of AGF are useful as an active ingredient of an antiobesity agent, an antidiabetic agent, and/or a hypolipidemic agent. The screening method of the present invention includes:
(1) a screening method using a change in an amount of AGF protein or an amount of AGF gene as an index (hereinafter referred to as an AGF-type screening method),
(2) a screening using a mouse AGF promoter (hereinafter referred to as a promoter-type screening method),
(3) a screening method using a change in a phosphorylation of MAPK as an index (hereinafter referred to as MAPK-type screening method), and
(4) a screening method using a change in an amount of AGF which binds to a cell as an index (hereinafter referred to as a cell-binding-type screening method).

Substances to be tested which may be used in the screening method of the present invention are not limited, but there may be mentioned, for example, commercially available compounds (including peptides), various known compounds (including peptides) registered in chemical files, compounds obtained by combinatorial chemistry techniques (Terrett et al., J. Steele. Tetrahedron, 51, 8135-8137, 1995), culture supernatants of microorganisms, natural components derived from plants or marine organisms, animal tissue extracts, or compounds (including peptides) obtained by chemically or biologically modifying compounds (including peptides) selected by the screening method of the present invention.

(1) AGF-type screening method
   The AGF-type screening method of the present invention comprises the steps of:
   i) bringing a substance to be tested into contact with a cell, and
   ii) measuring an amount of an endogenous AGF protein or gene contained in the cell, and analyzing a test substance dependent change in the amount thereof,
      and, if desired, may further comprise the step of:
      analyzing effects of a candidate, which is selected in the above step ii) and has an activity of promoting an expression of the AGF, on a change in body weight, a blood glucose level, and/or a lipid content in blood.

According to the AGF-type screening method of the present invention, an agent for promoting an expression of AGF can be obtained. The agent for promoting an expression of AGF obtained by the AGF-type screening method of the present invention can be used as an active ingredient of an antiobesity agent, an antidiabetic agent, and/or a hypolipidemic agent.

A cell which may be used in the AGF-type screening method of the present invention is not limited, so long as an endogenous AGF is being expressed or can be expressed. As the cell, a cell expressing the AGF is preferable, and there may be mentioned, for example, mouse myoblast cell line C2C12 (ATCC No.: CRL-1772) or human hepatocarcinoma cell line HepG2 (ATCC No.: HB-8065). The cell used in the AGF-type screening method is not limited to culture cells, and an embodiment in which a test substance is brought into contact with animal cells by administering the test substance to an animal is included in the AGF-type screening method. After the contact of a test substance with cells, a culture supernatant or a cell lysate, or a sample (such as a serum or tissue-derived cells) collected from the animal may be used as a sample, to measure an amount of an endogenous AGF protein or gene contained in the cells, and to analyze a test substance dependent change in the amount thereof.

An amount of AGF protein may be measured by a known method, such as an immunoassay or Western blotting, preferably an immunoassay, more preferably an ELISA described in Example 3, Example 4, Example 7, or Example 10.
The present invention includes, for example, an embodiment in which an amount of AGF protein is measured by an immunoassay comprising the steps of:
i) reacting a sample with an immobilized antibody (or a fragment thereof) specific to AGF, and
ii) reacting an unlabeled or labeled anti-AGF antibody (or a fragment thereof) with a complex formed by reacting the immobilized antibody (or the fragment) with AGF contained in the sample.

An amount of a gene of AGF (an amount of mRNA) may be measured by a known method, such as a quantitative PCR (polymerase chain reaction) method or Northern blotting, preferably an quantitative PCR method. Most preferably, an amount of a mouse AGF gene may be measured by the quantitative PCR method described in Example 13. Most preferably, an amount of a human AGF gene may be measured by a quantitative PCR method similar to that described in Example 13, except that a pair of oligonucleotides consisting of the nucleotide sequences of SEQ ID NOS: 43 and 44 are used as a primer set. An amount of a rat AGF gene may be measured by, most preferably a quantitative PCR method similar to that described in Example 13, except that a pair of oligonucleotides consisting of the nucleotide sequences of SEQ ID NOS: 51 and 52 are used as a primer set.

A primer set used in the quantitative PCR method is not limited, so long as it is a set of a forward primer and a reverse primer capable of specifically amplifying an AGF gene of interest. Such a primer set may be designed, for example, by selecting an appropriate region by using a software for analysis (Primer Express; Applied Biosystems) on the basis of a nucleotide sequence of an AGF gene [for example, GenBank database accession No. NM_145154 (mouse), NM_031917 (human), or XM_216613 (rat)]. Combinations of SEQ ID NOS: 29 and 30 (for measuring an amount of mouse AGF; the primer set used in Example 13), SEQ ID NOS: 43 and 44 (for measuring an amount of human AGF; the above-mentioned primers), and SEQ ID NOS: 51 and 52 (for measuring an amount of rat AGF; the above-mentioned primers) are preferable as a primer set.

In the AGF-type screening method of the present invention, a substance in which the activity of increasing an amount of AGF expressed (an amount of AGF protein or gene) is 1.5 times or more (preferably 2 times or more, more preferably 4 times or more), for example, under the conditions described in Example 3, Example 4, Example 7, or Example 10 (ELISA) or in Example 13 (quantitative PCR method) may be selected as a substance having an activity of promoting an expression of AGF. When a test substance is a compound, a compound having the above activity at a concentration of 10 µmol/L (preferably 1 µmol/L, more preferably 0.1 µmol/L) may be selected as a substance having an activity of promoting an expression of AGF.

(2) Promoter-type screening method
   The promoter-type screening method of the present invention comprises the steps of:
   i) bringing a substance to be tested into contact with a cell transformed with a vector in which a reporter gene is fused downstream of a DNA selected from
      a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor,
      a DNA comprising a nucleotide sequence having a 90% or more identity with that consisting of nucleotides 672-5572 of SEQ ID NO: 1, and having a promoter activity for an angiopoietin-related growth factor, or
      a DNA consisting of the nucleotides 672-5572, 548-5572, 239-5572, or 16-5572 of SEQ ID NO: 1, or the nucleotides 16-6684 of SEQ ID NO: 2, and
   ii) measuring an expression of the reporter gene, and analyzing a test substance dependent change in the expression,
      and, if desired, may further comprise the step of:
      analyzing effects of a candidate, which is selected in the above step ii) and has an activity of promoting an expression of the AGF, on a change in body weight, a blood glucose level, and/or a lipid content in blood.

According to the promoter-type screening method of the present invention, an agent for promoting an expression of AGF can be obtained. The agent for promoting an expression of AGF obtained by the promoter-type screening method of the present invention can be used as an active ingredient of an antiobesity agent, an antidiabetic agent, and/or a hypolipidemic agent.

The present invention includes a use of a DNA selected from
a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor,
a DNA comprising a nucleotide sequence having a 90% or more identity with that consisting of nucleotides 672-5572 of SEQ ID NO: 1, and having a promoter activity for an angiopoietin-related growth factor, or
a DNA consisting of nucleotides 672-5572, 548-5572, 239-5572, or 16-5572 of SEQ ID NO: 1, or nucleotides 16-6684 of SEQ ID NO: 2,
in screening for an antiobesity agent, an antidiabetic agent, and/or a hypolipidemic agent.

As a promoter which may be used in the promoter-type screening method of the present invention, there may be mentioned, preferably,
a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor, or
a DNA comprising a nucleotide sequence having a 90% or more identity with that consisting of nucleotides 672-5572 of SEQ ID NO: 1, and having a promoter activity for an
angiopoietin-related growth factor;
more preferably, a DNA consisting of any consecutive nucleotide sequence which is selected from nucleotides 16-5572 and contains nucleotides 672-5572;
most preferably,
a DNA consisting of nucleotides 672-5572 of SEQ ID NO: 1,
a DNA consisting of nucleotides 548-5572 of SEQ ID NO: 1,
a DNA consisting of nucleotides 239-5572 of SEQ ID NO: 1,
a DNA consisting of nucleotides 16-5572 of SEQ ID NO: 1, or
a DNA consisting of nucleotides 16-6684 of SEQ ID NO: 2.

The nucleotide sequence of SEQ ID NO: 1 is a mouse AGF promoter region having a length of approximately 5.6 kb upstream of exon 1 of a mouse AGF gene. The nucleotide sequence of SEQ ID NO: 2 is a mouse AGF promoter region having a length of approximately 6.7 kb (consisting of approximately 5.6 kb upstream of exon 1, approximately 100 b of exon 1, and approximately 1 kb of intron 1) upstream of exon 2 of a mouse AGF gene.

A reporter gene assay (Tamura et al., "Tensha inshi kenkyuhou", YODOSHA, 1993) is a method in which regulation of a gene expression is analyzed (for example, detected or measured) on the basis of an expression of a reporter gene as a marker. A gene expression is generally regulated by a promoter region located at the 5' upstream of the gene, and thus an amount of the gene expressed at the transcriptional stage may be estimated by measuring the promoter activity. When a test substance activates the promoter, the transcription of a reporter gene located downstream of the promoter region is activated. That is, the action of activating a promoter (i.e., the action of promoting an expression) may be detected by replacing it with an expression of the reporter gene. Therefore, the action of a test substance on the regulation of AGF expression may be detected by the reporter gene assay using the AGF promoter region, on the basis of the expression of the reporter gene.

The "reporter gene" fused to the above promoter is not particularly limited, so long as it is commonly used. As the reporter gene, an enzyme gene in which a quantitative measurement may be easily performed is preferable. As the enzyme gene, there may be mentioned, for example, a chloramphenicol acetyl transferase gene (CAT) derived from a bacterial transposon, a luciferase gene (Luc) derived from a firefly or renilla, or a green fluorescent protein gene (GFP) derived from a jellyfish. The reporter gene may be functionally fused to the above promoter. The reporter gene fused to the above promoter is stably or transiently expressed in cells such as animal cells or yeast. An amount of the reporter gene expressed in the transformants when a test substance is brought into contact therewith may be compared to that when a test substance is not added, and a test substance dependent change in the promoter activity may be analyzed.

The transformant with a vector in which a reporter gene is fused downstream of the promoter may be prepared in a conventional method, for example, the method described in Example 15. A method for analyzing an amount of a reporter gene expressed may be appropriately selected in accordance with a protein encoded by the reporter gene. For example, when the reporter gene encodes a fluorescent protein such as luciferase, an amount of the reporter gene expressed may be determined by dissolving the transformants by an appropriate method to obtain a cell lysate, adding luciferin as a substrate to a supernatant of the cell lysate, and measuring fluorescence by an appropriate fluorescence detector (for example, ML3000; Dinatech laboratories). The reaction may be carried out using a commercially available detection kit, for example, Luciferase Assay System (Promega).

In the promoter-type screening method of the present invention, a substance in which the activity of increasing a reporter activity is 1.5 times or more (preferably 2 times or more, more preferably 4 times or more), for example, under the conditions described in Example 15 may be selected as a substance having an activity of promoting an expression of AGF. When a test substance is a compound, a compound having the above activity at a concentration of 10 µmol/L (preferably 1 µmol/L, more preferably 0.1 µmol/L) may be selected as a substance having an activity of promoting an expression of AGF.

(3) MAPK-type screening method
   The MAPK-type screening method comprises the steps of:
   i) bringing a substance to be tested into contact with a cell;
   ii) measuring a phosphorylation of a MAPK, and analyzing a test substance dependent change in the phosphorylation; and
   iii) analyzing effects of a candidate, which is selected in the above step ii) and has an agonist activity of AGF, on a change in body weight, a blood glucose level, and/or a lipid content in blood.

As shown in Example 16, it was found that the addition of AGF to cells promoted the phosphorylation of MAPK p38, MAPK p42, and MAPK p44. The result indicates that the promotion of a function of AGF can be judged by analyzing a change in the phosphorylation of MAPK. According to the MAPK-type screening method of the present invention, an agent for promoting a function of AGF can be obtained. The agent for promoting a function of AGF obtained by the MAPK-type screening method of the present invention can be used as an active ingredient of an antiobesity agent, an antidiabetic agent, and/or a hypolipidemic agent.

As the cell used in the MAPK-type screening method of the present invention, a cell in which at least one MAPK is expressed and AGF can bind thereto is preferable, and there may be mentioned, for example, a human umbilical vein endothelial cell HUVEC (CAMBREX), a C2C12 cell differentiated into skeletal muscle, or a chondrogenic cell line ATDC5 (Cell Differ.Dev., 1990, 30(2), 109-116). After the contact of a test substance with cells, for example, a culture supernatant or a cell lysate may be prepared, to measure the phosphorylation of MAPK and analyze a test substance dependent change in the phosphorylation.

The phophorylation of MAPK may be measured by, for example, Western blotting or an immunoassay using an anti-phosphorylated MAPK antibody, preferably Western blotting, more preferably Western blotting described in Example 16.

As the MAPK, there may be mentioned, for example, MAPK p38, MAPK p42, or MAPK p44. In the MAPK-type screening method of the present invention, at least one MAPK (preferably two or more MAPKs) is analyzed.

In the MAPK-type screening method of the present invention, a substance having an agonist activity of AGF, such as a substance in which the activity of increasing an amount of a phosphorylated protein is 1.5 times or more (preferably 2 times or more, more preferably 4 times or more), for example, under the conditions described in Example 16, may be selected as a substance having an activity of promoting a function of AGF. When a test substance is a compound, a compound having the above activity at a concentration of 10 µmol/L (preferably 1 µmol/L, more preferably 0.1 µmol/L) may be selected as a substance having an activity of promoting a function of AGF.

(4) Cell-binding-type screening method
   The cell-binding-type screening method of the present invention comprises the steps of:
   i) bringing a substance to be tested and AGF into contact with a cell, and
   ii) measuring an amount of the AGF which binds to the cell, and analyzing a test substance dependent change in the amount thereof,
      and, if desired, may further comprise the step of:
      analyzing effects of a candidate, which is selected in the above step ii) and has an activity of promoting a binding of AGF to a cell, on a change in body weight, a blood glucose level, and/or a lipid content in blood.

As shown in Example 17, it was found that AGF bound to the surface of a cell. The result indicates that a substance which promotes a binding of AGF to a cell may be used as an agent for promoting a function of AGF. According to the cell-binding-type screening method of the present invention, an agent for promoting a function of AGF can be obtained. The agent for promoting a function of AGF obtained by the cell-binding-type screening method of the present invention can be used as an active ingredient of an antiobesity agent, an antidiabetic agent, and/or a hypolipidemic agent.

As the cell used in the cell-binding-screening method of the present invention, a cell to which AGF binds is preferable, and there may be mentioned, for example, the above-mentioned chondrogenic cell line ATDC5.

An amount of AGF which binds to a cell may be measured by a known binding assay, such as a flow cytometry, a cell ELISA, or a binding assay by measuring an amount of labeled AGF which binds to a cell, preferably the flow cytometry described in Example 17.

In the cell-binding-type screening method of the present invention, an agent for promoting a binding of AGF to a cell, such as a substance in which the activity of increasing an amount of AGF which binds to a cell is 1.5 times or more (preferably 2 times or more, more preferably 4 times or more), for example, under the conditions described in Example 17, may be selected as a substance having an activity of promoting a function of AGF. When a test substance is a compound, a compound having the above activity at a concentration of 10 µmol/L (preferably 1 µmol/L, more preferably 0.1 µmol/L) may be selected as a substance having an activity of promoting a function of AGF.

(5) Method for confirming antiobesity activity, antidiabetic activity, and hypolipidemic activity
   In the screening method (including the AGF-type, promoter-type, MAPK-type, and cell-binding-type screening methods) of the present invention, if desired, an antiobesity activity, an antidiabetic activity, and/or a hypolipidemic activity of a candidate, which is selected in the above steps i) and ii) and has an activity of promoting an expression of AGF, an agonist activity of AGF, or an activity of promoting a binding AGF to a cell, can be confirmed, for example, by administering the candidate to an experimental animal and analyzing a change in body weight, a blood glucose level, and/or a lipid content in blood of the animal.

The antidiabetic activity of a candidate may be confirmed, for example, by administering the candidate to a small animal and analyzing a change in body weight, fat weight, or a change in fat weight in the animal. As the small animal, there may be mentioned, for example, a mouse or a rat, including a normal animal and an animal model for obesity. As the model for obesity, many available animal models, such as an ob/ob mouse, a db/db mouse, or a Zucker rat, are known. Further, a model for obesity in which an increase in body weight and/or an increase in fat weight are promoted by providing a load such as a high fat diet or a high calorie diet therefor may be used.

The activity of changing body weight may be analyzed, for example, by sequentially measuring body weight and comparing a change in body weight between a group treated with the candidate and a control group. Fat weight may be analyzed, for example, by dissecting genital fat pads from mice sacrificed under anesthesia, measuring the weight thereof, and comparing fat weight between a group treated with the candidate and a control group. A change in fat weight may be analyzed, for example, by obtaining computed tomographic images of the area from the diaphragm to the bottom of the abdominal cavity of the mice under anesthesia at intervals of 2 mm using an X-ray CT for laboratory animals (Aloka), determining the weights of visceral fat tissue and subcutaneous fat tissue, and comparing fat weight between a group treated with the candidate and a control group. These assays may be carried out in accordance with a method similar to that described in Referential Examples 4, 5, or 17, except that a candidate is administered to a normal animal or an animal model for obesity.

The antidiabetic activity of a candidate may be confirmed by administering the candidate to a small animal and analyzing a change in a blood glucose value in the animal. As the small animal, there may be mentioned, for example, a mouse or a rat, including a normal animal and an animal model for diabetes. As the model for diabetes, many available animal models, such as a KK/Ay mouse or a db/db mouse, are known. Further, a model for diabetes in which diabetes is induced by streptozotocin may be used. The effect of a candidate on a blood glucose value may be examined, by measuring a fed blood glucose value and/or a fasting blood glucose value and analyzing a change in blood glucose values between a group treated with the candidate and a control group. Further, glucose tolerance or insulin sensitivity may be examined in a group treated with a test substance and a control group, by measuring blood glucose values in a glucose tolerance test or an insulin resistance test. These assays may be carried out in accordance with a method similar to that described in Referential Examples 8, 16, or 18, except that a candidate is administered to a normal animal or an animal model for diabetes.

The hypolipidemic activity of a candidate may be confirmed by administering the candidate to a small animal and analyzing a change in a lipid content in blood of the animal. As the small animal, there may be mentioned, for example, a mouse, a rat, or a rabbit, including a normal animal and an animal model for hyperlipemia. As the model for hyperlipemia, many available animal models, such as a Zucker rat or a WHHL rabbit, are known. The effect of a candidate on a lipid content in blood may be examined, by measuring each concentration of blood triglyceride, blood cholesterol, and/or blood free fatty acids and analyzing a change in a lipid content in blood between a group treated with the candidate and a control group. These assays may be carried out in accordance with a method similar to that described in Referential Example 15, except that a candidate is administered to a normal animal or an animal model for hyperlipemia.

### EXAMPLES

The present invention now will be further illustrated by, but is by no means limited to, the following Examples. In this connection, the following procedures may be performed in accordance with known methods (for example, "Molecular Cloning", Sambrook, J. et al., Cold Spring Harbor Laboratory Press, 1989), unless otherwise specified. Further, when a commercially available reagent or kit is used, procedures may be performed in accordance with a protocol attached thereto.

Knockout animals and transgenic animals may be prepared in accordance with "Manipulating the Mouse Embryo. A Laboratory Manual." 2nd Edition, B. Hogan, R. Beddington, F. Costantini, E. Lacy, Cold Spring Harbor, New York, Cold Spring Harbor Laboratory Press, 1994, unless otherwise specified. Further, chimeric mice may be prepared by using ES cells in accordance with AL Joyner: "Gene Targeting, A Practical Approach", OXFORD UNIVERSITY PRESS, 1993; or Shinich Aizawa, Biomanual series 8, "Gene Targeting, ES saibou wo mochiita henimausu no sakusei", Yodosya, 1994, unless otherwise specified.

### Referential Example 1: Preparation of AGF KO mice

### (1) construction of targeting vector

A targeting vector containing a genomic sequence (5' long arm) at the 5' side of the mouse AGF gene, a pgk promoter, a neomycin resistant gene, a genomic sequence (3' short arm) containing a part of exon 2 and the whole of exon 3 in the mouse AGF gene, and an HSV-tk gene, in this order, was prepared in accordance with the following procedures.

A cDNA corresponding to the full-length of the coding region of mouse AGF protein was prepared by the procedures described in Example 1 of WO03/083114 The cDNA was used as a probe to screen a mouse genomic library (Mouse Genomic, 129 SVJ Library; Stratagene) in accordance with a manual attached thereto. A phage clone containing a sequence of approximately 17.9 kbp (corresponding to 90644 to 108544 in mouse-pub-genome sequence AC073775.2 containing the AGF gene) was isolated and subcloned into plasmid pBluescript (Stratagene). The obtained plasmid clone (pBN2) was digested with restriction enzymes SalI and MfeI to obtain the 5' long arm of approximately 6.2 kbp (containing 90664 to 96900 in mouse-pub-genome sequence ACO73775.2). Further, a PCR was carried out using the plasmid pBN2 as a template, together with a primer set (SEQ ID NO: 6 and SEQ ID NO: 7) to obtain the 3' short arm of approximately 2.0 kbp (containing 105903 to 107914 in mouse-pub-genome sequence AC073775.2). The 5' long arm and the 3' short arm were inserted into the XhoI site and the XbaI site of plasmid pPNT [Cell, 1991, 65(7), 1153-1163], respectively, to construct the targeting vector.

### (2) Preparation of homologous recombinant ES cells

The obtained targeting vector was digested with restriction enzyme NotI, and introduced into ES cell line R1 (Proceedings of the National Academy of Sciences, Vol 90, 8424-8428, 1993) by electroporation. The ES cells were cultured in a medium containing G418 to obtain resistant strains. DNAs were extracted from ES cells, and clones in which only a desired homologous recombination occurred were identified by Southern blotting. More particularly, each DNA was digested with restriction enzyme HindIII, and analyzed by Southern blotting using, as a probe, a DNA consisting of the nucleotide sequence of SEQ ID NO: 8 containing exon 4 and exon 5 in AGF. As a result, a DNA fragment of 6.5 Kb was detected in homologous recombinant clones, in comparison with that of 4.6 Kb in the wild-type.

### (3) Preparation of AGF KO mice

The obtained ES cell line was microinjected into blastocysts prepared from BDF2 mice which were F2 hybrid mice of C57BL/6 and DBA/2 mice, and the manipulated embryos were transferred to a uterus, to obtain chimeric mice from the pregnant mice. The chimeric mice were mated with C57BL/6 mice to obtain heterozygous mice (hereinafter referred to as AGF heterozygous KO mice) having a mutated allele lacking in the initiation codon of AGF. The AGF heterozygous KO mice were mated with each other to obtain homozygous mice (hereinafter referred to as AGF homozygous KO mice). A PCR using genomic DNA isolated from the tail of each offspring mouse as a template was carried out to confirm the genotype thereof from the size of each DNA fragment obtained by the PCR, as described below. That is, the tail was treated with proteinase K, and a phenol/chloroform extraction was carried out to obtain DNA. The extracted DNA was collected by an isopropanol precipitation followed by an ethanol precipitation, and dissolved in a Tris-EDTA buffer (hereinafter referred to as TE solution). The following primers were designed on the basis of a sequence of the neomycin resistant gene and a genomic sequence to be deleted by targeting:
(neomycin resistant gene)
   Forward primer: SEQ ID NO: 9
   Reverse primer: SEQ ID NO: 10
(genomic DNA)
   Forward primer: SEQ ID NO: 11
   Reverse primer: SEQ ID NO: 12

The genomic DNA obtained from each offspring mouse was used together with the above primers and a DNA polymerase (ExTaq; Takara) to perform PCRs. In the PCRs, a thermal denature at 95°C for 5 minutes was carried out, a cycle composed of reactions at 95°C for 1 minute, at 60°C for 1 minute, and at 72°C for 1 minute was repeated 30 times, and an elongation reaction at 72°C for 7 minutes was carried out. The sizes of fragments amplified by the PCRs were analyzed. When an offspring mouse has the mutated allele, a band of 545 bp is detected in the PCR for detecting the neomycin resistant gene. When an offspring mouse has the wild-type allele, a band of 322 bp is detected in the PCR for detecting the genomic DNA containing mouse AGF exon 1. The genotype of each mouse was determined from the results. As a result, in the AGF homozygous KO mice, the band of the mutated allele was detected, but the band of the wild-type allele was not detected. In the AGF heterozygous KO mice, the band of the mutated allele and that of the wild-type allele were detected. In the wild-type mice (hereinafter referred to as littermate WT mice), the band of the mutated allele was not detected, but the band of the wild-type allele was detected.

Further, the genotype of each mouse was analyzed by Southern blotting described in Referential Example 1(2). As a result, in the AGF homozygous KO mice, a band of 6.5 kbp derived from the mutated allele was detected. In the AGF heterozygous KO mice, a band of 6.5 kbp derived from the mutated allele and that of 4.6 kbp derived from the wild-type allele were detected. In the littermate WT mice, the band of 4.6 kbp derived from the wild-type allele was detected.

Furthermore, each blood sample collected from the AGF KO mice was allowed to stand at 37°C for 30 minutes, and centrifuged to obtain a serum as the supernatant. The serum was diluted to 1/20 with a lysis buffer [0.5 mol/L HEPES (pH7.2), 1% Triton X-100, 10% glycerol, 10 mmol/L Na₄P₂O₇, 0.1 mol/L NaF, 0.1 mmol/L Na₃VO₄, 4 mmol/L EDTA (pH 8), 0.05 mg/mL aprotinin, 1 mmol/L PMSF, 0.1 mmol/L leupeptin, and 0.025 mmol/L Pepstatin A], and further diluted with an equal volume of a 2×SDS sample buffer. Each sample (20 µL per lane) was subjected to 10% acrylamide gel electrophoresis, followed by Western blotting. In the Western blotting, TBS-T [20 mmol/L Tris-HCl (pH 7.5), 150 mmol/L NaCl, and 0.05%(w/v) Tween 20] containing 5% bovine serum albumin (BSA) was used as a blocking agent, an anti-mouse AGF antibody (WO03/083114) was used as the primary antibody, and an anti-rabbit antibody (ALI3404; BIO SourCE) diluted to 1/5000 with TBS-T containing 3% BSA was used as the second antibody. The AGF band was not detected in the AGF homozygous KO mice, to confirm a deficiency of AGF.

### Referential Example 2: Preparation of CAG-AGF Tg mice

In this example, AGF transgenic mice (hereinafter referred to as CAG-AGF Tg mice) in which mouse AGF was systemically overexpressed under the control of a CAG (modified chicken beta-actin promoter with CMV-IE enhancer) promoter [GENE, 108(1991) 193-200] were prepared. A plasmid in which the CAG promoter (1.7 kb), a lox71 sequence, a blasticidin gene (bsr), a poly A signal sequence (0.5 kb), a lox P sequence, a mouse AGF cDNA sequence, and an IRES (internal ribosomal entry site)-β-geo-poly A sequence (4.5 kb) were inserted at the multicloning site of plasmid pBluescriptII KS(+) (Stratagene) in this order was prepared in accordance with the following procedures.

The full-length of mouse AGF cDNA (WO03/083114) was used as a template, together with a primer set (SEQ ID NO: 13 and SEQ ID NO: 14) to carry out a PCR. In the PCR, a reaction at 95°C for 10 minutes was carried out, and a cycle composed of reactions at 94°C for 15 seconds, at 60°C for 30 seconds, and at 72°C for 2 minutes was repeated 45 times. The obtained PCR product was subcloned into a pZErO-2 cloning vector (Invitrogen). The obtained plasmid was digested with restriction enzymes HindIII and SalI, and inserted between the HindIII and SalI sites of plasmid pBluescriptII SK (Stratagene) to construct plasmid pBS-mAGF containing the full-length of mouse AGF gene. To introduce the IRES-β-geo-poly A gene into the plasmid pBS-mAGF, plasmid pU-San (Hum Mol Genet 8:387-396 1999) carrying the IRES-β-geo-poly A gene was digested with restriction enzymes SalI and BglII, and the obtained IRES-β-geo-poly A gene was inserted between the BglII and SalI sites of pBS-mAGF to construct plasmid pBS-mAGF-βgeo containing the mouse AGF cDNA sequence and the IRES-β-geo-poly A sequence.

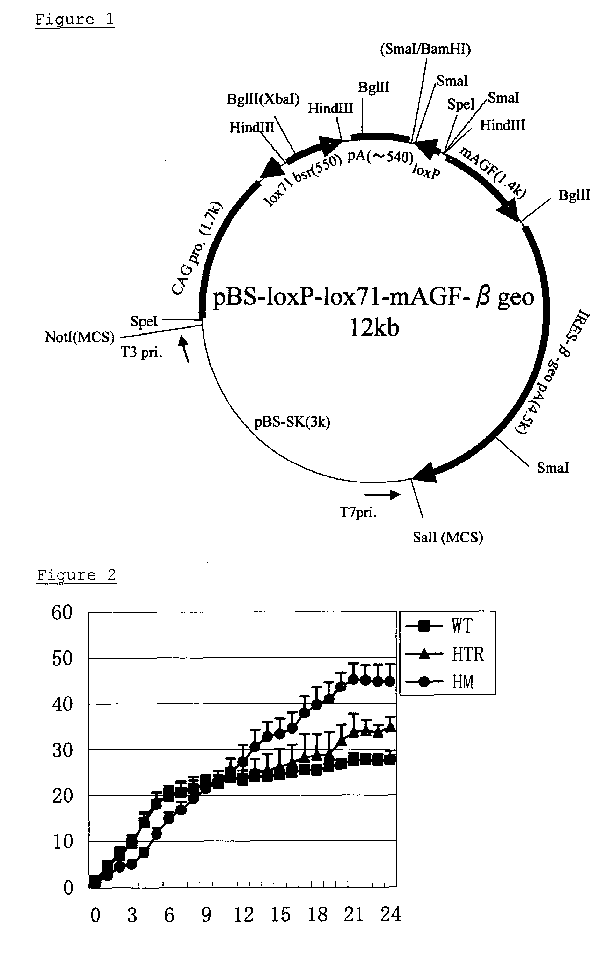

A plasmid in which bsr and the poly A signal sequence are interposed between the lox71 sequence and the loxP sequence by inserting the loxP sequence into the 3' side of the poly A signal of plasmid pCAGlox71bsr [Nucleic Acids Res. 1997; 25(4): 868-872] carrying the CAG promoter, the lox71 sequence, bsr, and the poly A signal sequence, was constructed as described below. That is, a phosphorylated fragment of 81 bp (SEQ ID NO: 15) carrying loxP was inserted into the plasmid pCAGlox71bsr, which had been previously digested with restriction enzyme SmaI and had been treated with BAP (bacterial alkaline phosphatase), to construct plasmid loxP-lox71. To confirm the loxP sequence inserted in the plasmid loxP-lox71, a nucleotide sequence containing the inserted loxP sequence was sequenced using a T3 primer (SEQ ID NO: 16). As a result, it was confirmed that the nucleotide sequence determined by the T3 primer accorded with that of SEQ ID NO: 15, and the loxP and lox71 sequences have the same direction. The plasmid loxP-lox71 was digested with restriction enzyme SpeI, and the obtained fragment containing the CAG promoter, the lox71 sequence, bsr, the poly A signal sequence, and the loxP sequence was inserted at the SpeI site of plasmid pBS-mAGF-βgeo to construct the desired plasmid pBS-loxP-lox71-mAGF-βgeo. The structure of the plasmid pBS-loxP-lox71-mAGF-βgeo is shown in Figure 1. The plasmid pBS-loxP-lox71-mAGF-βgeo was digested with restriction enzyme NotI to obtain a linearized DNA fragment containing the CAG promoter, the lox71 sequence, bsr, the poly A signal sequence, the loxP sequence, the mouse AGF cDNA sequence, and the IRES-β-geo-poly A sequence (pA).

The linearized DNA fragment was introduced into TT2 ES cells [Anal. Biochem., 1993, 214(1): 70-76] by electroporation (0.8 V, 3 µF). The ES cells were cultured in the presence of 4 µg/mL blasticidin, and cells in which the genes were introduced were selected to establish 20 clones. A CAG-Cre vector (Blood, 1326-1333, Vol.100, 2002) as a circular plasmid was introduced into each clone by electroporation (0.8 V, 3 µF).

When Cre recombinase expressed by the CAG promoter excises the gene between lox71 and loxP, AGF and β-geo will be expressed by the CAG promoter activity. To select clones expressing β-geo, the ES cells were cultured in the presence of G418 (200 µg/mL) to select cells having the genetic structure in which the region between lox71 and loxP was deleted. ES cells (15 clones) selected at this stage have the CAG promoter (1.7 kb), the lox71 sequence, the mouse AGF cDNA sequence, and the IRES-β-geo-poly A sequence (4.5 kb), and express AGF constitutively under the control of the CAG promoter. Each original clone before introducing the CAG-Cre vector (i.e., ES cells in which the region between lox71 and loxP was not deleted) was used as a negative control to confirm that AGF was expressed in the selected 15 clone by Western blotting using the anti AGF antibody described in Referential Example 1(3). That is, ES cells were lysed with the lysis buffer, followed by an equal volume of the 2×SDS sample buffer, the obtained samples were subjected to Western blotting, and the expression of AGF was confirmed. From the ES cells expressing AGF, three lines (8-1, 8-2, and 9-1) were selected. The selected cell lines were microinjected into blastocysts, and the manipulated embryos were transferred to a uterus to obtain chimeric mice from the pregnant mice. The chimeric mice were mated with C57BL/6 mice to obtain transgenic mice expressing AGF constitutively under the control of the CAG promoter. To identify the transgenic mice, a PCR using genomic DNA isolated from the tail of each offspring mouse as a template was carried out, as described below. That is, the tail was treated with proteinase K, and a phenol/chloroform extraction was carried out to obtain DNA. The extracted DNA was collected by an isopropanol precipitation followed by an ethanol precipitation, and dissolved in a TE solution.

The following primers were designed on the basis of the mouse AGF cDNA sequence and a LacZ sequence:
(AGF)
   Forward primer: SEQ ID NO: 17
   Reverse primer: SEQ ID NO: 18
(LacZ)
   Forward primer: SEQ ID NO: 19
   Reverse primer: SEQ ID NO: 20

In PCRs using these primers, fragments of 325 bp and 320 bp are amplified from the introduced gene in PCRs for detecting the AGF cDNA and for detecting LacZ, respectively, and such fragments are not amplified from the mouse genomic DNA. The above primers and each genomic DNA prepared from the offspring mice were used to perform PCRs using a DNA polymerase (ExTaq; Takara). In the PCRs, a thermal denature at 94°C for 5 minutes was carried out, a cycle composed of reactions at 94°C for 1 minute, at 62°C for 1 minute and 30 seconds, and at 72°C for 1 minute and 30 seconds was repeated 28 times, and an elongation reaction at 72°C for 7 minutes was carried out. The sizes of fragments amplified by the PCRs were analyzed. As a result, the expected bands were detected in both PCRs for detecting the AGF cDNA and LacZ, with respect to the three lines. The result shows that a germ line transmission occurred in the three lines, and that the three lines are transgenic mice expressing AGF constitutively under the control of the CAG promoter.

### Referential Example 3: Expression of AGF gene in CAG-AGF Tg mouse

In this example, the degree of the AGF gene expressed in the CAG-AGF Tg mouse prepared in Referential Example 2 was analyzed. Total RNAs were prepared from the CAG-AGF Tg mouse and the littermate WT mouse [white adipose tissue (WAT), brown adipose tissue (BAT), cerebrum, cerebellum, hypothalamus, heart, liver, kidney, spleen, skeletal muscle, and pancreas] using a trizol reagent (Invitrogen). A commercially available RNA purification reagent (RNeasy; Qiagen) and DNase (Qiagen) were used to perform a DNase treatment and cleanup of the total RNAs. After the DNase treatment, 0.5 µg of the total RNAs was converted to cDNAs using superscript first-strand system for RT-PCR (LIFE TECHNOLOGIES).

Expression amounts of AGF and 18S ribosomal RNA (18SrRNA) were determined by a quantitative PCR method. The 18SrRNA was used as an internal standard. The quantitative PCR was carried out by measuring an amount of real-time fluorescence using a sequence detection system (ABI PRISM 7900HT Sequence Detection System; Applied Biosystems). The above cDNAs were used as a template, and the following primers and a Taq Man probe designed for each gene were used as primers. Primers (SEQ ID NO: 21 and SEQ ID NO: 22) and a commercially available PCR reagent (SYBR Green PCR Master Mix; Applied Biosystems) were used to carry out a PCR for measuring an amount of the AGF gene expressed. Primers (SEQ ID NO: 23 and SEQ ID NO: 24), a Taq Man probe (SEQ ID NO: 25), and a commercially available PCR reagent (TaqMan Universal PCR Master Mix; Applied Biosystems) were used to carry out a PCR for measuring an amount of the 18SrRNA expressed.

In the PCR, an initial denaturing reaction at 95°C for 10 minutes was carried out, and a cycle composed of reactions at 94°C for 15 seconds and at 60°C for 60 seconds was repeated 45 times. Standard curves for calculating amounts of genes expressed were prepared by using the above cDNAs or mouse genomic DNA as a template. Amounts of genes expressed were calculated as relative values between samples.
As a result, it was revealed that an amount of the AGF gene expressed in tissues of the CAG-AGF Tg mouse was increased in comparison with that in tissues of the WT mouse. In particular, remarkably increased expressions were observed in skeletal muscle, BAT, and heart.

### Referential Example 4: Changes in body weight of genetically modified mice

### (1) Changes in body weight of CAG-AGF Tg mice

Two generations of backcrosses of the CAG-AGF Tg mouse with C57BL/6 were carried out to obtain CAG-AGF Tg mice (F2). The CAG-AGF Tg mice and the littermate WT mice were normally bred with a normal diet (CE-2; CLEA). When the body weights of 6 weeks old female mice were measured and compared, those of the CAG-AGF Tg mice and the WT mice were 15.7 g ± 0.8 g (SD) and 17.8 g ± 0.5 g (SD), respectively. When those of 12 weeks old female mice were measured and compared, those of the CAG-AGF Tg mice and the WT mice were 19.5 g ± 0.7 g (SD) and 23.5 g ± 2.5 g (SD), respectively. It was found from the results that the body weight of the CAG-AGF Tg mouse was lighter than that of the WT mouse.

Changes in body weight when the above mice were bred with a high fat diet were examined. The mice were normally bred with a high fat diet (high fat diet-32; CLEA) for 12 weeks, and changes in body weight were analyzed. As a result, the amounts of body weight increase for 12 weeks were 7.1 g ± 1 g (SD) in CAG-AGF Tg mice and 21.8 g ± 4 g (SD) in WT mice, respectively. The results show that when the CAG-AGF Tg mice were bred with a normal diet, an increase in body weight was suppressed, and that when the CAG-AGF Tg mice were bred with a high fat diet, an increase in body weight was remarkably suppressed. From the results, it was found that AGF exhibits an activity of suppressing an increase in body weight.

### (2) Changes in body weight of AGF KO mice

The AGF homozygous KO mice, the AGF heterozygous KO mice, and the littermate WT mice were normally bred with a normal diet. Each body weight was measured every week until the mice were 24 weeks old. The results are shown in Figure 2. As shown in Figure 2, the body weight of the AGF homozygous KO mice was higher than that of the WT mice from approximately 12 weeks old, and an increase in body weight of the AGF homozygous KO mice continued and the mice became remarkably obese. The AGF heterozygous mice exhibited an intermediate phenotype between those of the AGF homozygous KO mice and the littermate WT mice.

### Referential Example 5: Changes in tissue weight of genetically modified mice

### (1) Changes in tissue weight in CAG-AGF Tg mice

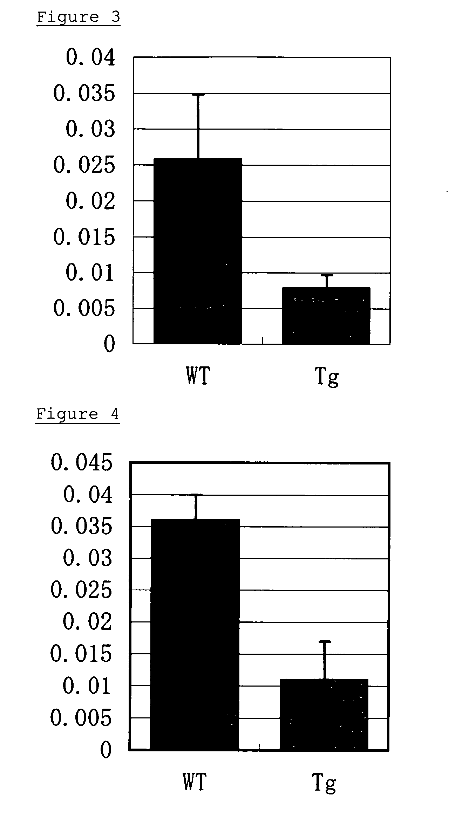

As described above, it was found that an increase in body weight was suppressed in the CAG-AGF Tg mouse. In this example, the weights of various organs were measured to reveal the mechanism. Each tissue [genital fat pads (WAT), brown adipose tissue (BAT), liver, heart, kidney, and spleen] was obtained from the CAG-AGF Tg mice and the littermate WT mice, and the weight of each tissue per body weight was measured. The measurement was carried out using 12 weeks old mice bred with a normal diet and mice bred with a high fat diet for 12 weeks (from 12 weeks old to 24 weeks old). As a result, no changes were observed in BAT, liver, heart, kidney, and spleen between the mice (i.e., CAG-AGF Tg mice and WT mice) bred with a normal diet or a high fat diet. In contrast, the weight per body weight of genital fat pads (white adipose tissue) in the CAG-AGF Tg mice bred with a normal diet or a high fat diet was decreased in comparison with that in the WT mice. The results show that, in the CAG-AGF Tg mouse, an increase in the weight of WAT was suppressed, and thus, an increase in the body weight thereof was suppressed. That is, it was found that AGF does not act on the weights of tissues other than WAT, but suppresses an increase in the weight of adipose tissue accompanied by obesity. The results in WAT are shown in Figure 3 (normal diet) and Figure 4 (high fat diet), respectively.

### (2) Changes in tissue weight in AGF KO mice

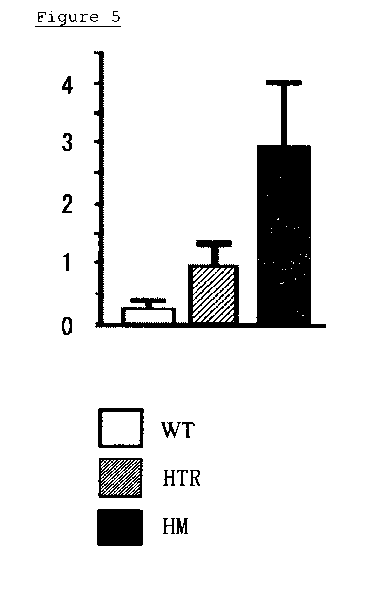

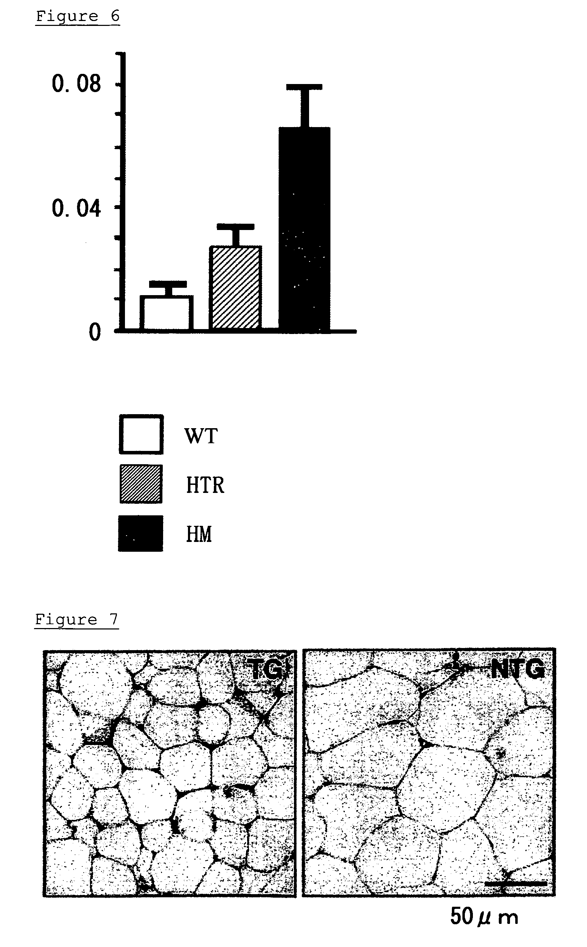

As described above, it was found that the body weight was increased in the AGF KO mouse. In this example, the weights of various tissues were measured to reveal the mechanism. Each tissue [genital fat pads (WAT), brown adipose tissue (BAT), liver, heart, kidney, and spleen] was obtained from 20 weeks old female AGF homozygous KO mice, AGF heterozygous mice, and littermate WT mice bred with a normal diet, and the weight of each tissue per body weight was measured. As a result, no changes were observed in BAT, liver, heart, kidney, and spleen among the AGF homozygous KO mice, the AGF heterozygous mice, and the littermate WT mice. In contrast, the weight per body weight or per mouse of genital fat pads in the AGF homozygous mice was increased in comparison with that in the WT mice. The AGF heterozygous mice exhibited tissue weights intermediate between those of the AGF homozygous KO mice and the WT mice. The results show that, in the AGF KO mouse, the weight of WAT such as genital fat pads was increased, and thus, the body weight thereof was increased. That is, it was found that the AGF KO mouse exhibits phenotypes opposite to the CAG-AGF Tg mouse, and that AGF does not act on the weights of tissues other than WAT, but suppresses an increase in the weight of adipose tissue. The results in genital fat pads (WAT) are shown in Figure 5 (weight of WAT) and Figure 6 (weight of WAT/body weight), respectively.

### Referential Example 6: Changes in form of adipocytes in genetically modified mice

### (1) Changes in form of adipocytes in CAG-AGF Tg mice

It is known that a high fat diet increases the weight of WAT and hypertrophy of adipocytes. It is known that the hypertrophy of adipocytes is involved in deteriorating diabetes [IGAKU NO AYUMI, 192, 513-518, 2000; and IGAKU NO AYUMI, 192, 541-545, 2000]. Therefore, the forms of adipocytes in the CAG-AGF Tg mice were analyzed in this example, as described below. Each fat tissue was obtained from CAG-AGF Tg mice and littermate WT mice bred with a high fat diet for 12 weeks from 12 weeks old to 24 weeks old. Each tissue was fixed with a 10% formalin neutral buffer solution (Wako) and embedded in paraffin. Sliced sections were prepared and a hematoxylin and eosin (H&E) stain was carried out. The result is shown in Figure 7. In the littermate WT mouse (NTG), adipocytes became hypertrophied. In the CAG-AGF Tg mouse (TG), the hypertrophy of adipocytes was suppressed and the sizes thereof were maintained as a normal size.

### (2) Changes in form of adipocytes in AGF KO mice

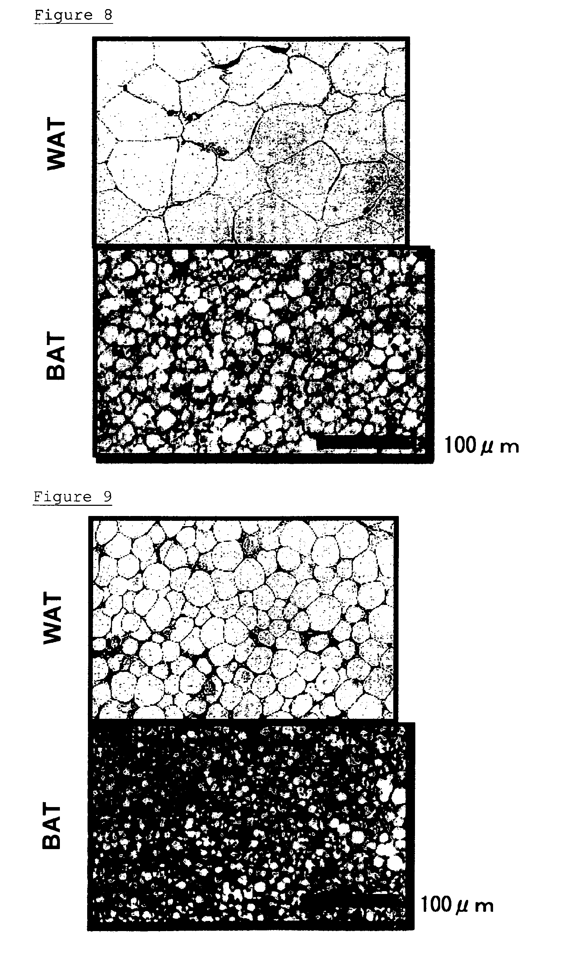

It is known that adipocytes become hypertrophied in model mice for diabetes or obesity, accompanied by an increase in the weight of adipocytes [Diabetologia, 14(3), 141-148, 1978]. It is known that the hypertrophy of adipocytes is involved in deteriorating diabetes, and thus, the forms of adipocytes in the AGF KO mice were analyzed in this example, as described below. Each genital fat pads (WAT) and brown fat tissue (BAT) were obtained from the AGF homozygous mice and the littermate WT mice. Each tissue was fixed with a 10% formalin neutral buffer solution and embedded in paraffin. Sliced sections were prepared and a hematoxylin and eosin (H&E) stain was carried out. The results are shown in Figure 8 (AGF homozygous KO mouse) and Figure 9 (littermate WT mouse), respectively. It was found that adipocytes in WAT of the littermate WT mice had a normal size, and that adipocytes in WAT of the AGF homozygous KO mice became hypertrophied. Further, an accumulation of fat in BAT of the AGF homozygous KO mice was observed in comparison with that of the littermate WT mice.

### Referential Example 7: Changes in triglyceride content in tissues of genetically modified mice

### (1) Changes in triglyceride content in tissues of CAG-AGF Tg mice

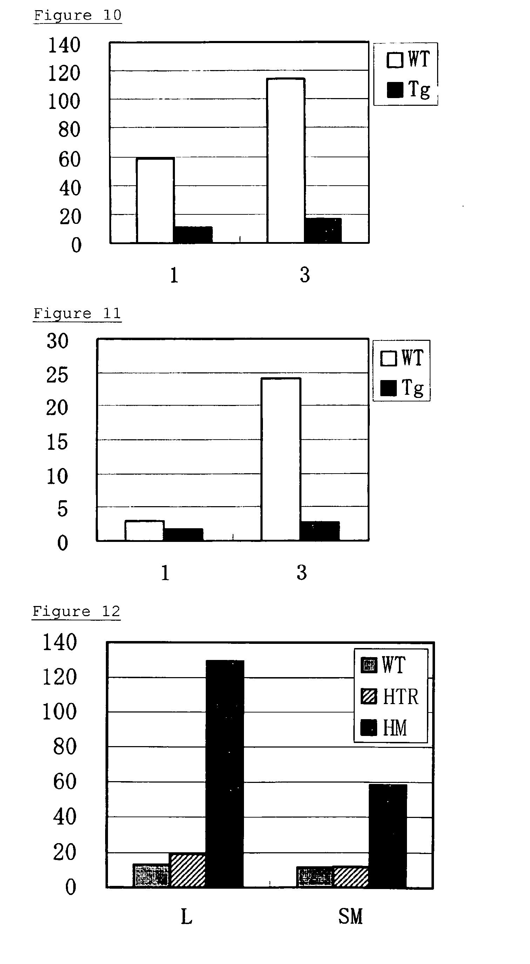

It is known that obesity causes not only an increase in fat tissues, but also an increase in triglyceride (TG) content in skeletal muscles or the liver (Nippon Rinsho, 1995, vol. 53, Special Issue in 1995, Himansho, p. 354-p358). In this example, TG content in skeletal muscles (gastrocnemial muscles) and the liver of the CAG-AGF Tg mice were analyzed, as described below. The CAG-AGF Tg mice (Tg) and the littermate WT mice (WT) were bred with a high fat diet (high fat diet 32; CLEA) for a month or three months. A chloroform-methanol solution was used to extract TG from skeletal muscles and the liver of each mouse (Seikagakujikkenkouza 3, Shishitsunokagaku, Tokyo Kagaku Dozin). A concentration of each extracted TG was measured using a kit (Triglyceride E test Wako; Wako) to determine TG content in the tissues. The results are shown in Figure 10 (liver) and Figure 11 (skeletal muscles), respectively. It was found that each TG content in tissues (skeletal muscles or the liver) of the CAG-AGF Tg mice was decreased in a comparison with that in the WT mice. It was found that AGF exhibits an activity of decreasing TG content in skeletal muscles or the liver. In this connection, it is known that the TG content in skeletal muscles or the liver is increased by obesity.

### (2) TG content in tissues of AGF KO mice

TG content in skeletal muscles (gastrocnemial muscles) and the liver of the AGF KO mice was analyzed. The method described in Referential Example 7(1) was repeated, except that the AGF KO mice and the littermate WT mice were used, to extract TG therefrom. A concentration of each extracted TG was measured using a kit (Triglyceride E test Wako; Wako) to determine TG content in the tissues. The results are shown in Figure 12. It was found that TG content in skeletal muscles and the liver of the AGF KO mice was remarkably increased in comparison of that in the WT mice. It was found that the AGF KO mouse exhibits phenotypes opposite to the CAG-AGF Tg mouse, and that AGF exhibits an activity of decreasing TG content in skeletal muscles or the liver.

### Referential Example 8: Changes in blood glucose level and blood insulin concentration in AGF KO mice

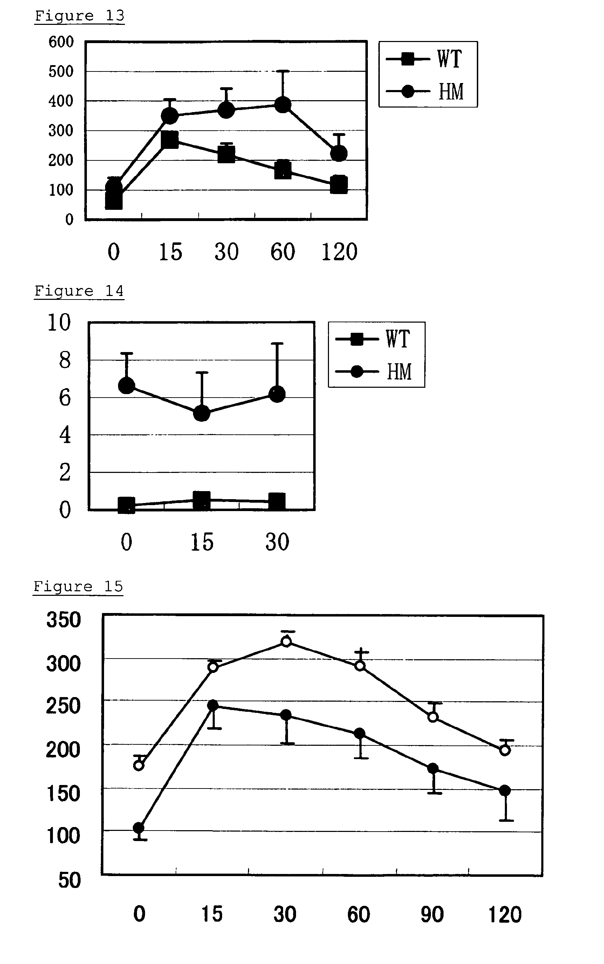

A glucose tolerance test for the AGF homozygous KO mice and the littermate WT mice was carried out to analyze a blood glucose level and a concentration of blood insulin, as described below. The mice were made to fast for 16 hours, and 1 g/kg of D-glucose was intraperitoneally administered. Blood was taken from ophthalmic veins before the administration, and at 15, 30, 60, and 120 minutes after the administration. The blood glucose level was measured using GLUTEST ACE (SANWA KAGAKU KENKYUSHO), and the concentration of blood insulin was measured using a RIA2 antibody method (SRL). The results are shown in Figure 13 (blood glucose value) and Figure 14 (insulin in plasma), respectively.

In the WT mice, the blood glucose level increased by the glucose administration began to decrease at 30 minutes after the administration. In the AGF homozygous mice, the blood glucose level was greatly increased by the glucose administration, and the elevated blood glucose value did not begin to decrease at 60 minutes after the administration. It was found from the results that the AGF homozygous KO mice exhibited an abnormality in glucose tolerance. With respect to the concentration in blood insulin, it was increased by the glucose administration in the WT mice, whereas the concentration of blood insulin in the AGF homozygous KO mice was remarkably high before the glucose administration. This shows that the AGF homozygous KO mice suffered from hyperinsulinemia. From the results, it was found that the AGF homozygous KO mouse deficient in the AGF gene suffered from diabetes, and that AGF exhibits an antidiabetic activity.

### Referential Example 9: Expression and purification of mouse AGF and human AGF

The human AGF and the mouse AGF were expressed and purified in accordance with the procedures described in WO03/083114 (Example 19) as described below. That is, DNA fragments of approximately 1.4 kbp (human) and approximately 1.3 kbp (mouse) were independently inserted into plasmid pcDNA-Signal-FLAG. Each resulting expression plasmid was introduced into HEK293 cells. Each culture supernatant of the cells expressing the human AGF or the mouse AGF was purified by affinity chromatograph using anti FLAG-M2 monoclonal antibody agarose affinity gel (Sigma) to obtain human and mouse recombinant AGF proteins.

### Referential Example 10: Preparation of adenovirus expressing mouse AGF

Plasmid pCR2.1-mNew (prepared in Example 1 of WO03/083114) containing the full-length of mouse AGF cDNA was used as a template, together with a forward primer (SEQ ID NO: 45) and a reverse primer (SEQ ID NO: 46) and a pfu DNA polymerase, to carry out a PCR. In the PCR, a reaction at 95°C for 10 minutes was carried out, and a cycle composed of reactions at 94°C for 15 seconds, at 60°C for 30 seconds, and at 72°C for 2 minutes was repeated 45 times. The resulting PCR product was subcloned into pCR4Blunt-TOPO (Invitrogen). The obtained plasmid was digested with restriction enzymes XbaI and NotI, and inserted between the XbaI and NotI sites of C-terminal-FLAG-addition-type expression vector pCEP4dE2-FLAG (prepared in accordance with Example 2 of WO02/42448) to obtain pCEPdE2-mAGF-FLAG. Plasmid pCEPdE2-mAGF-FLAG was used as a template, together with a forward primer (SEQ ID NO: 47) and a reverse primer (SEQ ID NO: 48) and a PyroBest DNA polymerase, to carry out a PCR. In the PCR, a reaction at 95°C for 2 minutes was carried out, a cycle composed of reactions at 98°C for 20 seconds, at 60°C for 30 seconds, and at 72°C for 1.5 minutes was repeated 20 times, and a reaction at 72°C for 7 minutes was carried out. The resulting PCR product was subcloned into pCR4Blunt-TOPO (Invitrogen). The obtained plasmid was digested with restriction enzymes BglII and XhoI, and the obtained DNA fragment of approximately 1.4 kbp was inserted between the BglII and XhoI sites of a shuttle vector pAdTrack-CMV for preparing adenovirus (Proc. Natl. Acad. Sci. USA 95:2509-2514, 1998) to construct pAdTracK-CMV-mAGF. This plasmid or a control vector pAdTrack-CMV (empty vector) was used to prepare viral particles in accordance with the following procedure.

Escherichia coil BJ5183 (stratagene) was transformed with plasmid pAdEasy-1 (He T.-C. et al., Proc. Natl. Acad. Sci. USA, 95, 2509-2514, 1998) to prepare Escherichia coil BJ5183 containing plasmid pAdEasy-1 [hereinafter referred to as Escherichia coil BJ5183 (pAdEasy-1)]. The previously prepared plasmid pAdTracK-CMV-mAGF or pAdTrack-CMV was digested with restriction enzyme PmeI, and used to transform Escherichia coil BJ5183 (pAdEasy-1). To select a clone in which plasmid pAdEasy-1 and plasmid pAdTracK-CMV-mAGF or pAdTrack-CMV were recombined, plasmids were prepared from obtained transformants, and digested with restriction enzyme PacI to analyze the digestion patterns. Clones in which an extra band of 3 kb or 4.5 kb was observed were selected to obtain desired clones in which the arm regions of the plasmids were recombined.
Concentrations of viral particles were determined in accordance with a method similar to that described in Example 15 of WO02/42448.

### Referential Example 11: Expression of mouse AGF protein using adenovirus

A human fetal kidney cell line 293 (ATCC No.: CRL-1573) was infected with the adenovirus expressing the mouse AGF protein or the control adenovirus pAdTrack-CMV prepared in Referential Example 10 (100 viral particles/cell). More particularly, 3×10⁵ of 293 cells were seeded into wells of a 6-well plate (ASAHI TECHNO GLASS) coated with type I collagen, and cultured overnight in 2 mL of a DMEM medium supplemented with 10% FBS, 100 IU/mL penicillin, and 100 µg/mL streptomycin. Adenoviral particles (6×10⁷ particles) were added, and further cultured overnight. The medium was exchanged with 2 mL of a DMEM medium supplemented with 100 IU/mL penicillin and 100 µg/mL streptomycin. After two days from the infection, each culture supernatant was collected and centrifuged at 3,000 rpm for 5 minutes. Each supernatant was subjected to an SDS-polyacrylamide gel electrophoresis (SDS-PAGE) using a gradient gel (gel concentration = 4-20%; Daiich Pure Chemicals). Western blotting was carried out using an anti-mouse AGF antibody (prepared in Example 5 of WO03/083114) as the primary antibody and an goat anti-rabbit IgG polyclonal antibody labeled with horseradish peroxidase (Biosource) as the second antibody. When the supernatant derived from cells infected with the adenovirus expressing the mouse AGF protein was used as a sample, a band was detected at the position of the deduced molecular weight of the mouse AGF, but no band was detected when the supernatant derived from cells infected with the control adenovirus was used. As a result, it was found that the adenovirus expressing the mouse AGF protein, prepared in Referential Example 10, was used to express the mouse AGF protein by the method described in Referential Example 11.

### Referential Example 12: Preparation of adenovirus expressing human AGF

Plasmid containing the full-length of human AGF cDNA (prepared in Example 18 of WO03/083114; hereinafter referred to as pCR2.1-hNew) was used as a template, together with a forward primer (SEQ ID NO: 49) and a reverse primer (SEQ ID NO: 50) and a PyroBest^{™} DNA polymerase (Takara), to carry out a PCR. In the PCR, a cycle composed of reactions at 98°C for 20 seconds, at 64°C for 30 seconds, and at 74°C for 3 minutes in the presence of 5% formamide was repeated 35 times. The resulting PCR product was subcloned into pCR4Blunt-TOPO (Invitrogen). The obtained plasmid was digested with restriction enzymes HindIII and XhoI, and the obtained DNA fragment was inserted between the HindIII and XhoI sites of pCEP4 (Invitrogen) to obtain pCEP-hAGF. A DNA fragment of approximately 0.43 kbp obtained by digesting pCEP-hAGF with restriction enzymes KpnI and MluI, and a DNA fragment of approximately 0.98 kbp obtained by digesting an expression plasmid containing the full-length of human AGF cDNA (prepared in Example 19 of WO03/083114; hereinafter referred to as pcDNA-SF-hAGF) with restriction enzymes MluI and XhoI were inserted between the KpnI and XhoI sites of a shuttle vector pAdTrack-CMV for preparing adenovirus (Proc. Natl. Acad. Sci. USA 95:2509-2514, 1998) to construct pAdTracK-CMV-hAGF. This plasmid or a control vector pAdTrack-CMV was used together with a pAdEasy system to prepare viral particles in accordance with methods similar to those described in Examples 14 and 15 of WO02/42448. Concentrations of viral particles were determined in accordance with a method similar to that described in Example 15 of WO02/42448.

### Referential Example 13: Expression of human AGF protein using adenovirus

In accordance with the method similar to that described in Referential Example 11, a human fetal kidney cell line 293 was infected with the adenovirus expressing the human AGF protein prepared in Referential Example 12 or the control adenovirus pAdTrack-CMV prepared in Referential Example 10. The method described in Referential Example 11 was repeated, except that the anti-SF-YP-030 IgG prepared in Example 1 was used as the primary antibody, to detect the expression of the human AGF protein in each culture supernatant. As a result, when the supernatant derived from cells infected with the adenovirus expressing the human AGF protein was used as a sample, a band was detected at the position of the deduced molecular weight of the human AGF, but no band was detected when the supernatant derived from cells infected with the control adenovirus was used. It was found that the adenovirus expressing the human AGF protein, prepared in Referential Example 12, was used to express the human AGF protein by the method described in Referential Example 13.

### Referential Example 14: Administration of adenovirus expressing mouse AGF to mice bred with high fat diet

Female C57BL/6 mice (CLEA) were bred with a high fat diet (high fat diet 32; CLEA) from 8 weeks old. The body weight was increased in the mice bred with a high fat diet, and the mice became obese (approximately 54 g) in 38 weeks old, from which adenovirus was administered. The adenovirus expressing the mouse AGF (mAGF-Adeno) or the control adenovirus (Cont-Adeno) prepared in Referential Example 10 was intravenously administered to mice (each group consisting of four mice) at a dose of 5×10⁹ PFU/mouse/week to the tails thereof. After 12 days from the first administration of each adenovirus, the body weight in the mAGF-Adeno administered mice was decreased by 7.3 g on average, and that in the Cont-Adeno administered mice was decreased by 1.4 g on average. It was found that, when the virus containing the AGF gene was administered to the mAGF-Adeno administered mice, the AGF protein was expressed in the living body and the expressed AGF protein caused a decrease in body weight.

Further, these mice were used to carry out a glucose metabolism test. These mice were made to fast for 16 hours, and 0.4 g/kg of D-glucose was intraperitoneally administered. Blood was taken from ophthalmic veins before the administration, and at 15, 30, 60, 90, and 120 minutes after the administration. The blood glucose level was measured using GLUTEST ACE (SANWA KAGAKU KENKYUSHO). The result is shown in Figure 15.
In the mAGF-Adeno administered mice, the blood glucose level was lower than that in the Cont-Adeno administered mice, with respect to all measurement points after the glucose administration, and it was found that glucose tolerance was improved. From the result, it was clarified that glucose tolerance was improved by an action of the AGF protein highly expressed in the mAGF-Adeno administered mice.

### Referential Example 15: blood insulin and lipid concentrations in CAG-AGF Tg mice bred with high fat diet

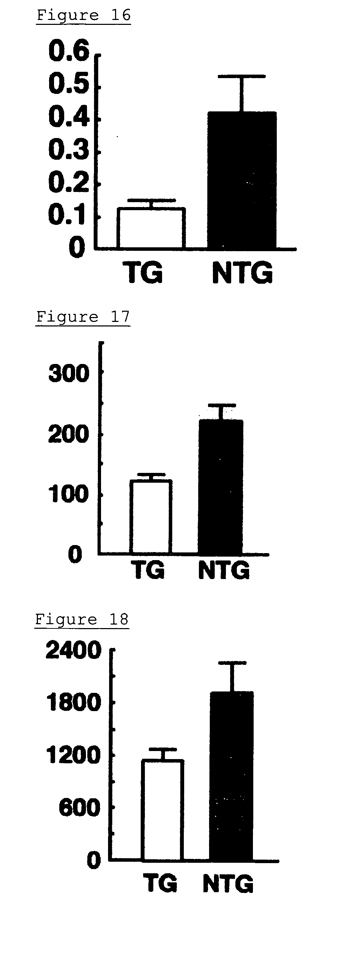

Six weeks old CAG-AGF Tg mice (prepared in Referential Example 2) and littermate WT mice (female) were bred with a high fat diet for 12 weeks. Blood was taken from ophthalmic veins. A concentration of insulin in plasma was measured using Insulin Immunoassay (Eiken Chemical), a concentration of cholesterol in serum was measured using L type Wako Cholesterol (Wako), and a concentration of free fatty acids in serum was measured using NEFA C-Test Wako (Wako), in accordance with protocols attached thereto. These results are shown in Figure 16 (plasma insulin concentration, ng/mL), Figure 17 (serum cholesterol concentration, mg/dL), and Figure 18 (serum free fatty acids concentration, µEq/L).
As a result, it was found that the insulin concentration, the cholesterol concentration, and the free fatty acids concentration in the CAG-AGF Tg mice (TG) were lower than those in the littermate WT mice (NTG). From the result, it was clarified that insulin sensitivity was increased and lipid metabolism was improved by an action of the AGF protein highly expressed in the CAG-AGF Tg mice.

### Referential Example 16: Glucose metabolism test in CAG-AGF Tg mice

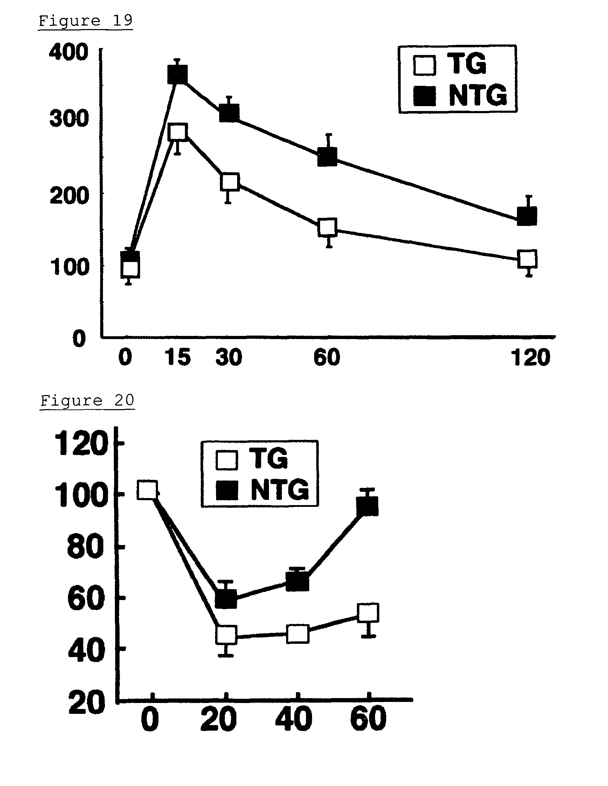

A glucose tolerance test was carried out using the CAG-AGF Tg mice (Referential Example 2) to evaluate the effect of the AGF on alleviating glucose tolerance. Four-month-old female CAG-AGF Tg mice and littermate WT mice were made to fast for 16 hours, and 1 g/kg of D-glucose was intraperitoneally administered. Blood was taken from ophthalmic veins before the administration, and at 15, 30, 60, and 120 minutes after the administration. The blood glucose level was measured using GLUTEST ACE (SANWA KAGAKU KENKYUSHO). The result is shown in Figure 19.
In the CAG-AGF Tg mice, the blood glucose level was lower than that in the littermate WT mice, with respect to all measurement points after the glucose administration, and it was found that glucose tolerance was improved. From the result, it was clarified that glucose tolerance was improved by an action of the AGF protein highly expressed in the CAG-AGF Tg mice.

Next, 0.75 units/kg of insulin was intraperitoneally administered to four-month-old female CAG-AGF Tg mice and littermate WT mice. Blood was taken from ophthalmic veins immediately after the insulin administration, and at 20, 40, and 60 minutes after the insulin administration. The blood glucose level was measured using GLUTEST ACE (SANWA KAGAKU KENKYUSHO). The ratio of the blood glucose level after the insulin administration to that before the insulin administration is shown in Figure 20.
In the CAG-AGF Tg mice, the blood glucose level was remarkably decreased in comparison with that in the littermate WT mice, with respect to all measurement points after the insulin administration, and it was found that insulin sensitivity was increased. It was clarified from the result that insulin sensitivity was increased by an action of the AGF protein highly expressed in the CAG-AGF Tg mice.

### Referential Example 17: Changes in body weight and fat weight in K14-AGF Tg mice

A transgenic mouse overexpressing AGF under the control of a K14 promoter was prepared (Example 8 of WO03/083114).
In this transgenic mouse, the AGF protein is overexpressed in epidermal cells, and angiogenesis and tissue generation are caused by an action of the expressed AGF protein. It was confirmed that the AGF overexpressed in epidermal cells exhibited an antiobesity activity and an antidiabetic activity, as shown in Referential Examples 4, 5, and 16 using mice systemically overexpressing the AGF.

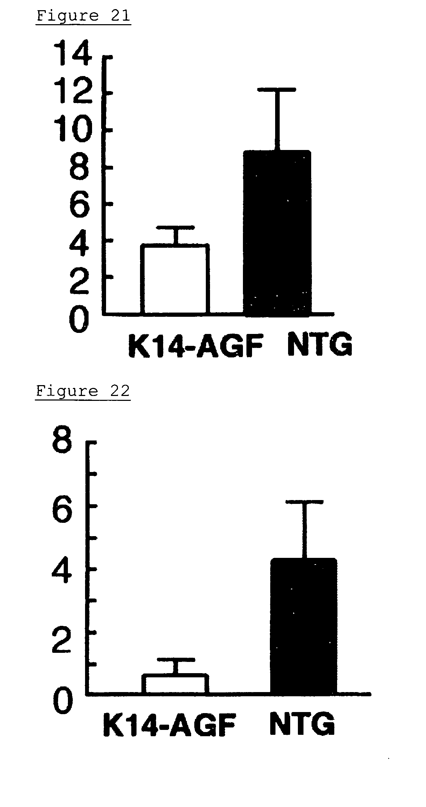

The body weights of 8-month-old female K14-AGF Tg mice and littermate WT mice (each group consisting of 5 mice) were measured. The body weight of the K14-AGF Tg mice was approximately 21 g on average, and was decreased in comparison with that (approximately 31 g on average) of the littermate WT mice. Computed tomographic images of the area from the diaphragm to the bottom of the abdominal cavity of the K14-AGF Tg mice and littermate WT mice were obtained at intervals of 2 mm using an X-ray CT for laboratory animals (Aloka), and the weights of visceral fat tissue and subcutaneous fat tissue were determined. The result is shown in Figure 21 (weight of visceral fat tissue) and Figure 22 (weight of subcutaneous fat tissue).
With respect to both visceral fat and subcutaneous fat, the weights of fat tissues of the K14-AGF Tg mice were remarkably decreased in comparison with those of the littermate WT mice. It was clarified from the result that the expressed AGF exhibited an antiobesity activity and an activity of decreasing fat tissue, as similarly shown in the above-mentioned case using the CAG-AGF Tg mice.

### Referential Example 18: Insulin sensitivity test in K14-AGF Tg mice

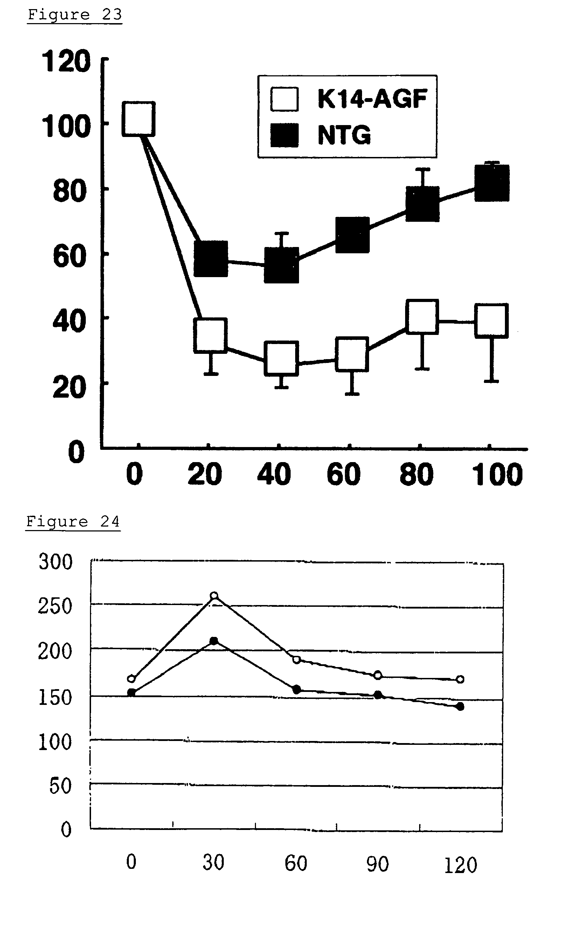

The insulin sensitivity of the K14-AGF Tg mice was examined. To 8-month-old female K14-AGF Tg mice and littermate WT mice, 0.75 units/kg of insulin was intraperitoneally administered. Blood was taken from ophthalmic veins immediately after the insulin administration, and at 20, 40, 60, 80, and 100 minutes after the insulin administration. The blood glucose level was measured using GLUTEST ACE (SANWA KAGAKU KENKYUSHO). The ratio of the blood glucose level after the insulin administration to that before the insulin administration is shown in Figure 23.
In the K14-AGF Tg mice, the blood glucose level was remarkably decreased in comparison with that in the littermate WT mice, with respect to all measurement points after the insulin administration, and it was found that insulin sensitivity was increased. It was clarified from the result that insulin sensitivity was increased by an action of the AGF protein highly expressed in the CAG-AGF Tg mice.

### Referential Example 19: Administration of adenovirus expressing human AGF to mice bred with high fat diet

It was shown in Referential Example 14 that the mouse AGF exhibited an activity of suppressing a decrease in body weight and an activity of improving glucose tolerance. In this example, it was confirmed that the human AGF exhibited the same activities by carrying out the following tests.
The adenovirus expressing the human AGF (hAGF-Adeno) prepared in Referential Example 12 and the control adenovirus (Cont-Adeno) were administered to mice (each group consisting of three mice) in accordance with a method similar to that described in Referential Example 14. After 13 days from the first administration, the body weight in the hAGF-Adeno administered mice was decreased by 4.6 g on average, and that in the Cont-Adeno administered mice was increased by 0.1 g on average.
Next, the glucose metabolism test described in Referential Example 14 was repeated, except that 0.5 g/kg of D-glucose was intraperitoneally administered and that blood was taken at 30, 60, 90, and 120 minutes after the administration. The result is shown in Figure 24. In the hAGF-Adeno administered mice, the blood glucose level was lower than that in the Cont-Adeno administered mice, with respect to all measurement points after the glucose administration, and it was found that glucose tolerance was improved.
It was clarified from these results that an action of the hAGF protein highly expressed in the hAGF-Adeno administered mice caused a decrease in body weight, and improved glucose tolerance.

### Example 1: Preparation of anti-human AGF antibodies

A rabbit was immunized with a purified full-length of human AGF protein prepared in Referential Example 9 to obtain an antibody. Immunizations were carried out every two weeks using 500 µg for the first immunization and 250 µg for the second to fourth immunizations. After 2 weeks from the last immunization, antiserum was obtained by exsanguination. The antiserum was subjected to a protein A column (Amersham Bioscience) to purify an IgG antibody (hereinafter referred to as anti-SF-YP-030 IgG).

A peptide (SEQ ID NO: 4; hereinafter referred to as peptide A) in which a Cys residue was added to the N-terminus of a peptide having a partial sequence (SEQ ID NO: 3) of the human AGF was synthesized and purified. An antigen was prepared by coupling peptide A with a carrier protein, bovine thyroglobulin (SIGMA), and a rabbit was immunized with 100 µg of this peptide eight times at intervals of 2 weeks. After 1 week from the last immunization, antiserum was obtained by exsanguination. Peptide A was used to prepare an affinity column. The serum was subjected to the affinity column to purify an IgG antibody (hereinafter referred to as anti-YP-030-A IgG) which binds to peptide A. The concentration of the antibody was determined by measuring an absorbance at 280 nm (molar absorption coefficient = 1.38).

### Example 2: HRP labeling of anti-SF-YP-030 IgG

To the anti-SF-YP-030 IgG dialyzed in a 0.1 mol/L citrate buffer, pepsin (SIGMA) was added while stirring, and the antibody was partially digested at 37°C for 1 hour. The reaction solution was subjected to a gel filtration using a Superdex 200 16/60HR column (Amersham Bioscience) equilibrated with a 0.1 mol/L phosphate buffer (pH 6.0) to collect F(ab')₂. The obtained F(ab')₂ was reduced with 2-mercapto-ethylamine hydrochloride (Nacalai Tesque) at 37°C for 90 minutes, and subjected to a gel filtration using an ULTROGEL ACA54 column (BIOSEPRA) to collect Fab'. A crosslinking agent EMCS [N-(6-Maleimidocaproyloxy) succinimide; DOJINDO] was reacted with horseradish peroxidase (HRP; TOYOBO) at 37°C for 60 minutes. The reaction solution was subjected to a gel filtration using a P6-DG column (BIORAD) equilibrated with a 0.1 mol/L phosphate buffer (pH 6.0) to collect a fraction containing a conjugate of EMCS and HRP (hereinafter referred to as EMCS-HRP). The Fab' was reacted with EMCS-HRP at 4°C for 16 hours. The reaction solution was subjected to a gel filtration using an Superdex 200 16/60HR column to collect a fraction containing a conjugate of Fab' and HRP and obtain an HRP-labeled anti-human AGF antibody Fab' fragment (HRP-anti-SF-YP-030 Fab'). The concentration of the antibody was determined by measuring an absorbance at 280 nm (molar absorption coefficient = 1.38).

### Example 3: Construction of ELISA system for detecting human AGF

To each well of a microplate for ELISA (NUNC), 100 µL/well of 20 µg/mL anti-YP-030-A IgG prepared in Example 1 was added, and allowed to stand at 4°C overnight. The solution in each well was removed by suction. After each well was washed twice with PBS (phosphate-buffered saline), 300 µL/well of a blocking solution (1% BSA, 0.05% NaN₃, and PBS) was added to each well, and allowed to stand at 4°C overnight to carry out a blocking treatment. After each well was washed twice with a washing solution (10 mmol/L phosphate buffer supplemented with 0.05% Tween20), 100 µL/well of a double-diluted series of a standard human AGF purified protein was added to each well to carry out a reaction at 37°C for 1 hour. In this connection, the double-diluted series was prepared by sequentially double-diluting the standard human AGF purified protein with a diluent (PBS supplemented with 1% BSA and 0.05% Tween20) from 100 ng/mL to 0.39 ng/mL. After each well was washed seven times with the washing solution, 100 µL/well of an HRP-labeled antibody solution was added to each well to carry out a reaction at 37°C for 0.5 hour. In this connection, the HRP-labeled antibody solution was prepared by diluting the HRP-labeled antibody solution (HRP-anti-SF-YP-030 Fab') prepared in Example 2 with the diluent to a concentration of 1 µg/mL. After each well was washed nine times with the washing solution, 100 µL/well of a TMBZ substrate solution (IBL) was added to each well to carry out a reaction at room temperature for 30 minutes. The developing reaction was stopped by adding 100 µL/well of a stopping solution (1N H₂SO₄) to each well. An absorbance at A450 nm was measured using an absorptiometer (Spectramax). The measurement values were analyzed using a program attached thereto, and a calibration curve could be prepared on the basis of a concentration range having linearity. As above, an ELISA system for detecting the human AGF could be constructed.

### Example 4: Measurement of concentration of AGF in cell culture supernatant and human serum

Concentrations of the AGF protein in culture supernatants of 293 cells, infected with Adeno-AGF or Adeno-Cont in Reference Example 13, were measured. Each culture supernatant was diluted to 1/2-1/32 with a diluent, and concentrations of the human AGF in the culture supernatants were measured by using the above ELISA system, except that the culture supernatants were used as a sample, instead of the standard human AGF purified protein. As a result, the AGF protein was not detected in the culture supernatant of cells infected with Adeno-Cont, but approximately 640 µg/mL of the AGF protein was detected in that of cells infected with Adeno-AGF.

Further, a human serum (Chemicon) was diluted to 1/20 with a diluent, and the concentration of the human AGF in the serum was measured by using the above ELISA system, except that the human serum was used as a sample, instead of the standard human AGF purified protein. As a result, the AGF protein was detected by the ELISA, and the concentration of the AGF protein in the serum was approximately 100 ng/mL.

As described above, it was found that the ELISA system prepared in Example 3 can be used in measuring a concentration of a human AGF contained in a sample. Therefore, culture supernatants or cell lysates of cells treated with each test substance were used as samples, and concentrations of AGF contained in the samples were measured using the ELISA system, to select an agent for promoting an expression of AGF (i.e., an antiobesity agent, an antidiabetic agent, and/or a hypolipidemic agent).

### Example 5: Expression and purification of human AGF fibrinogen domain protein

A vector for expressing a human AGF fibrinogen domain (hAGF-Fg) protein was constructed to use the hAGF-Fg protein as an immunogen for preparing an antibody. First, a vector pcDNA3.1-SF-Bam in which the BamHI site as a cloning site of pcDNA3.1-signal-FLAG (Example 7-1 of WO01/34785) was changed immediately upstream of the cDNA-insertion site was prepared, by using a primer set of SEQ ID NOS: 53 and 54, pcDNA3.1-signal-FLAG as a template, and a commercially available kit (Quickchange Site-Directed Mutagenesis Kit; Stratagene), in accordance with a protocol attached thereto.

Next, a PCR was carried out, using plasmid pcDNA-SF-hAGF (see Referential Example 12) containing the full-length of a human AGF cDNA as a template, a primer set of SEQ ID NOS: 55 and 56, and a pfu DNA polymerase (Stratagene), by performing a reaction at 95°C for 10 minutes and repeating a cycle consisting of reactions at 94°C for 15 seconds, at 55°C for 1 minute, and 72°C for 90 seconds 40 times. The amplified fragment was subcloned into the EcoRV site of pZEr02.1 (Invitrogen). A DNA fragment of approximately 0.7 kbp was excised with restriction enzymes BamHI and XhoI, and inserted into the BamHI-XhoI site of pcDNA3.1-SF-Bam to obtain a plasmid for expressing hAGF-Fg.

The obtained expression plasmid was introduced into a human fetal kidney cell line HEK293 (Invitrogen) using a transfection reagent (FuGENETM6 Transfection Reagent; Roche) in accordance with a protocol attached thereto. On the day following the introduction of the plasmid, the medium was changed to a medium containing 1 mg/mL of Geneticin. The transfected cells were cultured to obtain a stable cell line (hereinafter referred to as 293/hAGFFg) having a drug resistance.

The 293/hAGFFg was cultured with a culture medium (10% FBS, DMEM, and antibiotics) in a roller bottle (Corning) coated with type I collagen to become confluent. After the medium was replaced with a serum-free medium, the cells were cultured for 4 days to collect the culture supernatant. The culture supernatant was centrifuged, filtrated, and purified by an affinity chromatography using an M2 affinity gel (Sigma). The purified proteins were dialyzed in 0.1% CHAPS-containing 50 mmol/L Tris-HCl (pH8.0), adsorbed to an anion exchange column (HiTrap Q HP column), and eluted by an NaCl concentration gradient (0 to 250 mmol/L). The separated and purified hAGF-Fg protein fraction was subjected to SDS-PAGE and stained with CBB (Coomassie Brilliant Blue), to confirm that the hAGF-Fg protein was obtained with high purity. A concentration of the protein was determined using a commercially available kit (Micro BCA Protein Assay Kit; Pierce).

### Example 6: Preparation of anti-hAGF-Fg antibody

The purified hAGF-Fg protein was used as an immunogen in accordance with a conventional method to obtain clones producing a monoclonal antibody (anti-hAGFFgMoAb) as follows. An emulsion, prepared by mixing the purified hAGF-Fg protein with an equal amount of Freund's Complete Adjuvant (DIFCO), was subcutaneously and intracutaneously injected to 6-week-old BALB/c mice at a dose of 50 µg of protein per mouse, 4 times at intervals of 7 days. After 4 days from the fourth immunization, a little blood was taken from tail veins to prepare a serum. A binding activity of the serum against the hAGF-Fg protein was examined to confirm an increase in a titer. After 8 days from the collection of blood, the final immunization was carried out in a similar fashion. After 4 days from the final immunization, the spleen was taken to use in the following cell fusion. The cell fusion of the spleen cells and P3X63Ag8.653 cells (ATCC) were carried out using polyethylene glycol, and cultured with an HAT selection medium in a 96-well plate. A binding activity of each culture supernatant against the hAGF-Fg protein was examined by the method described below to select positive wells. To each well of a microplate for ELISA (NUNC), 50 µL/well of 50 ng/mL hAGF-Fg protein was added, and allowed to stand at 4°C overnight. The solution in each well was removed by suction. After each well was washed twice with PBS, 200 µL/well of a blocking solution (0.1% BSA, 0.05% NaN₃, and PBS) was added to each well, and allowed to stand at 4°C overnight to carry out a blocking treatment. After each well was washed twice with a washing solution (10 mmol/L phosphate buffer supplemented with 0.05% Tween20), 50 µL/well of each culture supernatant of hybridomas, which had been previously diluted to 1/2 with a diluent (PBS containing 0.1% BSA and 0.05% Tween20), was added to each well to carry out a reaction at 37°C for 1 hour. After each well was washed four times with the washing solution, 50 µL/well of an HRP-labeled anti-mouse-IgG antibody solution was added to each well to carry out a reaction at 37°C for 0.5 hour. In this connection, the HRP-labeled antibody solution was prepared by diluting an HRP-labeled anti-mouse-IgG antibody (Biosource) with the diluent to 1/5000. After each well was washed six times with the washing solution, 100 µL/well of o-phenylenediamine (OPD Tablet; Sigma) dissolved in a buffer for substrate [K₂HPO₄-citrate buffer (0.03% H₂O₂) pH5.1] was added to each well to carry out a reaction at room temperature for 15 minutes. The developing reaction was stopped by adding 100 µL/well of a stopping solution (1N H₂SO₄) to each well. An absorbance at A490 nm was measured using an absorptiometer.

Cells in selected wells were used to carry out the first cloning by a limiting dilution method. A binding activity against the hAGF-Fg protein was examined in a similar fashion described above to select positive wells.
Selected clones were cultured in a large-scale, and IgG antibodies were purified from each culture supernatant by using anti-mouse-IgG agarose beads (American Qualex). The concentration of each antibody was determined by measuring an absorbance at 260 nm (molar absorption coefficient = 1.3).

### Example 7: Construction of ELISA system for detecting human AGF

To each well of a microplate for ELISA (NUNC), 100 µL/well of 2.5 µg/mL IgG antibody (anti-hAGFFgMoAb #7A) prepared in Example 6 diluted with a 50 mmol/L carbonate buffer (pH9.6) was added, and allowed to stand at 4°C overnight. The solution in each well was removed by suction. After each well was washed twice with PBS, 300 µL/well of a blocking solution (1% BSA and PBS) was added to each well, and allowed to stand at 4°C overnight to carry out a blocking treatment. After each well was washed twice with a washing solution (PBS supplemented with 0.02% CHAPS), 100 µL/well of a diluted series of a standard human AGF purified protein was added to each well to carry out a reaction at room temperature for 1.5 hours. In this connection, the diluted series was prepared by sequentially diluting the standard human AGF purified protein with a diluent (PBS supplemented with 1% BSA and 0.02% CHAPS) from 30 ng/mL to 0.3 ng/mL. After each well was washed five times with the washing solution, 100 µL/well of a first antibody solution was added to each well to carry out a reaction at room temperature for 1 hour. In this connection, the first antibody solution was prepared by diluting the antibody (anti-YP-030-A IgG) prepared in Example 1 with the diluent to a concentration of 1.5 µg/mL. After each well was washed five times with the washing solution, 100 µL/well of an HRP-labeled second antibody solution was added to each well to carry out a reaction at room temperature for 1 hour. In this connection, the HRP-labeled second antibody solution was prepared by diluting an HRP-labeled second antibody (HRP-labeled Anti-rabbit IgG; MBL) with the diluent to 1/2000. After each well was washed five times with the washing solution, 100 µL/well of a substrate solution (R&D) was added to each well to carry out a reaction at room temperature for 20 minutes. The developing reaction was stopped by adding 100 µL/well of a stopping solution (1N H₂SO₄) to each well. An absorbance at A450 nm was measured using an absorptiometer (Safire; TACAN). As a result, a calibration curve having linearity could be prepared. As above, an ELISA system for detecting the human AGF could be constructed using a monoclonal antibody against the human AGF fibrinogen domain.

### Example 8: Preparation of anti-mouse AGF antibody

Peptide YP-126 (SEQ ID NO: 27) in which a Cys residue was added to the C-terminus of a partial sequence (SEQ ID NO: 5) of the mouse AGF was synthesized and purified. Peptide YP-127-B (SEQ ID NO: 28) in which a Cys residue was added to the N-terminus of another partial sequence (SEQ ID NO: 26) of the mouse AGF was synthesized and purified. Antigens were prepared by coupling each peptide with a carrier protein, bovine thyroglobulin (SIGMA), and rabbits were immunized with 100 µg of each peptide eight times at intervals of 2 weeks. After 1 week from the last immunization, antisera were obtained by exsanguination. Each peptide was used to prepare an affinity column. Each serum was subjected to the affinity column to purify an IgG antibody (hereinafter referred to as anti-YP-126 IgG) specific to peptide YP-126 and an IgG antibody specific to peptide YP-127-B (hereinafter referred to as anti-YP-127-B IgG).

### Example 9: HRP labeling of anti-YP-126 IgG

The anti-YP-126 IgG prepared in Example 8 was reacted with EMCS-HRP prepared in Example 2. The reaction mixture was subjected to a gel filtration to collect a fraction containing an antibody labeled with HRP, designated HRP-anti-YP-126 IgG.

### Example 10: Construction of ELISA system for detecting mouse AGF

To each well of a microplate for ELISA (NUNC), 100 µL/well of 20 µg/mL anti-YP-127-B IgG prepared in Example 8 was added, and allowed to stand at 4°C overnight. The solution in each well was removed by suction. After each well was washed twice with PBS, 300 µL/well of a blocking solution was added to each well, and allowed to stand at 4°C overnight to carry out a blocking treatment. After each well was washed twice with a washing solution, 100 µL/well of double-diluted series of a standard mouse AGF purified protein was added to each well to carry out a reaction at 37°C for 1 hour. In this connection, the double-diluted series was prepared by sequentially double-diluting the standard mouse AGF purified protein with a diluent from 100 ng/mL to 0.39 ng/mL. After each well was washed seven times with the washing solution, 100 µL/well of an HRP-labeled antibody solution was added to each well to carry out a reaction at 37°C for 0.5 hour. In this connection, the HRP-labeled antibody solution was prepared by diluting the HRP-labeled antibody solution (HRP-anti-YP-126 IgG) prepared in Example 9 with the diluent to a concentration of 1 µg/mL. After each well was washed nine times with the washing solution, 100 µL/well of a TMBZ substrate solution (IBL) was added to each well to carry out a reaction at room temperature for 30 minutes. The developing reaction was stopped by adding 100 µL/well of a stopping solution (1N H₂SO₄) to each well. An absorbance at A450 nm was measured using an absorptiometer (Spectramax). In the above procedures, the blocking agent, the washing solution, and the diluent same as those described in Example 3 were used. The measurement values were analyzed using a program attached to the absorptiometer, and a calibration curve could be prepared on the basis of a concentration range having linearity. As above, an ELISA system for detecting the mouse AGF could be constructed.

### Example 11: Differentiation of 3T3-L1 to adipocyte

A method for differentiation of a mouse 3T3-L1 cell (ATCC No.: CL-173), cultured in a Dulbecco's modified Eagle's medium (DMEM) supplemented with 10% bovine serum, to an adipocyte was basically carried out in accordance with a known method (Ezaki et al., "IGAKU NO AYUMI (Journal of Clinical and Experimental Medicine)", 503-506, 1998). The 3T3-L1 cell is a mouse-derived cultured cell strain which is widely used as a model cell of a white adipocyte.
The 3T3-L1 cells were plated on a 24-well collagen-coated plate (ASAHI TECHNO GLASS) to become 50000 cells per well. When the cells were confluent, the medium was changed to a Dulbecco's modified Eagle's medium (DMEM) supplemented with 0.5 mmol/L 3-isobutyl-1-methylxanthine, 0.25 µmol/L dexamethasone, 10 µg/mL insulin, and 10% fetal bovine serum to start the differentiation to adipocytes. After 2 days, the medium was changed to DMEM containing 10% fetal bovine serum, and then the medium change was repeated every three days to start an induction of differentiation. A culture supernatant after 6 to 8 days from the induction of differentiation was collected as a culture supernatant of the adipocyte 3T3-L1.

### Example 12: Preparation of template cDNA

C2C12 cells (ATCC No.: CRL-1772) were added to a 24-well collagen-coated plate (ASAHI TECHNO GLASS) to become 20000 cells per well, and cultured with a Dulbecco's modified Eagle's medium (DMEM) supplemented with 15% fetal bovine serum, for one day. After insulin (Biomedical Technologies Inc) was added to the medium to a final concentration of 100 nmol/L, or after the medium was changed to the culture supernatant of 3T3-L1 prepared in Example 11, the C2C12 cells were cultured for 48 hours. As a control, the C2C12 cells were cultured with DMEM supplemented with 15% fetal bovine serum for 48 hours, without such treatments. After the cultivation, total RNAs were prepared from treated cells using an RNeasy Mini Kit (Qiagen) in accordance with a protocol attached thereto, and 500 ng thereof was converted to cDNAs using a Thermoscript RT-PCR system kit (Invitrogen).

### Example 13: Measurement of amount of AGF gene expressed in C2C12 cell by quantitative PCR

Amounts of AGF gene expressed in C2C12 cells were measured by a quantitative PCR, using DNAs of SEQ ID NOS: 29 and 30 as primers for amplifying the mouse AGF, cDNAs (derived from culture supernatants of C2C12 cells) prepared in Example 12 as a template, and a sequence detector (ABI PRISM 7900; Applied Biosystems). In the PCR, an SYBR GREEN PCR Master Mix (Applied Biosystems) was used, and a reaction at 95°C for 10 minutes was carried out, and a cycle composed of reactions at 95°C for 15 seconds and at 60°C for 1 minute was repeated 50 times. As an internal standard, a PCR was carried out under the same conditions except that oligonucleotides of SEQ ID NOS: 31 and 32 were used as a primer set, to calculate an amount of mouse cyclophilin expressed. Further, a PCR was carried out under the same conditions except that mouse genome (Clontech) was used as a template and that the above primer set for the mouse AGF or cyclophilin was used as a primer set, to obtain a standard curve for calculating an amount of mRNA expressed. An amount of AGF expressed in each sample was compensated on the basis of an amount of cyclophilin expressed. As a result, in C2C12 cells, the expression of AGF was induced approximately 6 times and approximately 8 times by insulin and the culture supernatant of adipocyte 3T3-L1, respectively, in comparison with the untreated control case.

As above, it was found that an agent for promoting an expression of AGF (i.e., an antiobesity agent, an antidiabetic agent, and/or a hypolipidemic agent) can be obtained by carrying out a quantitative PCR using, as a template, cDNAs prepared from a culture supernatant of C2C12 cells treated with a test substance.

### Example 14: Cloning of mouse AGF promoter region and construction of reporter plasmid

To clone a mouse AGF promoter region having a length of approximately 5.6 kb upstream of exon 1, a PCR was carried out using a forward primer (SEQ ID NO: 33) and a reverse primer (SEQ ID NO: 34), mouse BAC clone (RP23-369H22; Children's Hospital Oakland), and a DNA polymerase (TaKaRa Ex taq^{™}; Takara). In the PCR, a reaction at 94°C for 5 minutes was carried out, a cycle composed of reactions at 94°C for 1 minute, at 58°C for 90 seconds, and at 72°C for 3 minutes was repeated 32 times, and a reaction at 72°C for 10 minutes was carried out. The obtained PCR product was subcloned into a cloning vector (pGEM^{R}-T Easy Vector Systems; Promega) to obtain pGEM-5.6kb. The DNA sequence thereof was analyzed using an agent for DNA sequence (BigDye3.1; Applied Biosystems) and a DNA sequencer (Model PRISM3700; Applied Biosystems) in accordance with protocols attached thereto to determine the nucleotide sequence of nucleotides 1-5572 of SEQ ID NO: 1.

Fragments having a length of approximately 0.5 kb, 0.9 kb, 1.4 kb, 2 kb, 2.15 kb, or 2.5 kb, upstream from the XhoI site of approximately 2.8 kb upstream of exon 1, were amplified by carrying out a PCR using primers listed in Table 1, pGEM-5.6kb as a template, and a DNA polymerase (TaKaRa Ex taq). In the PCR, a reaction at 94°C for 5 minutes was carried out, a cycle composed of reactions at 94°C for 1 minute, at 60°C for 2 minutes, and at 72°C for 2 minutes was repeated 25 times, and a reaction at 72°C for 10 minutes was carried out. As described below, each fragment was ligated to the end of the fragment having a length of approximately 2.8 kb upstream of exon 1, to prepare fragments having a length of approximately 3.3 kb, 3.7 kb, 4.2 kb, 4.8 kb, 5 kb, or 5.3 kb.

**Table 1**

| Name | Forward primer | Reverse primer | Length of PCR product |
|---|---|---|---|
| 3.3 kb | SEQ ID NO: 35 | SEQ ID NO: 41 | 0.5 kb |
| 3.7 kb | SEQ ID NO: 36 | SEQ ID NO: 41 | 0.9 kb |
| 4.2 kb | SEQ ID NO 37 | SEQ ID NO 41 | 1.4 kb |
| 4.8 kb | SEQ ID NO: 38 | SEQ ID NO 41 | 2 kb |
| 5 kb | SEQ ID NO: 39 | SEQ ID NO 41 | 2.15 kb |
| 5.3 kb | SEQ ID NO 40 | SEQ ID NO: 41 | 2.5 kb |

Each fragment amplified in the PCR was subcloned into a cloning vector (pGEM^{R}-T Easy Vector Systems; Promega) to obtain plasmid 3.3kb, plasmid 3.7kb, plasmid 4.2kb, plasmid 4.8kb, plasmid 5kb, and plasmid 5.3kb.
A DNA fragment containing nucleotides 16-5572 of SEQ ID NO: 1, obtained by digesting pGEM5.6kb with restriction enzyme BamHI, was inserted into a BglII-treated vector pGL3-basic (Promega) for a luciferase assay system, to obtain pGL-5.6kb and pGL-5.6kR. In the pGL5.6kb, the fragment was inserted in the forward direction, and the luciferase gene was located downstream (i.e., at the 3' side) of the inserted promoter sequence. In the pGL5.6kR, the fragment was inserted in the reverse direction, and the luciferase gene was located upstream (i.e., at the 5' side) of the inserted promoter sequence. A DNA fragment containing nucleotides 2741-5572 of SEQ ID NO: 1, obtained by digesting pGEM5.6kb with restriction enzymes XhoI and BamHI, was inserted into pGL3-basic treated with restriction enzymes XhoI and BglII, to obtain pGL-2.8kb. A DNA fragment containing nucleotides 5121-5572 of SEQ ID NO: 1, obtained by digesting pGEM5.6kb with restriction enzymes KpnI and BamHI, was inserted into pGL3-basic treated with restriction enzymes KpnI and BglII, to obtain pGL-0.5kb. The above plasmid 3.3kb, plasmid 3.7kb, plasmid 4.2kb, plasmid 4.8kb, plasmid 5kb, and plasmid 5.3kb were digested with a restriction enzyme XhoI, and each obtained fragment was inserted into a XhoI-treated pGL-2.8kb, to obtain pGL-3.3kb containing nucleotides 2174-5572 of SEQ ID NO: 1, pGL-3.7kb containing nucleotides 1760-5572 of SEQ ID NO: 1, pGL-4.2kb containing nucleotides 1267-5572 of SEQ ID NO: 1, pGL-4.8kb containing nucleotides 672-5572 of SEQ ID NO: 1, pGL-5kb containing nucleotides 548-5572 of SEQ ID NO: 1, pGL-5.3kb containing nucleotides 239-5572 of SEQ ID NO: 1, respectively.

To clone a region having a length of approximately 6.7 kb upstream of exon 2, the above PCR was carried out, except that a forward primer (SEQ ID NO: 33) and a reverse primer (SEQ ID NO: 42) were used. The region was composed of a region of having a length of approximately 5.6 kb upstream of exon 1, that of approximately 100 bp of exon 1, and that of approximately 1 kb of intron 1. The amplified fragment of 6.7 kb was subcloned and sequenced to determine the nucleotide sequence of nucleotides 1-6684 of SEQ ID NO: 2. The obtained clone was digested with a restriction enzyme BamHI. The fragment was inserted into BglII-treated pGL3-basic, to obtain an expression vector pGL-6.7kb containing nucleotides 16-6684 of SEQ ID NO: 2, which was a region having a length of approximately 6.7 kb upstream of exon 2, containing a region of approximately 5.6 kb upstream of exon 1.

### Example 15: Analysis of DNA sequence of mouse AGF promoter region

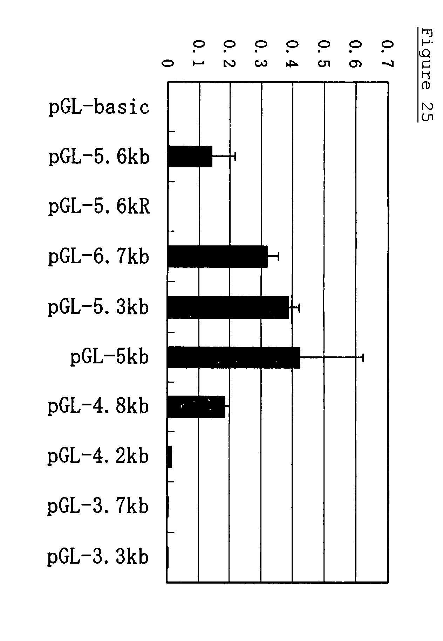

To measure a reporter activity of pGL-basic (empty vector) or the reporter plasmids prepared in Example 14, FaO cells (J.Cell Science, 2000, 113, 1069-1074) were cotransfected with each reporter plasmid to be assayed and phRL-TK (Promega) for expressing luciferase derived from renilla, as described below. The FaO cells were cultured with a growth medium [low glucose DMEM with 10% fetal calf serum (FCS)] in a type I collagen coated 12-well plate to become subconfluent. The medium was changed to a serum-free Opti-MEM^{R}I (Invitrogen), the FaO cells were transfected with the above plasmids using a transfection reagent (Lipofectamine^{™} Reagent; Invitrogen). After 3 hours from the transfection, an equal amount of low glucose DMEM supplemented with 20% FCS was added. After 12 hours, the medium was changed to low glucose DMEM with 10% FCS. After 12 hours, a luciferase activity was measured using a Dual-Glo^{™} Luciferase Assay System (Promega) in accordance with a protocol attached thereto. Each reporter activity calculated by dividing a firefly luciferase activity expressed from the plasmid containing each promoter by a renilla luciferase activity expressed from plasmid phRL-TK for internal compensation is shown in Figure 25. As a result, no reporter activity was observed when pGL-3.3kb, pGL-3.7kb, or pGL-4.2kb was introduced, and the reporter activity was observed when pGL-4.8kb, pGL-5kb, pGL-5.3kb, pGL-5.6kb, or pGL-6.7kb was introduced. Further, when the fragment of 5.6kb upstream of exon 1 was inserted in the reverse direction, the promoter activity was lost. Furthermore, no promoter activity was observed when pGL-0.5kb or pGL-2.8kb was used. From these results, it was found that the sequences composed of nucleotides 672-5572, nucleotides 548-5572, nucleotides 239-5572, or nucleotides 16-5572 of SEQ ID NO: 1, or nucleotides 16-6684 of SEQ ID NO: 2 had an AGF promoter activity.
As above, a reporter assay system could be constructed by using the above DNA sequences, and it was found that the AGF promoter activity was contained in the above regions. According to this assay system, a substance which promotes a promoter activity of an AGF gene and an expression of the AGF, i.e., an antiobesity agent, an antidiabetic agent, and/or a hypolipidemic agent, may be obtained by adding a test substance to cells transfected with a plasmid containing a DNA sequence having an AGF promoter activity and analyzing a change in a luciferase activity.

### Example 16: Promotion of MAPK phosphorylation by AGF stimulation

C2C12 cells were cultured with a growth medium (DMEM supplemented with 10% FCS) in a type I collagen coated 12-well plate to become confluent. After the medium was changed to a differentiation medium (DMEM supplemented with 2% horse serum), the C2C12 cells were cultured for 7 days to differentiate them into muscle. The cells were cultured in a serum-free medium containing 1% BSA, and the medium was changed to the same medium. The AGF protein (final concentration = 1 µg/mL) or an equal volume of PBS was added. After 10 minutes, the medium was remove by suction, and the reaction was stopped by adding 50 µL of a solution for cell lysis [50 mmol/L HEPES (pH7.2), 1% Triton X-100, 10% glycerol, 10 mmol/L Na₄P₂O₇, 100 mmol/L NaF, 100 µmol/L Na₃VO₄, 40 mmol/L EDTA, 50 µg/mL aprotinin, 1 mmol/L phenylmethyl sulfonyl fluoride (PMSF), 0.1 mmol/L leupeptin, and 25 µmol/L pepstatin A] to the cells. To each cell lysate sample obtained, an SDS-sample buffer [20% glycerol, 130 mmol/L Tris-HCl (pH6.8), 10% 2-mercaptoethanol, 6% SDS, and 0.2 mg/mL bromphenol blue] was added, and each sample was subjected to SDS-PAGE and blotted to a polyvinylidene fluoride (PVDF) membrane. Western blotting was carried out, using an anti-phosphorylated MAPK p38 antibody (Cell signaling #9211), an anti-phosphorylated MAPK p42/p44 antibody (Cell signaling #9101), an anti-MAPK p38 antibody (Cell signaling #9212), or an anti-MAPK p42/44 antibody (Cell signaling #9102) as the first antibody and an HRP-labeled anti-mouse IgG antibody (Example 9) as the second antibody, to detect a phosphorylation of MAPK p38, MAPK p42, and MAPK p44. The result of Western blotting was scanned using a scanner, and a concentration of each phosphorylated band was quantitated by an image analyzing software. As a result, it was found that an increase of phosphorylation in phosphorylated MAPK p38, phosphorylated MAPK p42, and phosphorylated MAPK p44 was made 1.6 times, 3.4 times, and 4.3 times, respectively, by adding the AGF protein.

As above, an assay system for detecting a reaction in a cell stimulated with an AGF agonist could be constructed. According to this assay system, a substance having an agonist activity of AGF, i.e., an antiobesity agent, an antidiabetic agent, and/or a hypolipidemic agent, may be obtained by adding a test substance to cells and analyzing a change in a phosphorylation of MAPK.
Further, the above procedure was repeated, except that a human umbilical vein endothelial cell (HUVEC) or a chondrogenic cell line ATDC5 was used instead of the C2C12 cell, and it was confirmed that the phosphorylation of MAPK was promoted by AGF stimulation. In this connection, the HUVEC cells and the ATDC5 cells were cultured with a growth medium (ASAHI TECHNO GLASS) and a growth medium (DMEM/F12 with 5% FCS), respectively, to become subconfluent, and used for the assay.

### Example 17: Binding of AGF protein to cell line

ATDC5 cells were cultured in a growth medium (DMEM/F12 with 5% FCS) to become subconfluent, and removed from a plate by a cell scraper. The cells were washed twice with an FCM buffer (0.1% BSA/1 mmol/L CaCl₂/1 mmol/L MgCl₂/PBS), and reacted with or without the mouse AGF protein or the human AGF protein at 4°C for 30 minutes, at a final concentration of 10 µg/mL of the protein. The cells were washed with the FCM buffer, and reacted with fluorescein isothiocyanate (FITC)-labeled M2 antibody (Sigma) at 4°C for 30 minutes, at a final concentration of 10 µg/mL. The cells were washed three times with the FCM buffer, and propidium iodide (PI) was added to the cells at a final concentration of 1 µg/mL, to stain death cells. The cells were analyzed using a flow cytometer (Cytomics FC 500; Beckman Coulter, Inc.), and an FITC intensity derived from PI negative cells other than death cells was measured.

Since the FLAG sequence was added to the human AGF protein and the mouse AGF protein, when the M2 antibody specific to the FLAG sequence was used, the FITC-labeled M2 antibody bound to the AGF protein which bound to cells, and thus, the fluorescence intensity of cells to which the AGF protein bound was increased. In the above measurement, an increase of the fluorescence intensity was made 1.5 times and 8.1 times by adding the mouse AGF protein and the human AGF protein, respectively, and it was found that the AGF proteins bound to cells. A substance which increases an amount of an AGF protein which binds to a cell strengthens a signal of the AGF, and thus, such a substance can be used as an agent for promoting an AGF function. According to this binding assay system, an activity of a test substance for promoting an AGF function may be measured by adding the test substance to cells, before or at the same time as adding the AGF protein, and thus, an agent for promoting an AGF function (i.e., an antiobesity agent, an antidiabetic agent, and/or a hypolipidemic agent) may be obtained.

### INDUSTRIAL APPLICABILITY

The screening method of the present invention may be used in screening for an antiobesity agent, an antidiabetic agent, and/or a hypolipidemic agent.
Although the present invention has been described with reference to specific embodiments, various changes and modifications obvious to those skilled in the art are possible without departing from the scope of the appended claims.

### FREE TEXT IN SEQUENCE LISTING

Features of "Artificial Sequence" are described in the numeric identifier <223> in the Sequence Listing. Each of the nucleotide sequences of SEQ ID NOS: 6, 7, 9, 10, 13, 14, 16, 19, 20, 34 to 40, 42, 45 to 50, and 53 to 56 is an artificially synthesized primer sequence. The amino acid sequence of SEQ ID NO: 4 is a sequence of peptide A. The nucleotide sequence of SEQ ID NO: 15 is a sequence containing loxP. The amino acid sequence of SEQ ID NO: 27 is a sequence of peptide YP-126. The amino acid sequence of SEQ ID NO: 28 is a sequence of peptide YP-127-B.

## Claims

1. A method of screening for an antiobesity agent, an antidiabetic agent, and/or a hypolipidemic agent, comprising the step of analyzing whether or not a substance to be tested promotes an expression and/or a function of an angiopoietin-related growth factor.

2. The method according to claim 1, wherein the analyzing step comprises the steps of:
i) bringing a substance to be tested into contact with a cell, and
ii) measuring an amount of an endogenous angiopoietin-related growth factor protein or gene contained in the cell, and analyzing a test substance dependent change in the amount thereof.

3. The method according to claim 2, wherein an amount of an angiopoietin-related growth factor protein is measured in the measuring step.

4. The method according to claim 2, wherein an amount of an angiopoietin-related growth factor gene is measured in the measuring step.

5. The method according to claim 1, wherein the analyzing step comprises the steps of:
i) bringing a substance to be tested into contact with a cell transformed with a vector in which a reporter gene is fused downstream of a DNA selected from
a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor, or
a DNA comprising a nucleotide sequence having a 90% or more identity with that consisting of nucleotides 672-5572 of SEQ ID NO: 1, and having a promoter activity for an angiopoietin-related growth factor, and
ii) measuring an expression of the reporter gene, and analyzing a test substance dependent change in the expression.

6. The method according to claim 1, wherein the analyzing step comprises the steps of:
i) bringing a substance to be tested into contact with a cell; and
ii) measuring a phosphorylation of a mitogen-activated protein kinase, and analyzing a test substance dependent change in the phosphorylation.

7. The method according to claim 1, wherein the analyzing step comprises the steps of:
i) bringing a substance to be tested and an angiopoietin-related growth factor into contact with a cell; and
ii) measuring an amount of the angiopoietin-related growth factor which binds to the cell, and analyzing a test substance dependent change in the amount thereof.

8. A method of screening for an agent for promoting an expression and/or a function of an angiopoietin-related growth factor, comprising the steps of:
analyzing whether or not a substance to be tested promotes the expression and/or the function of an angiopoietin-related growth factor, and
analyzing effects of a selected candidate on a change in body weight, a blood glucose level, and/or a lipid content in blood.

9. The method according to claim 8, wherein the step of analyzing whether or not the expression and/or the function of an angiopoietin-related growth factor is promoted comprises the steps of:
i) bringing a substance to be tested into contact with a cell, and
ii) measuring an amount of an endogenous angiopoietin-related growth factor protein or gene contained in the cell, and analyzing a test substance dependent change in the amount thereof.

10. The method according to claim 9, wherein an amount of an angiopoietin-related growth factor protein is measured in the measuring step.

11. The method according to claim 9, wherein an amount of an angiopoietin-related growth factor gene is measured in the measuring step.

12. The method according to claim 8, wherein the step of analyzing whether or not the expression and/or the function of an angiopoietin-related growth factor is promoted comprises the steps of:
i) bringing a substance to be tested into contact with a cell transformed with a vector in which a reporter gene is fused downstream of a DNA selected from a DNA comprising a nucleotide sequence in which 1 to 10 nucleotides are deleted, substituted, and/or inserted in the nucleotide sequence consisting of nucleotides 672-5572 of SEQ ID NO: 1, and having a promoter activity for an angiopoietin-related growth factor, or
a DNA comprising a nucleotide sequence having a 90% or more identity with that consisting of nucleotides 672-5572 of SEQ ID NO: 1, and having a promoter activity for an angiopoietin-related growth factor, and
ii) measuring an expression of the reporter gene, and analyzing a test substance dependent change in the expression.

13. The method according to claim 8, wherein the step of analyzing whether or not the expression and/or the function of an angiopoietin-related growth factor is promoted comprises the steps of:
i) bringing a substance to be tested into contact with a cell; and
ii) measuring a phosphorylation of a mitogen-activated protein kinase, and analyzing a test substance dependent change in the phosphorylation.

14. The method according to claim 8, wherein the step of analyzing whether or not the expression and/or the function of an angiopoietin-related growth factor is promoted comprises the steps of:
i) bringing a substance to be tested and an angiopoietin-related growth factor into contact with a cell; and
ii) measuring an amount of the angiopoietin-related growth factor which binds to the cell, and analyzing a test substance dependent change in the amount thereof.


SEQUENCE LISTING

<110> Astellas Pharma Inc.
<110> KEIO UNIVERSITY

<120> Method of screening antiobesity agents

<130> AP05056-751

<150> JP 2004-351814
<151> 2004-12-03

<160> 56

<170> PatentIn version 3.1

<210> 1
<211> 5572
<212> DNA
<213> Mus musculus

<220>
<223> Inventor: Yasunaga, Kunio; Yamaji, Noboru; Abe, Kunitake
      Inventor: Oike, Yuichi

<400> 1
atgtctatag agatggatcc aatatattaa cagagaggtc acccagccca gaaatcccta      60

atgtgcttta aatcaggcct gcaagctcac ttgggtgtct ctctgtcttg gtactggaga     120

gaccccaaca tgctggacct ctgctgaata aaacactctt cgcctttaca tactccttga     180

gtccagggtg tcattcttcg gcgaatcatg agcccttaca cctgagtgtt gggataaaag     240

gcatgtacca tcacacccag ggaatagttc gtgactgtgg tagtttgaag aaggctcata     300

tatttgaaca tttggtaccc acttggtgga acagtttggg aaggattagt gggtgtgacc     360

ttattggagg agattcgtca ctttgtggat tcaaaagtga tgttcttttt ctgcccgtga     420

gccaattcaa gacagattag attagattaa atgcagattt cttgggaaac tgctccccgg     480

tgggttcact agccccaagg actgaggcca cggaagtcac cacatggggg gttggaggga     540

aggataaaga gagaaagggc acagggcagg aagagagaga aagtgaaggg ggtggggaga 600

ccaaaatgtg gagagccgtg ccgtgagcaa tcgccattat aagatggcac tggcttccac 660

tgcgcctaac tagttaacaa gccttatgcg caagtgcaac agtgaactca tgcctagtca 720

ctgcccatct cgtcgcgtag taatggggtg atgggcgagc aacgaatcag gagctgtcac 780

gccacatcag gtgctgaaac gtcacactgc gggctatata agcagcgcca ttttcccggt 840

tcagagtctt ccctcctgat aagtaagcaa tacaagcttt gccgcagaag attccagttg 900

tcctgagtgt tttcttgccg gcggggacaa aagctcaggc aataagtggc gctgaggaac 960

ccaggaacta ccatcacacc ggcgcaaggg agatctctcg ttcgcggggc ggaaccagaa 1020

ctgcgggaca cattacagct ttacgaggta tgtctggcct tgaactttct ccagtgttag 1080

aagccttttg ttccttttca catgtcaaca agtggttaag gcagggcaga gaattctgga 1140

cgaaattcaa gacagtctat cagaggtaaa gccgggagag agagtaggag caaagaggaa 1200

acatggtgca ccaattaagc atacaggcct ttccacgggt cttgaacccg aggaaaagtt 1260

aatgtcaggg aagaatacct ggggagagat tggaaggaag gagaaggaaa aagaaaagaa 1320

aaaagatcaa tcagcggagg tttctaggag aaggaactta tacccgccac tagatgagtt 1380

aaagcggcta gctcttagta gctcagaatc agatgaagga cttagctcct ctgaagaaat 1440

ggacttggag gaggaagcag ttcgttataa gggagaaagg taccagcaag ataaaaagcg 1500

agctactcag tccagaaaaa ggcaaaaagc ggctgatgaa agccagcttg ctgcttggcc 1560

tccggactgt cggcttcagg gtcttagtgt acctcagccc tatgtgcaga ggtaccattc 1620

agagccttat gtgcagaggt atcattcaga ttcattcatt ccaaaagagg aacagagaaa 1680

agtgcagcaa gcattcccag tttttgaagg agctgaggga gggcgtgttc acgctccggt 1740

agagtatata cagattaagg agctcacaga agcagttcac aattatgtag tcagcgctaa 1800

tttcactata gcacaagtcg aaaggcttgc gaatcacgct atgactcctg gtgattggca 1860

gactgtagta aaagctgcag cccccagtat gggaatgtat cttgagtgga aagccttgtg 1920

gcaggactcc tgccagacac aggcaagggc caatgccacc atagaagggg accaaagaac 1980

gtggacgttt gaattattta caggtcaggg acagcatgtt gctaaccaaa cagattatca 2040

ttggggagca tacgctcaga tctcagctgc cgctgttaag gcgtgaaagg cactctctaa 2100

gaagggagag gcaagtgggc atttgaccaa gattactcaa ggtgcacagg agtccttctc 2160

agactttgtg gacagaatga cggaggcagc agggaggata tttggagatc cagaagcagc 2220

tatgccttta attgaacaat tggtttatga acaagctacg caggaatgca gatcagcaat 2280

tacacctaga aagagtaagg ggttacagga ctggttaaag gtttgccgtg agcttggagg 2340

gccgcttact aatgctggat tggcagcagc cattctgcag gggaggaggt gctcggattc 2400

aactgagctc aaactttgct ttaattgtgg caaaccaaga catttaaaaa aggactgtag 2460

agcccctata aaaaggacag caccaggatt gtgtactaaa tgtggaaaag gatatcattg 2520

ggctaaggac tgtcgttcaa ttagagatat acgaggcagg ctcttgcggc ctacaaggtc 2580

ttattgtcca tcctggagtt gtagatcagg attataaagg ggaacttcag gttctctgtt 2640

cttgtcctca aggtgtcttt tctatatcac aaggagacag aatagctcaa ctaataattt 2700

tgccaagcct acatggccgt tttccttcct ctggtattcc tcgagctgcc agagggattg 2760

gttctactgg aaatgattct gcttacttaa taatgccttt agattccagg ccatccttgg 2820

agttagctat agaaggaaaa caatttaagg gaattttaga tacaggagca gacaaaagta 2880

ttatttcttc tcactggtgg ccaaaaacct ggcccgtaac tcagtcatca cactctttac 2940

aagggctagg ctatcagtcc tgctccacta ttagctcccg ttctttgagc tggcaaatgg 3000

atgtcacgca tatttcctcc tttgggaaaa atcaatattt acatgtttgc taatctgtct 3060

aggtagattt ctttgtatct tgcaggaaat tggtctgaaa aattcgatga gacccttgag 3120

gccctgagag cggctgttct caggatcaac tcgatacgag tggacctgtc attgacagag 3180

ggtctctcct cctggatttc atctgctttt tcttatttta aggaatgggt ggggtggttt 3240

tgtttgctgc tgccatctgc tgtggacttg tgtttatgct ctggttagtt tgcaagctca 3300

gaacccaaca aaaccgtgac aagatcgtta tcgctcaagc acttgcagcc attgaacaag 3360

gggcctcccc tgaaatttgg ctgtccatgc ttaagaccta aatggcatct gagttctctg 3420

ctattgcgtc ccacggattt gtggatacat tgcactggga tgtgagagac tcaacgctca 3480

ttgatcagca cttacacctc gctgaggttt actaattcgt tttttggctg gcctctttta 3540

tagagttcgc cctaggtcat ttagcagtta ctgtcacaca gtactgcggt attcagagac 3600

gggcaacttt cctcatgcac agaccaacct aagacacggg accccgtggc gatagggtta 3660

ccctatgaca ggaaaggact gacctgtcat agggtaacct gtcattcctg gaggaatgac 3720

ctaagacagg agccacaata gatggacatg actgcagagg cctagaccag cctcaaattt 3780

taataaaata aaaaggggga gatgtggaga gccgtgccgt gagcaatcgc cattataaaa 3840

tggcactggc ttccactgcg cctaactagt taacaagcct tatgcgcaag tgcaagagtg 3900

aactcacgcc tatcactgcc catctcgcag tgtagtaatg gggtgatggg cgagcaacga 3960

atcaggagct gtcacaccac atcaggtgct gaaacgtcac gctgtgggct atacaagcag 4020

cgccatttcc ccggttcggg gtcttccctc ctgataagta agcaataaaa gctttgccgc 4080

agaagattct ggttgcagga ttgggggcag ggtgtgccag gttgggactg agggatgctg 4140

ggagaactta gaagccaggt ctgagctggg cagtggtggc gcacgcctct aataccagca 4200

ctcaggaggc agaggcaggc ggatttctga gtttgaggcc agcctggtct acaaagtgag 4260

ttccaggaca gccagggcta cacagagaaa ccctgtcttg aaaaaccaaa gaaacaaaaa 4320

agaaagaaag aaaaaaaaag aagccaggtc tgctttggta tgcaaaacat gtgcatgctc 4380

gcgtgtgtgt gtgcacacac acacactgtc ccctgtccca gggtctgaag ccaaacaaaa 4440

aggtcactcc tctgcttcct gcttatgtgt agcatgcaag ctctcagcta ctgctccagg 4500

atcatgtttg tctatctgtc tgtctgctgc catgctccct gtcataatgg ttatagactc 4560

accctctgaa actgtaacca ggcccccaat taaatgcttt cttgtataag ttggtcatgg 4620

tgtctcttca cagcagaaga accatgacta agacaaggat ctaaagcaaa gagcctcgga 4680

cagctccaaa acttgttggg tagcatatgg acagaaacat cttgtctgac aagaaagcca 4740

acatagtaat gagagcatct gcaggcagga gccaggagaa gactaacgac taataagtca 4800

acataaaaca accctcagat aaaatcttcc tccgcaatgt tcccttttgt gtcctctaac 4860

attccaagcc ccctgcccct cgtacaatct ccatttctct agtaaaggac tgcctgctct 4920

gttgagctga tgctggcttt tatttccatg ttgccttcat cagttccgat ctaacaccca 4980

ctcccactcc cttttaagac aggggctcac tggctggctt ggaactcaca gagctccaac 5040

tgctgctgcc tcccaggtac tgtgattaag tctgtgctac cacatcctaa ggttttgttt 5100

cttctggcaa tattgggtac caaggacttt gtaagtgtcc tactactgag ctacaccccc 5160

agcccctcac tgggggattc taggcagggg ctcacacctc tactggctgc tggggttaaa 5220

tgagaaagtt ttaccccttt aagggtgtcc ccagcgccag gcatcagggt actcactgat 5280

acatcatcat tgtcatggtt gtctgagtct ggagcccaca agtctaccag ggcctagggc 5340

ctcccatgct ctgtgagcct gagagaaagg aagagactcg tagggcagtg gcctctgtta 5400

tcatgctccg gaggcctggg caaggggatc tggctattct gcccagggtt ctcacactgg 5460

aaatactaaa tatttacact tgctgtgctc ccaggctcca tccatgctct gggggatggg 5520

tggtttccaa gtcttctctc ggcttctgtc ttttcagtct cctgcaccca ga 5572

<210> 2
<211> 6684
<212> DNA
<213> Mus musculus

<400> 2
atgtctatag agatggatcc aatatattaa cagagaggtc acccagccca gaaatcccta 60

atgtgcttta aatcaggcct gcaagctcac ttgggtgtct ctctgtcttg gtactggaga 120

gaccccaaca tgctggacct ctgctgaata aaacactctt cgcctttaca tactccttga 180

gtccagggtg tcattcttcg gcgaatcatg agcccttaca cctgagtgtt gggataaaag 240

gcatgtacca tcacacccag ggaatagttc gtgactgtgg tagtttgaag aaggctcata 300

tatttgaaca tttggtaccc acttggtgga acagtttggg aaggattagt gggtgtgacc 360

ttattggagg agattcgtca ctttgtggat tcaaaagtga tgttcttttt ctgcccgtga 420

gccaattcaa gacagattag attagattaa atgcagattt cttgggaaac tgctccccgg 480

tgggttcact agccccaagg actgaggcca cggaagtcac cacatggggg gttggaggga 540

aggataaaga gagaaagggc acagggcagg aagagagaga aagtgaaggg ggtggggaga 600

ccaaaatgtg gagagccgtg ccgtgagcaa tcgccattat aagatggcac tggcttccac 660

tgcgcctaac tagttaacaa gccttatgcg caagtgcaac agtgaactca tgcctagtca 720

ctgcccatct cgtcgcgtag taatggggtg atgggcgagc aacgaatcag gagctgtcac 780

gccacatcag gtgctgaaac gtcacactgc gggctatata agcagcgcca ttttcccggt 840

tcagagtctt ccctcctgat aagtaagcaa tacaagcttt gccgcagaag attccagttg 900

tcctgagtgt tttcttgccg gcggggacaa aagctcaggc aataagtggc gctgaggaac 960

ccaggaacta ccatcacacc ggcgcaaggg agatctctcg ttcgcggggc ggaaccagaa 1020

ctgcgggaca cattacagct ttacgaggta tgtctggcct tgaactttct ccagtgttag 1080

aagccttttg ttccttttca catgtcaaca agtggttaag gcagggcaga gaattctgga 1140

cgaaattcaa gacagtctat cagaggtaaa gccgggagag agagtaggag caaagaggaa 1200

acatggtgca ccaattaagc atacaggcct ttccacgagt cttgaacccg aggaaaagtt 1260

aatgtcaggg aagaatacct ggggagagat tggaaggaag gagaaggaaa aagaaaagaa 1320

aaaagatcaa tcagcggagg tttctaggag aaggaactta tacccgccac tagatgagtt 1380

aaagcggcta gctcttagta gctcagaatc agatgaagga cttagctcct ctgaagaaat 1440

ggacttggag gaggaagcag ttcgttataa gggagaaagg taccagcaag ataaaaagcg 1500

agctactcag tccagaaaaa ggcaaaaagc ggctggtgaa agccagcttg ctgcttggcc 1560

tccggactgt cggcttcagg gtcttagtgt acctcagccc tatgtgcaga ggtaccattc 1620

agagccttat gtgcagaggt atcattcaga ttcattcatt ccaaaagagg aacagagaaa 1680

agtgcagcaa gcattcccag tttttgaagg agctgaggga gggcgtgttc acgctccggt 1740

agagtatata cagattaagg agctcacaga agcagttcac aattatgtag tcagcgctaa 1800

tttcactata gcacaagtcg aaaggcttgc gaatcacgct atgactcctg gtgattggca 1860

gactgtagta aaagctgcag cccccagtat gggaatgtat cttgagtgga aagccttgtg 1920

gcaggactcc tgccagacac aggcaagggc caatgccacc atagaagggg accaaagaac 1980

gtggacgttt gaattattta caggtcaggg acagcatgtt gctaaccaaa cagattatca 2040

ttggggagca tacgctcaga tctcagctgc cgctgttaag gcatgaaagg cactctctaa 2100

gaagggagag gcaagtgggc atttgaccaa gattactcaa ggtgcacagg agtccttctc 2160

agactttgtg gacagaatga cggaggcagc agggaggata tttggagatc cagaagcagc 2220

tatgccttta attgaacaat tggtttatga acaagctacg caggaatgca gatcagcaat 2280

tacacctaga aagagtaagg ggttacagga ctggttaaag gtttgccgtg agcttggagg 2340

gccgcttact aatgctggat tggcagcagc cattctgcag gggaggaggt gctcggattc 2400

aactgagctc aaactttgct ttaattgtgg caaaccaaga catttaaaaa aggactgtag 2460

agcccctata aaaaggacag caccaggatt gtgtactaaa tgtggaaaag gatatcattg 2520

ggctaaggac tgtcgttcaa ttagagatat acgaggcagg ctcttgcagc ctacaaggtc 2580

ttattgtcca tcctggagtt gtagatcagg attataaagg ggaacttcag gttctctgtt 2640

cttgtcctca aggtgtcttt tctatatcac aaggagacag aatagctcaa ctaataattt 2700

tgccaagcct acatggccgt tttccttcct ctggtattcc tcgagctgcc agagggattg 2760

gttctactgg aaatgattct gcttacttaa taatgccttt agattccagg ccatccttgg 2820

agttagttat agaagggaaa caatttaagg gaattttaga tacaggagca gacaaaagta 2880

ttatttcttc tcactggtgg ccaaaaacct ggcccgtaac tcagtcatca cactctttac 2940

aagggctagg ctatcagtcc tgctccacta ttagctcccg ttctttgagc tggcaaatgg 3000

atgtcacgca tatttcctcc tttgggaaaa atcaatattt acatgtttgc taatctgtct 3060

aggtagattt ctttgtatct tgcaggaaat tggtctgaaa gattcgatga gacccttgag 3120

gccctgagag cggctgttct caggatcaac tcgatacgag tggacctgtc attgacagag 3180

ggtctctcct cctggatttc atctgctttt tcttatttta aggaatgggt ggggtggttt 3240

tgtttgctgc tgccatctgc tgtggacttg tgtttatgct ctggttagtt tgcaagctca 3300

gaacccaaca aaaccgtgac aaggtcgtta tcgctcaagc acttgcagcc attgaacaag 3360

gggcctcccc tgaaatttgg ctgtccatgc ttaagaccta aatggcatct gagttctctg 3420

ctattgcgtc ccacggattt gtggatacat tgcactggga tgtgagagac tcaacgctca 3480

ttgatcagca cttacacctc gctgaggttt actaattcgt tttttggctg gcctctttta 3540

tagagttcgc cctaggtcat ttagcagtta ctgtcacaca gtactgcggt attcagagac 3600

gggcaacttt cctcatgcac agaccaacct aagacacggg accccgtggc gatagggtta 3660

ccctatgaca ggaaaggact gacctgtcat agggtaacct gtcattcctg gaggaatgac 3720

ctaagacagg agccacaata gatggacatg actgcagagg cctagaccag cctcaaattt 3780

taataaaata aaaaggggga gatgtggaga gccgtgccat gagcaatcgc cattataaaa 3840

tggcactggc ttccactgcg cctaactagt taacaagcct tatgcgcaag tgcaagagtg 3900

aactcacgcc tatcactgcc catctcgcag tgtagtaatg gggtgatggg cgagcaacga 3960

atcaggagct gtcacaccac atcaggtgct gaaacgtcac gctgtgggct atacaagcag 4020

cgccatttcc ccggttcggg gtcttccctc ctgataagta agcaataaaa gctttgccgc 4080

agaagattct ggttgcagga ttgggggcag ggtgtgccag gttgggactg agggatgctg 4140

ggagaactta gaagccaggt ctgagctggg cagtggtggc gcacgcctct aataccagca 4200

ctcaggaggc agaggcaggc ggatttctga gtttgaggcc agcctggtct acaaagtgag 4260

ttccaggaca gccagggcta cacagagaaa ccctgtcttg aaaaaccaaa gaaacaaaaa 4320

agaaagaaag aaaaaaaaag aagccaggtc tgctttggta tgcaaaacat gtgcatgctc 4380

gcgtgtgtgt gtgcacacac acacactgtc ccctgtccca gggtctgaag ccaaacaaaa 4440

aggtcactcc tctgcttcct gcttatgtgt agcatgcaag ctctcagcta ctgctccagg 4500

atcatgtttg tctatctgtc tgtctgctgc catgctccct gtcataatgg ttatagactc 4560

accctctgaa actgtaacca ggcccccaat taaatgcttt cttgtataag ttggtcatgg 4620

tgtctcttca cagcggaaga accatgacta agacaaggat ctaaagcaaa gagcctcgga 4680

cagctccaaa acttgctggg tagcatatgg acagaaacat cttgtctgac aagaaagcca 4740

acatagtaat gagagcatct gcaggcagga gccaggagaa gactaacgac taataagtca 4800

acataaaaca accctcagat aaaatcttcc tccgcaatgt tcccttttgt gtcctctaac 4860

attccaagcc ccctgcccct cgtacaatct ccatttctct agtaaaggac tgcctgctct 4920

gttgagctga tgctggcttt tatttccatg ttgccttcat cagttccgat ctaacaccca 4980

ctcccactcc cttttaagac aggggctcac tggctggctt ggaactcaca gagctccaac 5040

tgctgctgcc tcccaggtac tgtgattaag tctgtgctac cacatcctaa ggttttgttt 5100

cttctggcaa tattgggtac caaggacttt gtaagtgtcc tactactgag ctacaccccc 5160

agcccctcac tgggggattc taggcagggg ctcacacctc tactggctgc tggggttaaa 5220

tgagaaagtt ttaccccttt aagggtgtcc ccagcgccag gcatcagggt actcactgat 5280

acatcatcat tgtcatggtt gtctgagtct ggagcccaca agtctaccag ggcctagggc 5340

ctcccatgct ctgtgagcct gagagaaagg aagagactcg tagggcagtg gcctctgtta 5400

tcatgctccg gaggcctggg caaggggatc tggctattct gcccagggtt ctcacactgg 5460

aaatactaaa tatttacact tgctgtgctc ccaggctcca tccatgctct gggggatggg 5520

tggtttccaa gtcttctctc ggcttctgtc ttttcagtct cctgcaccca gatactcacc 5580

cagaaggact cccggtcctt cgtcctggtc cttcaggcct tcctcagatt ttgtggaatt 5640

gccgcaaacc tcactggagc cagcgtctgg gctgtgagag gtgagagtgg gtaggagtgg 5700

atgtaggtag atgctgtttg tagagtgcat ctggtcttga ttgtgtgtgt acgggtgtgt 5760

atgtatgttg tctatgtatg tgcatgtata tgtatgtgcg tatgtatgta tgtgtatgtg 5820

tgtgtgtgtg tgtatacgtt gtgtgtgggg gggcacaggg aggctcccag gtctctcatt 5880

tactccaagc ttgccaacga aggcggccac atgtgcccaa gactgcccag tgcttgggag 5940

ctgacgcctg atgagcagcc actgtagttg atgtaccttg cctctagctc agaagtttcc 6000

acggctctct cacttccctt ctatgccacc ccacaggcct ctttgctatg tcttcttttc 6060

agcacatctg aaatgagatc caattccagg acctttgcac ttgtggtttc ttctgagcag 6120

gtccaagtgt gtgcccgagt gcctgggagt ggtggcatag ggccctgggt gtgaattctg 6180

cagggcagga gtggggagtc acgaatatta acctggtatg gtggcacaca caagccatct 6240

agggatgaag aggcccaggc agtagcatcc taacctagtt cggtaacgga ctgggctact 6300

agtgacacca tgtctcaaat tagaaaagag ggcccagcca tgagtgatgg atgctccatc 6360

ccctgcacgc cccgcccccc acgcaaaatg gaagttttct ctttaagtgg gggccaccga 6420

aggttctgag gggagaggtg accaggtcca agtgtccgga accgaggatg gagagggatt 6480

tggggtgtaa aatgattctg gttgttgtat gaactgtggt aaacggacaa tctgcgctgc 6540

gagctgcagc ccagctctgt tgtggagtac aaccgtagga agtgacgccc agggatcaat 6600

actttagctc tccttggcta ccttggagct ttgggacccg tctaggctaa aacagtagtc 6660

tctttcctct tcactgcagg tgac                                              6684


<210>  3
<211>  17
<212>  PRT
<213>  Homo sapiens

<400>  3

Ser Arg Met Leu Asp Pro Ala Pro Glu Pro Gln Arg Asp Gln Thr Gln
1               5                   10                  15


Arg




<210>  4
<211>  18
<212>  PRT
<213>  Artificial Sequence

<220>
<223>  Description of Artificial Sequence: Peptide A

<400>  4

Cys Ser Arg Met Leu Asp Pro Ala Pro Glu Pro Gln Arg Asp Gln Thr
1               5                   10                  15


Gln Arg




<210>  5
<211>  16
<212>  PRT
<213>  Mus musculus

<400>  5

Asn Thr Ser Arg Arg Leu Asp Gln Thr Pro Glu His Gln Arg Glu Gln
1               5                   10                  15

<210> 6
<211> 28
<212> DNA
<213> Artificial Sequence

<220>
<223> Description of Artificial Sequence: an artificially synthesized
      primer sequence

<400> 6
ctagactagt tgcaaaggcg tgcggcgg                                        28

<210> 7
<211> 36
<212> DNA
<213> Artificial Sequence

<220>
<223> Description of Artificial Sequence: an artificially synthesized
      primer sequence

<400> 7
ctagactagt ggatccgcag gcttgctttg acttac                               36

<210> 8
<211> 536
<212> DNA
<213> Mus musculus

<400> 8
gcccatggag ggattgtgca gaggctcacg gggcaggtca ctggcagagt ggagtgtatg     60

acctgcggct gggccgtcgt gtagtagccg tgtggtgtga acagcagcag gaagtggagg    120

ctggactgtc atccagagac ggcaggacgg ctctgtcaac ttcttcacca actggcagca    180

ctacaaggtg tgtgcttgtg gtgggggtgt cagagactgc tgggcagaga ggacgccccc    240

accctcttcc tcctaccctt ccaggcgggc tttgggcgtc cagaaggaga atactggctg    300

ggcctggaac ctgtgcatca ggtgacaagc cgtggggacc acgagctgct gatactccta    360

gaggactggg ggggccgtgc agcacgcgcc cactacgaca gcttctcctt ggagcctgag    420

agtgaccact accgtctgcg gcttggccag taccacggcg atgccggaga ctccctctct    480

tggcacaatg acaaaacctt tcagcactgt ggatagggac agagactcat attctg        536


<210>  9
<211>  20
<212>  DNA
<213>  Artificial Sequence

<220>
<223>  Description of Artificial Sequence: an artificially synthesized
       primer sequence

<400>  9
agaggctatt cggctatgac                                                  20


<210>  10
<211>  20
<212>  DNA
<213>  Artificial Sequence

<220>
<223>  Description of Artificial Sequence: an artificially synthesized
       primer sequence

<400>  10
caccatgata ttcggcaagc                                                  20


<210>  11
<211>  20
<212>  DNA
<213>  Mus musculus

<400>  11
tggcctctgt tatcatgctc                                                  20

<210>  12
<211>  20
<212>  DNA
<213>  Mus musculus

<400>  12
ctacctacat ccactcctac                                                    20


<210>  13
<211>  31
<212>  DNA
<213>  Artificial Sequence

<220>
<223>  Description of Artificial Sequence: an artificially synthesized
       primer sequence

<400>  13
agaagcttca ccatggggac cgccaggcta c                                       31


<210>  14
<211>  39
<212>  DNA
<213>  Artificial Sequence

<220>
<223>  Description of Artificial Sequence: an artificially synthesized
       primer sequence

<400>  14
ccgtcgacat tagatcttca caagcgcaca agccgggtc                               39


<210>  15
<211>  81
<212>  DNA
<213>  Artificial Sequence

<220>
<223>  Description of Artificial Sequence: Sequence containing loxP

<400>  15
gatccggaac ccttaatata acttcgtata atgtatgcta tacgaagtta ttaggtccct     60

cgacctgcag cccgggggat c                                               81



<210>  16
<211>  20
<212>  DNA
<213>  Artificial Sequence

<220>
<223>  Description of Artificial Sequence: an artificially synthesized
       primer sequence

<400>  16
aattaaccct cactaaaggg                                                 20



<210>  17
<211>  20
<212>  DNA
<213>  Mus musculus

<400>  17
cccactacga cagcttctcc                                                 20



<210>  18
<211>  20
<212>  DNA
<213>  Mus musculus

<400>  18
agccgggtca acataacagc                                                 20



<210>  19
<211>  19
<212>  DNA
<213>  Artificial Sequence

<220>

<223>  Description of Artificial Sequence: an artificially synthesized
       primer sequence

<400>  19
gcgttaccca acttaatcg                                                    19



<210>  20
<211>  18
<212>  DNA
<213>  Artificial Sequence

<220>
<223>  Description of Artificial Sequence: an artificially synthesized
       primer sequence

<400>  20
tgtgagcgag taacaacc                                                     18



<210>  21
<211>  22
<212>  DNA
<213>  Mus musculus

<400>  21
tcgtgtagta gccgtgtggt gt                                                22



<210>  22
<211>  20
<212>  DNA
<213>  Mus musculus

<400>  22
cacctgatgc acaggttcca                                                   20



<210>  23
<211>  19
<212>  DNA
<213>  Mus musculus

<400>  23

tggttgatcc tgccagtag 19

<210> 24
<211> 20
<212> DNA
<213> Mus musculus

<400> 24
cgaccaaagg aaccataact 20

<210> 25
<211> 23
<212> DNA
<213> Mus musculus

<400> 25
ccggtacagt gaaactgcga atg 23

<210> 26
<211> 17
<212> PRT
<213> Mus musculus

<400> 26

Asn Asp Lys Pro Phe Ser Thr Val Asp Arg Asp Arg Asp Ser Tyr Ser
1               5                   10                  15

Gly

<210> 27
<211> 17
<212> PRT
<213> Artificial Sequence

<220>
<223> Description of Artificial Sequence: Peptide YP-126

<400>  27

Asn Thr Ser Arg Arg Leu Asp Gln Thr Pro Glu His Gln Arg Glu Gln
1               5                   10                  15

Cys

<210>  28
<211>  18
<212>  PRT
<213>  Artificial Sequence

<220>
<223>  Description of Artificial Sequence: Peptide YP-127-B

<400>  28

Cys Asn Asp Lys Pro Phe Ser Thr Val Asp Arg Asp Arg Asp Ser Tyr
1               5                   10                  15

Ser Gly

<210>  29
<211>  20
<212>  DNA
<213>  Mus musculus

<400>  29
ccgtgtggtg tgaacagcag                                                  20

<210>  30
<211>  22
<212>  DNA
<213>  Mus musculus

<400>  30
tgaagaagtt gacagagccg tc                                               22

<210> 31
<211> 19
<212> DNA
<213> Mus musculus

<400> 31
cggagcgcaa tatgaaggt 19

<210> 32
<211> 20
<212> DNA
<213> Mus musculus

<400> 32
caaaaggaag acgacggagc 20

<210> 33
<211> 20
<212> DNA
<213> Mus musculus

<400> 33
atgtctatag agatggatcc 20

<210> 34
<211> 28
<212> DNA
<213> Artificial Sequence

<220>
<223> Description of Artificial Sequence: an artificially synthesized
primer sequence

<400> 34
ggggatcctc tgggtgcagg agactgaa 28

<210> 35

<211>  27
<212>  DNA
<213>  Artificial Sequence

<220>
<223>  Description of Artificial Sequence: an artificially synthesized
       primer sequence

<400>  35
cccctcgaga atgacgaagg cagcagg                                          27

<210>  36
<211>  27
<212>  DNA
<213>  Artificial Sequence

<220>
<223>  Description of Artificial Sequence: an artificially synthesized
       primer sequence

<400>  36
cccctcgagc tcacagaagc agttcac                                          27

<210>  37
<211>  27
<212>  DNA
<213>  Artificial Sequence

<220>
<223>  Description of Artificial Sequence: an artificially synthesized
       primer sequence

<400>  37
cccctcgagg gaagaatacc tggggag                                          27

<210>  38
<211>  27
<212>  DNA
<213>  Artificial Sequence

<220>
<223>  Description of Artificial Sequence: an artificially synthesized
       primer sequence

<400>  38
cccctcgagt taacaagcct tatgcgc                                          27


<210>  39
<211>  27
<212>  DNA
<213>  Artificial Sequence

<220>
<223>  Description of Artificial Sequence: an artificially synthesized
       primer sequence

<400>  39
cccctcgaga gagaaagggc acagggc                                          27


<210>  40
<211>  27
<212>  DNA
<213>  Artificial Sequence

<220>
<223>  Description of Artificial Sequence: an artificially synthesized
       primer sequence

<400>  40
cccctcgagg catgtaccat cacaccc                                          27


<210>  41
<211>  20
<212>  DNA
<213>  Mus musculus

<400>  41
gtagaaccaa tccctctggc                                                  20


<210>  42

<211>  26
<212>  DNA
<213>  Artificial Sequence

<220>
<223>  Description of Artificial Sequence: an artificially synthesized
       primer sequence

<400>  42
ggggatccgt cacctgcagt gaagag                                          26


<210>  43
<211>  20
<212>  DNA
<213>  Homo sapiens

<400>  43
gaacccgaga gcgaccacta                                                 20


<210>  44
<211>  20
<212>  DNA
<213>  Homo sapiens

<400>  44
ccctatccac ggtgctgaag                                                 20


<210>  45
<211>  32
<212>  DNA
<213>  Artificial Sequence

<220>
<223>  Description of Artificial Sequence: an artificially synthesized
       primer sequence

<400>  45
aatctagaca ccatggggac cgccaggcta cg                                   32


<210>  46

<211>  31
<212>  DNA
<213>  Artificial Sequence

<220>
<223>  Description of Artificial Sequence: an artificially synthesized
      primer sequence

<400>  46
aagcggccgc caagcgcaca agccgggtca a                                    31


<210>  47
<211>  32
<212>  DNA
<213>  Artificial Sequence

<220>
<223>  Description of Artificial Sequence: an artificially synthesized
      primer sequence

<400>  47
gaagatctac catggggacc gccaggctac gc                                   32


<210>  48
<211>  30
<212>  DNA
<213>  Artificial Sequence

<220>
<223>  Description of Artificial Sequence: an artificially synthesized
      primer sequence

<400>  48
ccgctcgagt gatgggacag tcacaagcgc                                      30


<210>  49
<211>  41
<212>  DNA
<213>  Artificial Sequence

<220>

<223>  Description of Artificial Sequence: an artificially synthesized
      primer sequence

<400>  49
ccaagcttac catggggaag ccctggctgc gtgcgctaca g                          41



<210>  50
<211>  35
<212>  DNA
<213>  Artificial Sequence

<220>
<223>  Description of Artificial Sequence: an artificially synthesized
      primer sequence

<400>  50
aactcgagca gcttcagggg ccgaatgagc atggc                                35



<210>  51
<211>  21
<212>  DNA
<213>  Rattus sp.

<400>  51
ctggcaacat tacaaggtgg g                                                21



<210>  52
<211>  21
<212>  DNA
<213>  Rattus sp.

<400>  52
ctagtcacct gatgcacggg t                                                21



<210>  53
<211>  34
<212>  DNA
<213>  Artificial Sequence

<220>

<223> Description of Artificial Sequence: an artificially synthesized
      primer sequence

<400> 53
ggacgatgat gacaagggat ccactagtct cgag                                    34


<210> 54
<211> 34
<212> DNA
<213> Artificial Sequence

<220>
<223> Description of Artificial Sequence: an artificially synthesized
      primer sequence

<400> 54
ctcgagacta gtggatccct tgtcatcatc gtcc                                    34


<210> 55
<211> 28
<212> DNA
<213> Artificial Sequence

<220>
<223> Description of Artificial Sequence: an artificially synthesized
      primer sequence

<400> 55
ttggatccgc ttctcccatg cctgcagg                                           28


<210> 56
<211> 28
<212> DNA
<213> Artificial Sequence

<220>
<223> Description of Artificial Sequence: an artificially synthesized
      primer sequence

<400> 56
ttctcgagtc acagcttcag ggccgaa                                            28

SEQUENCE LISTING

<110> Astellas Pharma Inc.
<110> KEIO UNIVERSITY

<120> Method of screening antiobesity agents

<130> AP05056-751

<150> JP 2004-351814
<151> 2004-12-03

<160> 56

<170> PatentIn version 3.1

<210> 1
<211> 5572
<212> DNA
<213> Mus musculus

<220>
<223> Inventor: Yasunaga, Kunio; Yamaji, Noboru; Abe, Kunitake
Inventor: Oike, Yuichi

<400> 1
atgtctatag agatggatcc aatatattaa cagagaggtc acccagccca gaaatcccta 60

atgtgcttta aatcaggcct gcaagctcac ttgggtgtct ctctgtcttg gtactggaga 120

gaccccaaca tgctggacct ctgctgaata aaacactctt cgcctttaca tactccttga 180

gtccagggtg tcattcttcg gcgaatcatg agcccttaca cctgagtgtt gggataaaag 240

gcatgtacca tcacacccag ggaatagttc gtgactgtgg tagtttgaag aaggctcata 300

tatttgaaca tttggtaccc acttggtgga acagtttggg aaggattagt gggtgtgacc 360

ttattggagg agattcgtca ctttgtggat tcaaaagtga tgttcttttt ctgcccgtga 420

gccaattcaa gacagattag attagattaa atgcagattt cttgggaaac tgctccccgg 480

tgggttcact agccccaagg actgaggcca cggaagtcac cacatggggg gttggaggga 540

aggataaaga gagaaagggc acagggcagg aagagagaga aagtgaaggg ggtggggaga 600

ccaaaatgtg gagagccgtg ccgtgagcaa tcgccattat aagatggcac tggcttccac 660

tgcgcctaac tagttaacaa gccttatgcg caagtgcaac agtgaactca tgcctagtca 720

ctgcccatct cgtcgcgtag taatggggtg atgggcgagc aacgaatcag gagctgtcac 780

gccacatcag gtgctgaaac gtcacactgc gggctatata agcagcgcca ttttcccggt 840

tcagagtctt ccctcctgat aagtaagcaa tacaagcttt gccgcagaag attccagttg 900

tcctgagtgt tttcttgccg gcggggacaa aagctcaggc aataagtggc gctgaggaac 960

ccaggaacta ccatcacacc ggcgcaaggg agatctctcg ttcgcggggc ggaaccagaa 1020

ctgcgggaca cattacagct ttacgaggta tgtctggcct tgaactttct ccagtgttag 1080

aagccttttg ttccttttca catgtcaaca agtggttaag gcagggcaga gaattctgga 1140

cgaaattcaa gacagtctat cagaggtaaa gccgggagag agagtaggag caaagaggaa 1200

acatggtgca ccaattaagc atacaggcct ttccacgggt cttgaacccg aggaaaagtt 1260

aatgtcaggg aagaatacct ggggagagat tggaaggaag gagaaggaaa aagaaaagaa 1320
aaaagatcaa tcagcggagg tttctaggag aaggaactta tacccgccac tagatgagtt 1380
aaagcggcta gctcttagta gctcagaatc agatgaagga cttagctcct ctgaagaaat 1440
ggacttggag gaggaagcag ttcgttataa gggagaaagg taccagcaag ataaaaagcg 1500
agctactcag tccagaaaaa ggcaaaaagc ggctgatgaa agccagcttg ctgcttggcc 1560
tccggactgt cggcttcagg gtcttagtgt acctcagccc tatgtgcaga ggtaccattc 1620
agagccttat gtgcagaggt atcattcaga ttcattcatt ccaaaagagg aacagagaaa 1680
agtgcagcaa gcattcccag tttttgaagg agctgaggga gggcgtgttc acgctccggt 1740
agagtatata cagattaagg agctcacaga agcagttcac aattatgtag tcagcgctaa 1800
tttcactata gcacaagtcg aaaggcttgc gaatcacgct atgactcctg gtgattggca 1860
gactgtagta aaagctgcag cccccagtat gggaatgtat cttgagtgga aagccttgtg 1920
gcaggactcc tgccagacac aggcaagggc caatgccacc atagaagggg accaaagaac 1980
gtggacgttt gaattattta caggtcaggg acagcatgtt gctaaccaaa cagattatca 2040
ttggggagca tacgctcaga tctcagctgc cgctgttaag gcgtgaaagg cactctctaa 2100
gaagggagag gcaagtgggc atttgaccaa gattactcaa ggtgcacagg agtccttctc 2160
agactttgtg gacagaatga cggaggcagc agggaggata tttggagatc cagaagcagc 2220
tatgccttta attgaacaat tggtttatga acaagctacg caggaatgca gatcagcaat 2280
tacacctaga aagagtaagg ggttacagga ctggttaaag gtttgccgtg agcttggagg 2340
gccgcttact aatgctggat tggcagcagc cattctgcag gggaggaggt gctcggattc 2400
aactgagctc aaactttgct ttaattgtgg caaaccaaga catttaaaaa aggactgtag 2460
agcccctata aaaaggacag caccaggatt gtgtactaaa tgtggaaaag gatatcattg 2520
ggctaaggac tgtcgttcaa ttagagatat acgaggcagg ctcttgcggc ctacaaggtc 2580
ttattgtcca tcctggagtt gtagatcagg attataaagg ggaacttcag gttctctgtt 2640
cttgtcctca aggtgtcttt tctatatcac aaggagacag aatagctcaa ctaataattt 2700
tgccaagcct acatggccgt tttccttcct ctggtattcc tcgagctgcc agagggattg 2760
gttctactgg aaatgattct gcttacttaa taatgccttt agattccagg ccatccttgg 2820
agttagctat agaaggaaaa caatttaagg gaattttaga tacaggagca gacaaaagta 2880
ttatttcttc tcactggtgg ccaaaaacct ggcccgtaac tcagtcatca cactctttac 2940
aagggctagg ctatcagtcc tgctccacta ttagctcccg ttctttgagc tggcaaatgg 3000
atgtcacgca tatttcctcc tttgggaaaa atcaatattt acatgtttgc taatctgtct 3060
aggtagattt ctttgtatct tgcaggaaat tggtctgaaa aattcgatga gacccttgag 3120
gccctgagag cggctgttct caggatcaac tcgatacgag tggacctgtc attgacagag 3180
ggtctctcct cctggatttc atctgctttt tcttatttta aggaatgggt ggggtggttt 3240
tgtttgctgc tgccatctgc tgtggacttg tgtttatgct ctggttagtt tgcaagctca 3300

gaacccaaca aaaccgtgac aagatcgtta tcgctcaagc acttgcagcc attgaacaag 3360
gggcctcccc tgaaatttgg ctgtccatgc ttaagaccta aatggcatct gagttctctg 3420
ctattgcgtc ccacggattt gtggatacat tgcactggga tgtgagagac tcaacgctca 3480
ttgatcagca cttacacctc gctgaggttt actaattcgt tttttggctg gcctctttta 3540
tagagttcgc cctaggtcat ttagcagtta ctgtcacaca gtactgcggt attcagagac 3600
gggcaacttt cctcatgcac agaccaacct aagacacggg accccgtggc gatagggtta 3660
ccctatgaca ggaaaggact gacctgtcat agggtaacct gtcattcctg gaggaatgac 3720
ctaagacagg agccacaata gatggacatg actgcagagg cctagaccag cctcaaattt 3780
taataaaata aaaaggggga gatgtggaga gccgtgccgt gagcaatcgc cattataaaa 3840
tggcactggc ttccactgcg cctaactagt taacaagcct tatgcgcaag tgcaagagtg 3900
aactcacgcc tatcactgcc catctcgcag tgtagtaatg gggtgatggg cgagcaacga 3960
atcaggagct gtcacaccac atcaggtgct gaaacgtcac gctgtgggct atacaagcag 4020
cgccatttcc ccggttcggg gtcttccctc ctgataagta agcaataaaa gctttgccgc 4080
agaagattct ggttgcagga ttgggggcag ggtgtgccag gttgggactg agggatgctg 4140
ggagaactta gaagccaggt ctgagctggg cagtggtggc gcacgcctct aataccagca 4200
ctcaggaggc agaggcaggc ggatttctga gtttgaggcc agcctggtct acaaagtgag 4260
ttccaggaca gccagggcta cacagagaaa ccctgtcttg aaaaaccaaa gaaacaaaaa 4320
agaaagaaag aaaaaaaaag aagccaggtc tgctttggta tgcaaaacat gtgcatgctc 4380
gcgtgtgtgt gtgcacacac acacactgtc ccctgtccca gggtctgaag ccaaacaaaa 4440
aggtcactcc tctgcttcct gcttatgtgt agcatgcaag ctctcagcta ctgctccagg 4500
atcatgtttg tctatctgtc tgtctgctgc catgctccct gtcataatgg ttatagactc 4560
accctctgaa actgtaacca ggcccccaat taaatgcttt cttgtataag ttggtcatgg 4620
tgtctcttca cagcagaaga accatgacta agacaaggat ctaaagcaaa gagcctcgga 4680
cagctccaaa acttgttggg tagcatatgg acagaaacat cttgtctgac aagaaagcca 4740
acatagtaat gagagcatct gcaggcagga gccaggagaa gactaacgac taataagtca 4800
acataaaaca accctcagat aaaatcttcc tccgcaatgt tcccttttgt gtcctctaac 4860
attccaagcc ccctgcccct cgtacaatct ccatttctct agtaaaggac tgcctgctct 4920
gttgagctga tgctggcttt tatttccatg ttgccttcat cagttccgat ctaacaccca 4980
ctcccactcc cttttaagac aggggctcac tggctggctt ggaactcaca gagctccaac 5040
tgctgctgcc tcccaggtac tgtgattaag tctgtgctac cacatcctaa ggttttgttt 5100
cttctggcaa tattgggtac caaggacttt gtaagtgtcc tactactgag ctacaccccc 5160
agcccctcac tgggggattc taggcagggg ctcacacctc tactggctgc tggggttaaa 5220
tgagaaagtt ttaccccttt aagggtgtcc ccagcgccag gcatcagggt actcactgat 5280
acatcatcat tgtcatggtt gtctgagtct ggagcccaca agtctaccag ggcctagggc 5340

ctcccatgct ctgtgagcct gagagaaagg aagagactcg tagggcagtg gcctctgtta 5400

tcatgctccg gaggcctggg caaggggatc tggctattct gcccagggtt ctcacactgg 5460

aaatactaaa tatttacact tgctgtgctc ccaggctcca tccatgctct gggggatggg 5520

tggtttccaa gtcttctctc ggcttctgtc ttttcagtct cctgcaccca ga 5572

<210> 2
<211> 6684
<212> DNA
<213> Mus musculus

<400> 2
atgtctatag agatggatcc aatatattaa cagagaggtc acccagccca gaaatcccta 60

atgtgcttta aatcaggcct gcaagctcac ttgggtgtct ctctgtcttg gtactggaga 120

gaccccaaca tgctggacct ctgctgaata aaacactctt cgcctttaca tactccttga 180

gtccagggtg tcattcttcg gcgaatcatg agcccttaca cctgagtgtt gggataaaag 240

gcatgtacca tcacacccag ggaatagttc gtgactgtgg tagtttgaag aaggctcata 300

tatttgaaca tttggtaccc acttggtgga acagtttggg aaggattagt gggtgtgacc 360

ttattggagg agattcgtca ctttgtggat tcaaaagtga tgttcttttt ctgcccgtga 420

gccaattcaa gacagattag attagattaa atgcagattt cttgggaaac tgctccccgg 480

tgggttcact agccccaagg actgaggcca cggaagtcac cacatggggg gttggaggga 540

aggataaaga gagaaagggc acagggcagg aagagagaga aagtgaaggg ggtggggaga 600

ccaaaatgtg gagagccgtg ccgtgagcaa tcgccattat aagatggcac tggcttccac 660

tgcgcctaac tagttaacaa gccttatgcg caagtgcaac agtgaactca tgcctagtca 720

ctgcccatct cgtcgcgtag taatggggtg atgggcgagc aacgaatcag gagctgtcac 780

gccacatcag gtgctgaaac gtcacactgc gggctatata agcagcgcca ttttcccggt 840

tcagagtctt ccctcctgat aagtaagcaa tacaagcttt gccgcagaag attccagttg 900

tcctgagtgt tttcttgccg gcggggacaa aagctcaggc aataagtggc gctgaggaac 960

ccaggaacta ccatcacacc ggcgcaaggg agatctctcg ttcgcggggc ggaaccagaa 1020

ctgcgggaca cattacagct ttacgaggta tgtctggcct tgaactttct ccagtgttag 1080

aagccttttg ttccttttca catgtcaaca agtggttaag gcagggcaga gaattctgga 1140

cgaaattcaa gacagtctat cagaggtaaa gccgggagag agagtaggag caaagaggaa 1200

acatggtgca ccaattaagc atacaggcct ttccacgagt cttgaacccg aggaaaagtt 1260

aatgtcaggg aagaatacct ggggagagat tggaaggaag gagaaggaaa aagaaaagaa 1320

aaaagatcaa tcagcggagg tttctaggag aaggaactta tacccgccac tagatgagtt 1380

aaagcggcta gctcttagta gctcagaatc agatgaagga cttagctcct ctgaagaaat 1440

ggacttggag gaggaagcag ttcgttataa gggagaaagg taccagcaag ataaaaagcg 1500

agctactcag tccagaaaaa ggcaaaaagc ggctggtgaa agccagcttg ctgcttggcc 1560

tccggactgt cggcttcagg gtcttagtgt acctcagccc tatgtgcaga ggtaccattc 1620

agagccttat gtgcagaggt atcattcaga ttcattcatt ccaaaagagg aacagagaaa 1680
agtgcagcaa gcattcccag tttttgaagg agctgaggga gggcgtgttc acgctccggt 1740
agagtatata cagattaagg agctcacaga agcagttcac aattatgtag tcagcgctaa 1800
tttcactata gcacaagtcg aaaggcttgc gaatcacgct atgactcctg gtgattggca 1860
gactgtagta aaagctgcag cccccagtat gggaatgtat cttgagtgga aagccttgtg 1920
gcaggactcc tgccagacac aggcaagggc caatgccacc atagaagggg accaaagaac 1980
gtggacgttt gaattattta caggtcaggg acagcatgtt gctaaccaaa cagattatca 2040
ttggggagca tacgctcaga tctcagctgc cgctgttaag gcatgaaagg cactctctaa 2100
gaagggagag gcaagtgggc atttgaccaa gattactcaa ggtgcacagg agtccttctc 2160
agactttgtg gacagaatga cggaggcagc agggaggata tttggagatc cagaagcagc 2220
tatgccttta attgaacaat tggtttatga acaagctacg caggaatgca gatcagcaat 2280
tacacctaga aagagtaagg ggttacagga ctggttaaag gtttgccgtg agcttggagg 2340
gccgcttact aatgctggat tggcagcagc cattctgcag gggaggaggt gctcggattc 2400
aactgagctc aaactttgct ttaattgtgg caaaccaaga catttaaaaa aggactgtag 2460
agcccctata aaaaggacag caccaggatt gtgtactaaa tgtggaaaag gatatcattg 2520
ggctaaggac tgtcgttcaa ttagagatat acgaggcagg ctcttgcagc ctacaaggtc 2580
ttattgtcca tcctggagtt gtagatcagg attataaagg ggaacttcag gttctctgtt 2640
cttgtcctca aggtgtcttt tctatatcac aaggagacag aatagctcaa ctaataattt 2700
tgccaagcct acatggccgt tttccttcct ctggtattcc tcgagctgcc agagggattg 2760
gttctactgg aaatgattct gcttacttaa taatgccttt agattccagg ccatccttgg 2820
agttagttat agaagggaaa caatttaagg gaattttaga tacaggagca gacaaaagta 2880
ttatttcttc tcactggtgg ccaaaaacct ggcccgtaac tcagtcatca cactctttac 2940
aagggctagg ctatcagtcc tgctccacta ttagctcccg ttctttgagc tggcaaatgg 3000
atgtcacgca tatttcctcc tttgggaaaa atcaatattt acatgtttgc taatctgtct 3060
aggtagattt ctttgtatct tgcaggaaat tggtctgaaa gattcgatga gacccttgag 3120
gccctgagag cggctgttct caggatcaac tcgatacgag tggacctgtc attgacagag 3180
ggtctctcct cctggatttc atctgctttt tcttatttta aggaatgggt ggggtggttt 3240
tgtttgctgc tgccatctgc tgtggacttg tgtttatgct ctggttagtt tgcaagctca 3300
gaacccaaca aaaccgtgac aaggtcgtta tcgctcaagc acttgcagcc attgaacaag 3360
gggcctcccc tgaaatttgg ctgtccatgc ttaagaccta aatggcatct gagttctctg 3420
ctattgcgtc ccacggattt gtggatacat tgcactggga tgtgagagac tcaacgctca 3480
ttgatcagca cttacacctc gctgaggttt actaattcgt tttttggctg gcctctttta 3540
tagagttcgc cctaggtcat ttagcagtta ctgtcacaca gtactgcggt attcagagac 3600
gggcaacttt cctcatgcac agaccaacct aagacacggg accccgtggc gatagggtta 3660

ccctatgaca ggaaaggact gacctgtcat agggtaacct gtcattcctg gaggaatgac 3720
ctaagacagg agccacaata gatggacatg actgcagagg cctagaccag cctcaaattt 3780
taataaaata aaaaggggga gatgtggaga gccgtgccat gagcaatcgc cattataaaa 3840
tggcactggc ttccactgcg cctaactagt taacaagcct tatgcgcaag tgcaagagtg 3900
aactcacgcc tatcactgcc catctcgcag tgtagtaatg gggtgatggg cgagcaacga 3960
atcaggagct gtcacaccac atcaggtgct gaaacgtcac gctgtgggct atacaagcag 4020
cgccatttcc ccggttcggg gtcttccctc ctgataagta agcaataaaa gctttgccgc 4080
agaagattct ggttgcagga ttgggggcag ggtgtgccag gttgggactg agggatgctg 4140
ggagaactta gaagccaggt ctgagctggg cagtggtggc gcacgcctct aataccagca 4200
ctcaggaggc agaggcaggc ggatttctga gtttgaggcc agcctggtct acaaagtgag 4260
ttccaggaca gccagggcta cacagagaaa ccctgtcttg aaaaaccaaa gaaacaaaaa 4320
agaaagaaag aaaaaaaaag aagccaggtc tgctttggta tgcaaaacat gtgcatgctc 4380
gcgtgtgtgt gtgcacacac acacactgtc ccctgtccca gggtctgaag ccaaacaaaa 4440
aggtcactcc tctgcttcct gcttatgtgt agcatgcaag ctctcagcta ctgctccagg 4500
atcatgtttg tctatctgtc tgtctgctgc catgctccct gtcataatgg ttatagactc 4560
accctctgaa actgtaacca ggcccccaat taaatgcttt cttgtataag ttggtcatgg 4620
tgtctcttca cagcggaaga accatgacta agacaaggat ctaaagcaaa gagcctcgga 4680
cagctccaaa acttgctggg tagcatatgg acagaaacat cttgtctgac aagaaagcca 4740
acatagtaat gagagcatct gcaggcagga gccaggagaa gactaacgac taataagtca 4800
acataaaaca accctcagat aaaatcttcc tccgcaatgt tcccttttgt gtcctctaac 4860
attccaagcc ccctgcccct cgtacaatct ccatttctct agtaaaggac tgcctgctct 4920
gttgagctga tgctggcttt tatttccatg ttgccttcat cagttccgat ctaacaccca 4980
ctcccactcc cttttaagac aggggctcac tggctggctt ggaactcaca gagctccaac 5040
tgctgctgcc tcccaggtac tgtgattaag tctgtgctac cacatcctaa ggttttgttt 5100
cttctggcaa tattgggtac caaggacttt gtaagtgtcc tactactgag ctacaccccc 5160
agcccctcac tgggggattc taggcagggg ctcacacctc tactggctgc tggggttaaa 5220
tgagaaagtt ttaccccttt aagggtgtcc ccagcgccag gcatcagggt actcactgat 5280
acatcatcat tgtcatggtt gtctgagtct ggagcccaca agtctaccag ggcctagggc 5340
ctcccatgct ctgtgagcct gagagaaagg aagagactcg tagggcagtg gcctctgtta 5400
tcatgctccg gaggcctggg caaggggatc tggctattct gcccagggtt ctcacactgg 5460
aaatactaaa tatttacact tgctgtgctc ccaggctcca tccatgctct gggggatggg 5520
tggtttccaa gtcttctctc ggcttctgtc ttttcagtct cctgcaccca gatactcacc 5580
cagaaggact cccggtcctt cgtcctggtc cttcaggcct tcctcagatt ttgtggaatt 5640
gccgcaaacc tcactggagc cagcgtctgg gctgtgagag gtgagagtgg gtaggagtgg 5700

atgtaggtag atgctgtttg tagagtgcat ctggtcttga ttgtgtgtgt acgggtgtgt 5760

atgtatgttg tctatgtatg tgcatgtata tgtatgtgcg tatgtatgta tgtgtatgtg 5820

tgtgtgtgtg tgtatacgtt gtgtgtgggg gggcacaggg aggctcccag gtctctcatt 5880

tactccaagc ttgccaacga aggcggccac atgtgcccaa gactgcccag tgcttgggag 5940

ctgacgcctg atgagcagcc actgtagttg atgtaccttg cctctagctc agaagtttcc 6000

acggctctct cacttccctt ctatgccacc ccacaggcct ctttgctatg tcttcttttc 6060

agcacatctg aaatgagatc caattccagg acctttgcac ttgtggtttc ttctgagcag 6120

gtccaagtgt gtgcccgagt gcctgggagt ggtggcatag ggccctgggt gtgaattctg 6180

cagggcagga gtggggagtc acgaatatta acctggtatg gtggcacaca caagccatct 6240

agggatgaag aggcccaggc agtagcatcc taacctagtt cggtaacgga ctgggctact 6300

agtgacacca tgtctcaaat tagaaaagag gggcccagca tgagtgatgg atgctccatc 6360

ccctgcacgc cccgcccccc acgcaaaatg gaagttttct ctttaagtgg gggccaccga 6420

aggttctgag gggagaggtg accaggtcca agtgtccgga accgaggatg gagagggatt 6480

tggggtgtaa aatgattctg gttgttgtat gaactgtggt aaacggacaa tctgcgctgc 6540

gagctgcagc ccagctctgt tgtggagtac aaccgtagga agtgacgccc agggatcaat 6600

actttagctc tccttggcta ccttggagct ttgggacccg tctaggctaa aacagtagtc 6660

tctttcctct tcactgcagg tgac 6684

<210> 3
<211> 17
<212> PRT
<213> Homo sapiens

<400> 3

Ser Arg Met Leu Asp Pro Ala Pro Glu Pro Gln Arg Asp Gln Thr Gln
1               5                   10                  15

Arg

<210> 4
<211> 18
<212> PRT
<213> Artificial Sequence

<220>
<223> Description of Artificial Sequence: Peptide A

<400> 4

Cys Ser Arg Met Leu Asp Pro Ala Pro Glu Pro Gln Arg Asp Gln Thr
1               5                   10                  15

Gln Arg

<210>  5
<211>  16
<212>  PRT
<213>  Mus musculus

<400>  5

Asn Thr Ser Arg Arg Leu Asp Gln Thr Pro Glu His Gln Arg Glu Gln
1               5                   10                  15


<210>  6
<211>  28
<212>  DNA
<213>  Artificial Sequence

<220>
<223>  Description of Artificial Sequence: an artificially synthesized
       primer sequence

<400>  6
ctagactagt tgcaaaggcg tgcggcgg                                          28


<210>  7
<211>  36
<212>  DNA
<213>  Artificial Sequence

<220>
<223>  Description of Artificial Sequence: an artificially synthesized
       primer sequence

<400>  7
ctagactagt ggatccgcag gcttgctttg acttac                                 36


<210>  8
<211>  536
<212>  DNA
<213>  Mus musculus

<400>  8
gcccatggag ggattgtgca gaggctcacg gggcaggtca ctggcagagt ggagtgtatg      60

acctgcggct gggccgtcgt gtagtagccg tgtggtgtga acagcagcag gaagtggagg     120

ctggactgtc atccagagac ggcaggacgg ctctgtcaac ttcttcacca actggcagca     180

ctacaaggtg tgtgcttgtg gtgggggtgt cagagactgc tgggcagaga ggacgccccc     240

accctcttcc tcctaccctt ccaggcgggc tttgggcgtc cagaaggaga atactggctg     300

ggcctggaac ctgtgcatca ggtgacaagc cgtggggacc acgagctgct gatactccta     360

gaggactggg ggggccgtgc agcacgcgcc cactacgaca gcttctcctt ggagcctgag     420

agtgaccact accgtctgcg gcttggccag taccacggcg atgccggaga ctccctctct     480

tggcacaatg acaaaacctt tcagcactgt ggatagggac agagactcat attctg         536


<210>  9
<211>  20
<212>  DNA
<213>  Artificial Sequence

<220>
<223>  Description of Artificial Sequence: an artificially synthesized
      primer sequence

<400>  9
agaggctatt cggctatgac                                                    20


<210>  10
<211>  20
<212>  DNA
<213>  Artificial Sequence

<220>
<223>  Description of Artificial Sequence: an artificially synthesized
      primer sequence

<400>  10
caccatgata ttcggcaagc                                                    20


<210>  11
<211>  20
<212>  DNA
<213>  Mus musculus

<400>  11
tggcctctgt tatcatgctc                                                    20


<210>  12
<211>  20
<212>  DNA
<213>  Mus musculus

<400>  12
ctacctacat ccactcctac                                                    20


<210>  13
<211>  31
<212>  DNA
<213>  Artificial Sequence

<220>
<223>  Description of Artificial Sequence: an artificially synthesized
      primer sequence

<400>  13
agaagcttca ccatggggac cgccaggcta c                                       31


<210>  14
<211>  39
<212>  DNA
<213>  Artificial Sequence

<220>
<223>  Description of Artificial Sequence: an artificially synthesized
      primer sequence

<400>  14
ccgtcgacat tagatcttca caagcgcaca agccgggtc                               39


<210>  15
<211>  81
<212>  DNA

<213>  Artificial Sequence

<220>
<223>  Description of Artificial Sequence: Sequence containing loxP

<400>  15
gatccggaac ccttaatata acttcgtata atgtatgcta tacgaagtta ttaggtccct     60

cgacctgcag cccgggggat c                                               81


<210>  16
<211>  20
<212>  DNA
<213>  Artificial Sequence

<220>
<223>  Description of Artificial Sequence: an artificially synthesized
       primer sequence

<400>  16
aattaaccct cactaaaggg                                                 20


<210>  17
<211>  20
<212>  DNA
<213>  Mus musculus

<400>  17
cccactacga cagcttctcc                                                 20


<210>  18
<211>  20
<212>  DNA
<213>  Mus musculus

<400>  18
agccgggtca acataacagc                                                 20


<210>  19
<211>  19
<212>  DNA
<213>  Artificial Sequence

<220>
<223>  Description of Artificial Sequence: an artificially synthesized
       primer sequence

<400>  19
gcgttaccca acttaatcg                                                  19


<210>  20
<211>  18
<212>  DNA
<213>  Artificial Sequence

<220>
<223>  Description of Artificial Sequence: an artificially synthesized
       primer sequence

<400>  20
tgtgagcgag taacaacc                                                   18

<210>  21
<211>  22
<212>  DNA
<213>  Mus musculus

<400>  21
tcgtgtagta gccgtgtggt gt                                                22


<210>  22
<211>  20
<212>  DNA
<213>  Mus musculus

<400>  22
cacctgatgc acaggttcca                                                   20


<210>  23
<211>  19
<212>  DNA
<213>  Mus musculus

<400>  23
tggttgatcc tgccagtag                                                    19


<210>  24
<211>  20
<212>  DNA
<213>  Mus musculus

<400>  24
cgaccaaagg aaccataact                                                   20


<210>  25
<211>  23
<212>  DNA
<213>  Mus musculus

<400>  25
ccggtacagt gaaactgcga atg                                               23


<210>  26
<211>  17
<212>  PRT
<213>  Mus musculus

<400>  26

Asn Asp Lys Pro Phe Ser Thr Val Asp Arg Asp Arg Asp Ser Tyr Ser
1               5                   10                  15


Gly



<210>  27
<211>  17
<212>  PRT
<213>  Artificial Sequence

<220>
<223>  Description of Artificial Sequence: Peptide YP-126

<400>  27

Asn Thr Ser Arg Arg Leu Asp Gln Thr Pro Glu His Gln Arg Glu Gln
1               5                   10                  15

Cys

<210>  28
<211>  18
<212>  PRT
<213>  Artificial Sequence

<220>
<223>  Description of Artificial Sequence: Peptide YP-127-B

<400>  28

Cys Asn Asp Lys Pro Phe Ser Thr Val Asp Arg Asp Arg Asp Ser Tyr
1               5                   10                  15

Ser Gly

<210>  29
<211>  20
<212>  DNA
<213>  Mus musculus

<400>  29
ccgtgtggtg tgaacagcag                                                20

<210>  30
<211>  22
<212>  DNA
<213>  Mus musculus

<400>  30
tgaagaagtt gacagagccg tc                                             22

<210>  31
<211>  19
<212>  DNA
<213>  Mus musculus

<400>  31
cggagcgcaa tatgaaggt                                                 19

<210>  32
<211>  20
<212>  DNA
<213>  Mus musculus

<400>  32
caaaaggaag acgacggagc                                                20

<210>  33
<211>  20
<212>  DNA

<213>  Mus musculus

<400>  33
atgtctatag agatggatcc                                                    20


<210>  34
<211>  28
<212>  DNA
<213>  Artificial Sequence

<220>
<223>  Description of Artificial Sequence: an artificially synthesized
       primer sequence

<400>  34
ggggatcctc tgggtgcagg agactgaa                                           28


<210>  35
<211>  27
<212>  DNA
<213>  Artificial Sequence


<220>
<223>  Description of Artificial Sequence: an artificially synthesized
       primer sequence

<400>  35
cccctcgaga atgacgaagg cagcagg                                            27


<210>  36
<211>  27
<212>  DNA
<213>  Artificial Sequence

<220>
<223>  Description of Artificial Sequence: an artificially synthesized
       primer sequence

<400>  36
cccctcgagc tcacagaagc agttcac                                            27


<210>  37
<211>  27
<212>  DNA
<213>  Artificial Sequence

<220>
<223>  Description of Artificial Sequence: an artificially synthesized
       primer sequence

<400>  37
cccctcgagg gaagaatacc tggggag                                            27


<210>  38
<211>  27
<212>  DNA
<213>  Artificial Sequence

<220>
<223>  Description of Artificial Sequence: an artificially synthesized
       primer sequence

<400>  38
cccctcgagt taacaagcct tatgcgc                                          27


<210>  39
<211>  27
<212>  DNA
<213>  Artificial Sequence

<220>
<223>  Description of Artificial Sequence: an artificially synthesized
       primer sequence

<400>  39
cccctcgaga gagaaagggc acagggc                                          27


<210>  40
<211>  27
<212>  DNA
<213>  Artificial Sequence

<220>
<223>  Description of Artificial Sequence: an artificially synthesized
       primer sequence

<400>  40
cccctcgagg catgtaccat cacaccc                                          27


<210>  41
<211>  20
<212>  DNA
<213>  Mus musculus

<400>  41
gtagaaccaa tccctctggc                                                  20


<210>  42
<211>  26
<212>  DNA
<213>  Artificial Sequence

<220>
<223>  Description of Artificial Sequence: an artificially synthesized
       primer sequence

<400>  42
ggggatccgt cacctgcagt gaagag                                           26


<210>  43
<211>  20
<212>  DNA
<213>  Homo sapiens

<400>  43
gaacccgaga gcgaccacta                                                  20


<210>  44
<211>  20
<212>  DNA
<213>  Homo sapiens

<400>  44
ccctatccac ggtgctgaag                                                  20

<210>  45
<211>  32
<212>  DNA
<213>  Artificial Sequence

<220>
<223>  Description of Artificial Sequence: an artificially synthesized
       primer sequence

<400>  45
aatctagaca ccatggggac cgccaggcta cg                                    32


<210>  46
<211>  31
<212>  DNA
<213>  Artificial Sequence

<220>
<223>  Description of Artificial Sequence: an artificially synthesized
       primer sequence

<400>  46
aagcggccgc caagcgcaca agccgggtca a                                     31


<210>  47
<211>  32
<212>  DNA
<213>  Artificial Sequence

<220>
<223>  Description of Artificial Sequence: an artificially synthesized
       primer sequence

<400>  47
gaagatctac catggggacc gccaggctac gc                                    32


<210>  48
<211>  30
<212>  DNA
<213>  Artificial Sequence

<220>
<223>  Description of Artificial Sequence: an artificially synthesized
       primer sequence

<400>  48
ccgctcgagt gatgggacag tcacaagcgc                                       30


<210>  49
<211>  41
<212>  DNA
<213>  Artificial Sequence

<220>
<223>  Description of Artificial Sequence: an artificially synthesized
       primer sequence

<400>  49
ccaagcttac catggggaag ccctggctgc gtgcgctaca g                          41


<210>  50

<211>  35
<212>  DNA
<213>  Artificial Sequence

<220>
<223>  Description of Artificial Sequence: an artificially synthesized
       primer sequence

<400>  50
aactcgagca gcttcagggg ccgaatgagc atggc                                35


<210>  51
<211>  21
<212>  DNA
<213>  Rattus sp.

<400>  51
ctggcaacat tacaaggtgg g                                               21


<210>  52
<211>  21
<212>  DNA
<213>  Rattus sp.

<400>  52
ctagtcacct gatgcacggg t                                               21


<210>  53
<211>  34
<212>  DNA
<213>  Artificial Sequence

<220>
<223>  Description of Artificial Sequence: an artificially synthesized
       primer sequence

<400>  53
ggacgatgat gacaagggat ccactagtct cgag                                 34


<210>  54
<211>  34
<212>  DNA
<213>  Artificial Sequence

<220>
<223>  Description of Artificial Sequence: an artificially synthesized
       primer sequence

<400>  54
ctcgagacta gtggatccct tgtcatcatc gtcc                                 34


<210>  55
<211>  28
<212>  DNA
<213>  Artificial Sequence

<220>
<223>  Description of Artificial Sequence: an artificially synthesized
       primer sequence

<400>  55
ttggatccgc ttctcccatg cctgcagg                                        28

<210>  56
<211>  28
<212>  DNA
<213>  Artificial Sequence

<220>
<223>  Description of Artificial Sequence: an artificially synthesized
       primer sequence

<400>  56
ttctcgagtc acagcttcag gggccgaa                                          28